(19) Europäisches Patentamt
European Patent Office
Office européen des brevets

(11) **EP 4 162 823 B1**

(12) **EUROPÄISCHE PATENTSCHRIFT**

(45) Veröffentlichungstag und Bekanntmachung des Hinweises auf die Patenterteilung:
**09.04.2025   Patentblatt 2025/15**

(21) Anmeldenummer: **22204218.6**

(22) Anmeldetag: **21.10.2009**

(51) Internationale Patentklassifikation (IPC):
***A24F 40/44*** *(2020.01)*        ***A24F 40/46*** *(2020.01)*

(52) Gemeinsame Patentklassifikation (CPC):
**A61M 11/042; A24F 40/44; A24F 40/46;**
**A24F 40/485; A61K 31/465; A61M 11/041;**
**A61M 15/0021; A61M 15/0086; A61M 15/06;**
A24F 40/10; A61M 2016/0021; A61M 2205/11;
A61M 2205/3606; A61M 2205/3653;
A61M 2205/7536;                              (Forts.)

(54) **INHALATOR**

INHALER

INHALATEUR

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität:  **23.10.2008  AT 16602008**
           **17.04.2009  AT 5972009**

(43) Veröffentlichungstag der Anmeldung:
**12.04.2023   Patentblatt 2023/15**

(62) Dokumentnummer(n) der früheren Anmeldung(en) nach Art. 76 EPÜ:
**18205608.5 / 3 527 086**
**16166656.5 / 3 117 860**
**09756651.7 / 2 358 418**

(73) Patentinhaber: **Nicoventures Trading Limited London WC2R 3LA (GB)**

(72) Erfinder: **BUCHBERGER, Helmut**
**4482 Ennsdorf (AT)**

(74) Vertreter: **Schrell, Andreas et al**
**Gleiss Große Schrell und Partner mbB**
**Patentanwälte Rechtsanwälte**
**Leitzstrasse 45**
**70469 Stuttgart (DE)**

(56) Entgegenhaltungen:
**US-A1- 2003 108 342    US-A1- 2007 107 879**
**US-A1- 2008 092 912**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
A61M 2205/8206; A61M 2205/8237

# EP 4 162 823 B1

**Beschreibung**

Die Erfindung betrifft eine Inhalatorkomponente für die intermittierende, inhalations- oder zugsynchrone Bildung eines Dampf-Luft-Gemisches oder/und Kondensationsaerosols, umfassend:

> ein Gehäuse;

> eine im Gehäuse angeordnete Kammer;

> eine Lufteinlaßöffnung für die Zufuhr von Luft aus der Umgebung in die Kammer;

> ein elektrisches Heizelement zur Verdampfung einer Portion eines flüssigen Materials, wobei der gebildete Dampf sich in der Kammer mit der durch die Lufteinlaßöffnung zugeführten Luft mischt, und sich das Dampf-Luft-Gemisch oder/und Kondensationsaerosol bildet;

> und einen Docht mit einer Kapillarstruktur, welcher Docht mit dem Heizelement einen Verbund bildet und das Heizelement nach einer Verdampfung von neuem selbsttätig mit dem flüssigen Material versorgt.

Die Erfindung bezieht sich auf Inhalatoren, welche einen intermittierenden, inhalations- oder zugsynchronen Betrieb erlauben. Eine solche Betriebsart liegt vor, wenn das flüssige Material nur während eines Zuges oder während einer Inhalation aufgeheizt und verdampft wird. In Intervallen zwischen zwei Zügen oder Inhalationen ist das Heizelement weitgehend deaktiviert. Die Aktivierung bzw. Bestromung des Heizelements erfolgt in der Regel gleich am Beginn eines Zuges oder einer Inhalation, entweder manuell, beispielsweise mittels eines Schalters, vorzugsweise jedoch automatisch über einen geeigneten Sensor und einen elektronischen Schaltkreis. Im letzteren Fall spricht man auch von einem inhalations- oder zugaktivierten Betrieb des Inhalators.

In der gegenständlichen Patentanmeldung bezieht sich der Begriff "Inhalator" auf medizinische wie nicht-medizinische Inhalatoren. Der Begriff bezieht sich ferner auf Inhalatoren zur Verabreichung von Arzneimitteln und solchen Stoffen, welche nicht als Arzneimittel deklariert sind. Der Begriff bezieht sich außerdem auf Rauchartikel und Zigarettenersatz-Artikel, soweit diese dazu bestimmt sind, dem Benutzer ein Dampf-Luft-Gemisch oder/und Kondensationsaerosol darzureichen. Der Begriff "Inhalator" soll auch keine Einschränkungen dahingehend machen, wie das gebildete Dampf-Luft-Gemisch oder/und Kondensationsaerosol dem Benutzer bzw. dessen Körper zugeführt wird. Das Dampf-Luft-Gemisch oder/und Kondensationsaerosol kann in die Lunge inhaliert werden, oder aber auch nur der Mundhöhle zugeführt werden - ohne Inhalation in die Lunge. Schließlich schließt der Begriff "Inhalator" sowohl solche Geräte ein, welche eine direkte Lungeninhalation in einem einzigen Schritt gestatten ("klassische Inhalatoren"), als auch solche Geräte ein, welche - wie bei einer Zigarette - zumindest zwei Schritte erfordern, nämlich zuerst einen Zug in die Mundhöhle (Zugvolumen: ca. 20-80mL) und - nach Absetzen des Inhalators - eine daran anschließende Lungeninhalation ("Zug-Inhalatoren"). Klassische Inhalatoren weisen im Vergleich zu Zug-Inhalatoren einen deutlich höheren Luftdurchsatz durch den Inhalator auf: ca. 100-750mL/s gegenüber 10-40mL/s. Zug-Inhalatoren weisen demgegenüber in der Regel einen signifikant höheren Strömungswiderstand bzw. Zugwiderstand auf als klassische Inhalatoren.

Begriffsdefinition:

Verdampfungsenergie: Sensible plus latente Wärmemenge, welche auf das tatsächlich verdampfende flüssige Material übertragen wird.

Verdampfungsleistung: pro Zeiteinheit umgesetzte Verdampfungsenergie. Spezifische Verdampfungsleistung: auf die Masseneinheit des verdampfenden, flüssigen Materials bezogene Verdampfungsleistung.

Verdampfer-Wirkungsgrad: Quotient aus Verdampfungsenergie und vom Heizelement erzeugte Energie.

Über die Jahre wurde eine Vielzahl von Inhalatoren und elektrischen Rauchartikeln vorgeschlagen, welche elektrische Energie dazu nutzen, um Arzneimittel oder/und Aromastoffe zu verdampfen, und den erzeugten Dampf oder/und das gebildete Kondensationsaerosol einem Benutzer gegebenenfalls zur Inhalation bereitstellen.

GB 25,575 A.D.1911 (Elwin Kendal Hill) beschreibt einen Inhalator mit einem elektrischen Verdampfer zur Verdampfung von Medikamenten. Der Verdampfer besteht aus einer Scheibe 38 und einer perforierten Abdeckung 39. Im Raum zwischen der Scheibe 38 und der Abdeckung 39 befinden sich einerseits ein das Medikament aufnehmendes Absorptionsmaterial 40 und andererseits ein elektrisches Heizelement 41 - beispielsweise in Form eines Widerstand-Heizdrahtes. Das flüssige Medikament wird dem Absorptionsmaterial 40 bzw. Heizelement 41 selbsttätig über eine entsprechende Anzahl von Dochten 45 aus einem Vorratsbehälter 30 zugeführt. Die während der Inhalation angesaugte Luft strömt durch einen kegelförmigen Kanal 36, wodurch der Luftstrom auf den Verdampfer fokussiert wird, und nimmt auf diese Weise das verdampfte Medikament auf. Die Verdampferscheibe 38 wird mittels Abstandshülsen 44 in Position gehalten.

Nachteilig bei dieser Anordnung ist vor allem der komplizierte Aufbau des Verdampfers, seiner Halterung, sowie der Anbindung des Dochts an den Verdampfer. Die Vielteiligkeit und komplexe Struktur dieser Konstruktion macht den Inhalator teuer in der Herstellung und macht die Montage aufwendig.

Ein schwerwiegender Nachteil ist darin zu sehen, daß das Verhältnis der Dampfaustrittsfläche zum Verdampfervolumen verhältnismäßig klein ist. Dies liegt einerseits an der konkreten Geometrie des Verdampfers, und ist andererseits dadurch bedingt, daß das Absorptionsmaterial 40 und das elektrische Heizelement 41 weitgehend abgedeckt sind und zwar durch die Scheibe 38 und die Abdeckung 39. Diese Abdeckungen sind konstruktionsbedingt erforderlich, um das Absorptionsmaterial 40 und das elektrische Heizelement 41 zusammenzuhalten. Der im Inneren des Verdampfers gebildete Dampf kann ausschließlich durch die Löcher in der Abdeckung 39 entweichen. Dadurch kann es schon bei einer vergleichsweise mäßigen Verdampfungsleistung im Verdampfer zu einer Siedekrise kommen, weswegen diese Anordnung für einen intermittierenden, inhalations- oder zugsynchronen Betrieb, welcher grundsätzlich eine höhere spezifische Verdampfungsleistung bei einem gleichzeitig hohen Verdampfer-Wirkungsgrad voraussetzt, ungeeignet erscheint.

Nachteilig ist ferner, daß trotz der Vorkehrungen, welche gegen einen Austritt des flüssigen Medikaments aus dem Vorratsbehälter 30 getroffen wurden, ein solcher Austritt konstruktionsbedingt nicht gänzlich ausgeschlossen werden kann, insbesondere, wenn der Vorratsbehälter 30 z.B. durch eine fehlerhafte Bedienung überfüllt wird. Schließlich ist kritisch zu beurteilen, daß das flüssige Medikament im Vorratsbehälter 30 praktisch frei der Umgebungsluft ausgesetzt ist, was zu einer Oxidation des Medikaments oder/und zu einer Änderung seiner Zusammensetzung aufgrund von Verdunstungseffekten führen kann.

US 2,057,353 (Clinton L. Whittemore) beschreibt eine Verdampfereinheit für einen therapeutischen Apparat, bestehend aus einem Gefäß A zur Aufnahme eines flüssigen Medikaments x, durch den Gefäßboden in das Gefäß ragende elektrische Leiter 1 und 2, einen Heizdraht 3, welcher mit den elektrischen Leitern verbunden ist, sowie einem Docht D, welcher vom Heizdraht 3 umwickelt ist und sich von diesem bis zum Gefäßboden erstreckt. Das Gefäß weist eine Lufteinlaßöffnung 4 und eine Dampfaustrittsöffnung 5 auf, welche beide nach innen gewölbt sind, um einen Austritt des Medikaments aus dem Gefäß zu vermeiden.

Nachteilig bei dieser Konstruktion ist der aufwendige Herstellungsprozeß der Verbindung zwischen dem Heizelement und dem Docht. Der Docht muß vor der Montage mit dem Heizdraht umwickelt werden. Diese Prozedur gestaltet sich vor allem deshalb aufwendig, weil die zu fügenden Teile üblicherweise ausgesprochen klein dimensioniert sind. Außerdem ist schwer sicherzustellen, daß die Heizdrahtwicklungen alle am Docht anliegen. Lokale Ablösungen können zu Überhitzungen des Heizdrahtes in diesen Bereichen führen und das Widerstandsmaterial schneller altern lassen. Diese Problematik betrifft auch die Bereiche wo der Heizdraht mit den elektrischen Leitern 1 und 2 verbunden ist.

Ein weiterer Nachteil besteht darin, daß die äußere Oberfläche des Dochts D durch die Umwicklung mit dem Heizelement 3 teilweise abgedeckt wird. Die Umwicklung stellt insofern ein Hindernis für den aus dem Docht austretenden Dampf dar. Diese Behinderung der Dampfströmung kann ähnliche Folgen nach sich ziehen, wie bereits zuvor für die Schrift GB 25,575 A.D.1911 näher ausgeführt wurde. Darüber hinaus kommt der gebildete Dampf beim Ausströmen zumindest partiell mit dem heißen Heizdraht in Berührung, was zu einer thermischen Zersetzung des Medikaments x führen kann.

Nachteilig ist ferner, daß der Docht D lediglich von dem verhältnismäßig dünnen Heizdraht 3 in Position gehalten wird. Schon eine Erschütterung könnte die Position des Dochts D verändern und die Strömungs- und Mischungsverhältnisse zwischen der durch die Öffnung 4 angesaugten Luft und des vom Docht D abströmenden Dampfes erheblich verändern und die Aerosolbildung beeinträchtigen. Die Apparatur kann nur in aufrechter oder leicht geneigter Position betrieben werden; ein Austritt des Medikaments x aus dem Gefäß A kann trotz der ergriffenen konstruktiven Maßnamen nicht gänzlich ausgeschlossen werden. Schließlich ist das Medikament x im Gefäß A praktisch frei der Umgebungsluft ausgesetzt, ein Umstand, welcher ebenfalls als sehr ungünstig gewertet werden muß.

FR 960,469 (M. Eugène Vacheron) beschreibt einen Inhalationsapparat mit einem elektrischen Verdampfer. Der Inhalationsapparat umfaßt eine elektrische Heizpatrone 4, 5, 6 und einen Docht 16, welcher Docht mit der im Behälter 1 gespeicherten Flüssigkeit getränkt ist. Die Heizpatrone befindet sich außerhalb des Behälters 1, ist also nicht direkt mit dem Docht verbunden. Die speziellen konstruktiven Bedingungen machen den Inhalationsapparat wärmetechnisch träge und lassen ihn allenfalls für einen kontinuierlichen Verdampferbetrieb geeignet erscheinen; ein intermittierender, inhalations- oder zugsynchroner Betrieb erscheint nicht realisierbar.

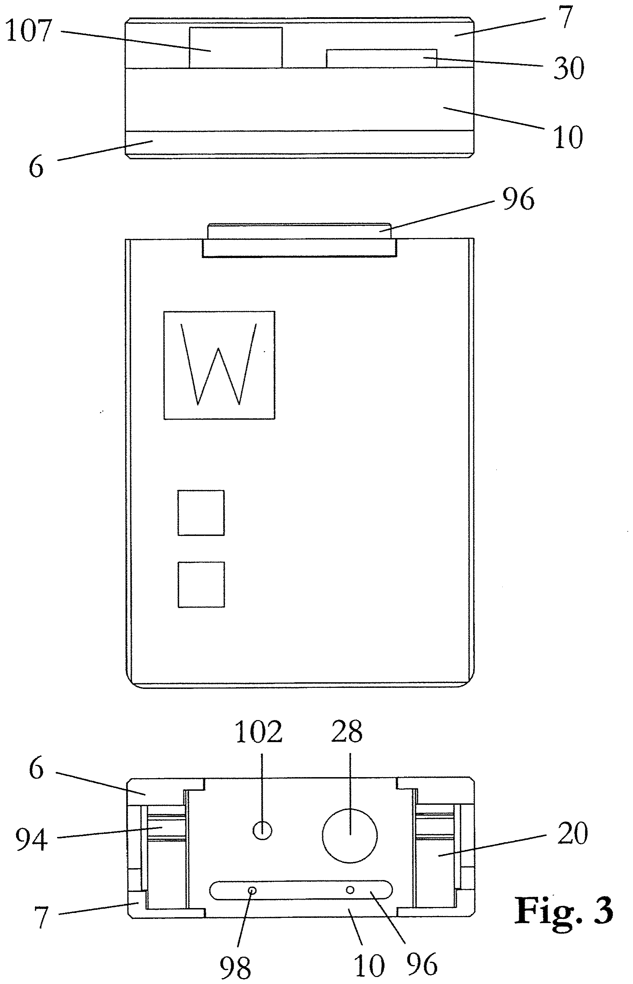

CA 2,309,376 (Matsuyama Futoshi) beschreibt einen Verdampfer bzw. Zerstäuber für medizinische Anwendungen, bestehend aus (Fig. 3) einem Gefäß 1 mit einer flüssigen Rezeptur und einem stabförmigen, porösen Material 3, welches im Gefäß 1 installiert ist. Das stabförmige, poröse Material 3 taucht mit einem Ende in die flüssige Rezeptur ein, während das andere Ende sich außerhalb des Gefäßes 1 frei nach oben erstreckt. Das Gefäß 1 und das stabförmige, poröse Material 3 sind in einem gewölbten Behälter 5 angeordnet. Der gewölbte Behälter 5 hält einerseits das Gefäß 1 in Position und beinhaltet andererseits eine elektrische Heizvorrichtung 6, welche das stabförmige, poröse Material 3 in einem oberen Endabschitt beabstandet ummantelt, wobei der Abstand vorzugsweise im Bereich 0,8-2,5mm liegt. Die Kapillarkräfte im stabförmigen, porösen Material 3 bewirken, daß die flüssige Rezeptur nach oben gesaugt wird, wo die Rezeptur schließlich von der elektrischen Heizvorrichtung 6 verdampft wird. Die in der flüssigen Rezeptur enthaltenen Wirkstoffe werden dabei zerstäubt und treten durch die Öffnung 9 aus dem gewölbten Behälter 5 in den Raum über, so daß sie vom Benutzer inhaliert werden können. Die flüssige Rezeptur besteht aus einer wässrigen Lösung, in welcher ein Wirkstoffkonzentrat gelöst oder dispergiert ist. Die wässrige Lösung besteht vorzugsweise aus Wasser oder einer Mischung aus Wasser und Ethanol, Das Wirkstoffkonzentrat wird aus den Blättern von Lagerstroemia Speciosa gewonnen, und enthält bis zu 15 Massen-% Corosol-Säure. Das Wirkstoffkonzentrat wirkt angeblich blutzuckersenkend. Der Anteil des Wirkstoffkonzentrats (gerechnet als Corosol-Säure) in der wässrigen Lösung beträgt 0,5-3,0 Massen-%.

Der Verdampfer ist für einen kontinuierlichen Betrieb ausgelegt. Die elektrische Heizvorrichtung 6 ist beabstandet zum porösen Material 3 angeordnet, bildet folglich mit diesem keinen Verbund. Der Spalt dazwischen stellt einen hohen Wärmeleitungswiderstand dar. Ein intermittierender Betrieb mit einer entsprechend hohen spezifischen Verdampfungsleistung wäre nur realisierbar, wenn die Wärme durch Wärmestrahlung übertragen würde. Hierzu wäre die elektrische Heizvorrichtung 6 blitzartig auf eine sehr hohe Temperatur aufzuheizen. Die flüssige Rezeptur würde in erster Linie in der der Heizvorrichtung zugewandten Randzone verdampfen und durch den bereits genannten Spalt in die Umgebung strömen. Ungeachtet der praktischen Realisierbarkeit dieses Konzeptes würde jedenfalls der gebildete Dampf in Kontakt mit der glühenden Öberfläche der Heizvorrichtung 6 kommen, wodurch das Wirkstoffkonzentrat zumindest teilweise thermisch zersetzt würde.

US 6,155,268 (Manabu Takeuchi) beschreibt ein Aroma-erzeugendes Gerät, bestehend aus (Fig. 1) einer Kammer 121 mit einem Lufteinlaß 18 und einer Mundstücköffnung 22 bzw. Mundstück 16, wodurch ein Gasdurchgangskanal 20 gebildet wird, und umfaßt ferner einen Flüssigkeitsbehälter 32 zur Aufnahme eines flüssigen Aromastoffes 34, und schließlich ein Kapillarrohr 36 mit einem ersten Endabschnitt, welcher in die Flüssigkeit im Behälter 32 eintaucht und einem zweiten Endabschnitt, welcher mit dem Gasdurchgangskanal 20 kommuniziert und weiters ein Heizelement 42 umfaßt. Der flüssige Aromastoff 34 strömt durch die im Kapillarrohr 36 wirkenden Kapillarkräfte zum Heizelement 42, wo er verdampft und als Dampfstrom aus der Öffnung 36b in den Gasdurchgangskanal 20 ausströmt. Der von außen durch den Lufteinlaß 18 in die Kammer 121 eintretende Luftstrom wird durch die Lochblende 24, 24a auf die Kapillaröffnung 36b fokussiert, wodurch günstige Bedingungen für eine innige Mischung zwischen Dampf und angesaugter Luft bzw. für die Bildung eines Aerosols geschaffen werden sollen.

In alternativen Ausführungen (Fig. 8-13) werden plattenförmige Heizelemente vorgeschlagen. In weiteren Ausführungsbeispielen (Fig. 14 und 15) ist das Kapillarrohr im Inneren mit einer Porenstruktur 302 gefüllt, welche in einer Variante auch aus dem Kapillarrohr herausragen kann, wobei in diesem letzteren Fall das Heizelement 425 am Ende der herausragenden Porenstruktur angeordnet sein kann.

Nachteilig bei diesen Anordnungen ist wiederum der verhältnismäßig komplizierte Aufbau der Verdampfereinheit - in diesem Fall bestehend aus dem Kapillarrohr und dem Heizelement. Diese beiden Mikrokomponenten müssen miteinander verbunden werden und das Heizelement muß an die elektrische Versorgung angeschlossen werden, was sich im konkreten Fall wohl nur über elektrische Drähte realisieren läßt. Leider gibt die Schrift diesbezüglich keine genaueren Hinweise.

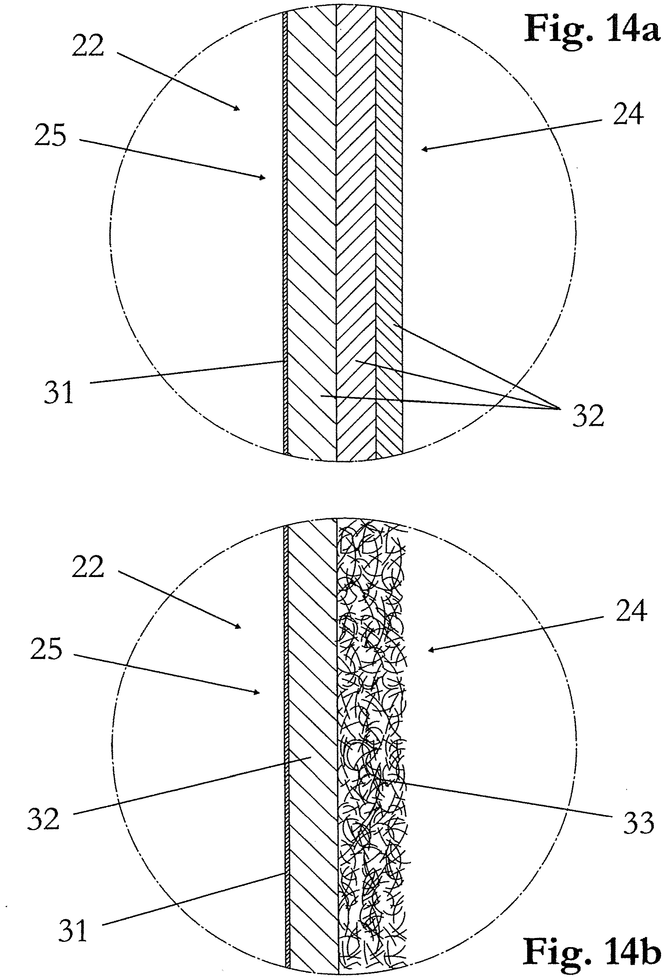

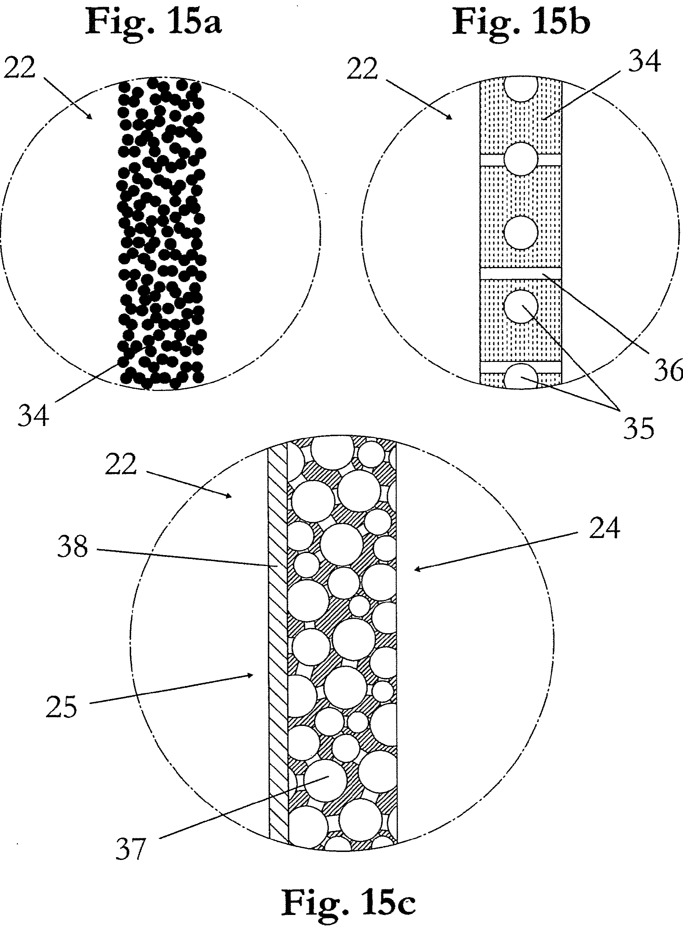

Für die Anordnungen nach Fig. 14 und 15 gilt Ähnliches wie bereits zu GB 25,575 A.D.1911 ausgeführt wurde: das Verhältnis der Dampfaustrittsfläche zum Verdampfervolumen ist ausgesprochen klein. Dies liegt daran, daß die Porenstruktur 302 durch die Ummantelung 301 und das Heizelement 425 weitgehend abgedeckt wird. Dadurch kann es schon bei einer mäßigen Verdampfungsleistung zu einer Siedekrise kommen, weshalb die Funktion dieser Anordnungen grundsätzlich in Frage zu stellen ist, vor allem wenn ein intermittierender, inhalations- oder zugsynchroner Betrieb gefordert wird.

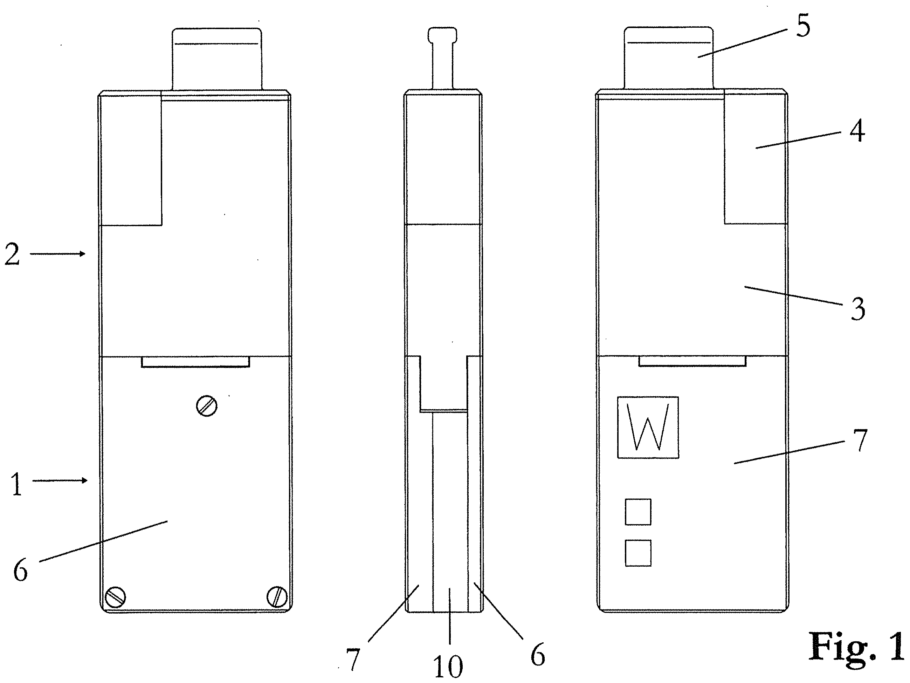

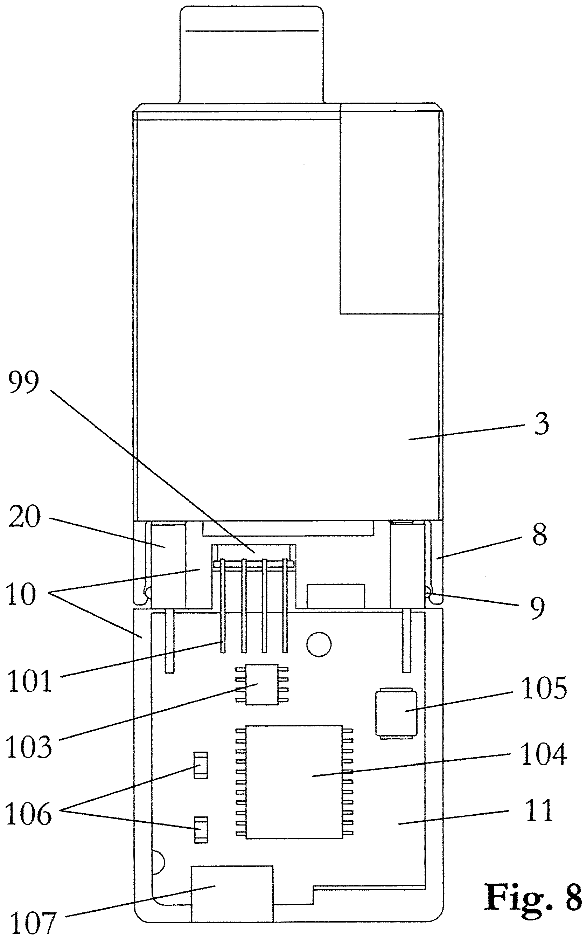

Für den Flüssigkeitsbehälter 32 werden zwei Varianten vorgeschlagen: in einer ersten Variante (Fig. 1) ist der Flüssigkeitsbehälter ein fixer Bestandteil des Aroma-erzeugenden Gerätes. Der Flüssigkeitsbehälter kann über eine Füllöffnung wiederbefüllt werden. Eine solche Wiederbefüllung birgt jedoch Risken für die Umwelt, vor allem dann, wenn der flüssige Aromastoff Arzneimittel oder Gifte wie beispielsweise Nikotin enthält, und die Wiederbefüllung durch den Benutzer selbst durchgeführt wird. In einer alternativen Variante (Fig. 8) ist der Flüssigkeitsbehälter als kleiner austauschbarer Behälter ausgebildet. Details über die Ankopplung wurden nicht offenbart. Kleine austauschbare Behälter bergen stets das Risiko, von Kleinkindern verschluckt zu werden, was potentiell letal enden kann, insbesondere wenn der flüssige Aromastoff Arzneimittel oder Gifte wie beispielsweise Nikotin enthält.

Die Anordnung nach Fig. 8 zeigt ferner ein auswechselbares Mundstück 161 mit einem hohlzylindrischen Fortsatz, welcher einen Großteil der Kammer 121 auskleidet und sich fast bis zur Mündung der Kapillare 371 erstreckt. In der Kammer 121 anfallende Kondensatrückstände lagern sich vornehmlich auf der Innenfläche des hohlzylindrischen Fortsatzes ab, und können gemeinsam mit dem Mundstück entfernt werden. Problematisch ist, daß die Innenfläche für Kondensat nur begrenzt aufnahmefähig ist. Vor allem, wenn der flüssige Aromastoff größere Anteile an niedrig siedenden Fraktionen mit hohem Dampfdruck enthält - z.B. Ethanol oder/und Wasser, muß das Mundstück in kurzen Abständen gewechselt werden. Andernfalls formieren sich auf der Innenfläche des Mundstücks unter dem Einfluß von Oberflächenspannungen Tropfen, welche stetig an Volumen zunehmen, bis die Adhesionskräfte schließlich nicht mehr ausreichen, um die Tropfen zu halten, und diese sich zu größeren Flüssigkeitsansammlungen vereinigen. Diese Flüssigkeitsansammlungen können die Funktion des Gerätes beeinträchtigen, können aber auch ein Risiko für den Benutzer und die Umwelt darstellen, sofern diese Ansammlungen Arzneimittelreste oder Gifte wie beispielsweise Nikotin enthalten. Aber auch schon die Möglichkeit an sich, das Kondensat vom Benutzer selbst aus dem Gerät entfernen zu lassen, birgt ein Risiko für die Umwelt.

US 4,922,901, US 4,947,874 und US 4,947,875 (Johnny L. Brooks et al.) beschreiben Artikel für die Freisetzung bzw. Verabreichung von Arzneimitteln oder/ und Aromen mit einer auswechselbaren Einheit 12, welche ein elektrisches Widerstandsheizelement 18 beinhaltet, dessen Oberfläche größer als zumindest 1m^2/g ist; das elektrische Widerstandsheizelement 18 trägt aerosolbildende Substanzen. Vorzugsweise besteht das elektrische Widerstandsheizelement 18 aus einem porösen oder faserigen Material - z.B. aus Kohlenstofffasern, welches Material mit einem flüssigen Aerosolbildner durchtränkt ist. Die Artikel beinhalten ferner eine zugaktivierte elektronische Kontrolleinheit 14 zur Steuerung des Stroms durch das elektrische Widerstandsheizelement 18 und sind imstande, pro Zug mindestens 0,8mg Aerosol bzw. Arzneimittel zu verabreichen, wobei insgesamt mindestens 10 Züge ermöglicht werden, bevor die auswechselbare Einheit 12 samt Widerstandsheizelement 18 durch eine neue ersetzt werden muß.

Bei diesem Artikel liegt also das gesamte zu verdampfende, flüssige Material bereits im Widerstandsheizelement 18 vorgespeichert vor. Eine Flüssigkeitszufuhr über einen Docht ist nicht vorgesehen. Daraus resultieren auch die Nachteile: die aerosolbildenden Substanzen bzw. das Arzneimittel oder/ und etwaige zugesetzte Aromastoffe, welche beispielsweise während des letzten Zuges freigesetzt werden, wurden zuvor schon vielfach aufgeheizt, welcher Umstand eine thermische Zersetzung der aerosolbildenden Substanzen begünstigt. Diese vorangegangenen Aufheizungen sind außerdem insofern ungünstig, als hierfür zusätzliche elektrische Energie erforderlich ist, welche keinen Beitrag zur eigentlichen Verdampfung bzw. Aerosolbildung leistet. Dies hat einen sehr niedrigen Verdampfer-Wirkungsgrad zur Folge. Ein weiterer Nachteil ist, daß im Fall von Mischungen von verschiedenen aerosolbildenden Substanzen, Arzneimittel und Aromastoffen mit unterschiedlichen Siedepunkten der Einzelsubstanzen die chemische Zusammensetzung des gebildeten Aerosols und dessen organoleptische und pharmakologische Wirkung von einer Inhalation zur nächsten variiert, wobei während der ersten Züge vermehrt niedersiedende Fraktionen verdampft werden, und während der letzten Züge vermehrt höhersiedende Substanzen freigesetzt werden. Schließlich muß die in der Herstellung relativ aufwendige auswechselbare Einheit 12 und damit auch das Heizelement 18 schon nach etwa 10 Zügen ersetzt werden, was den Gebrauch dieser Artikel teuer macht.

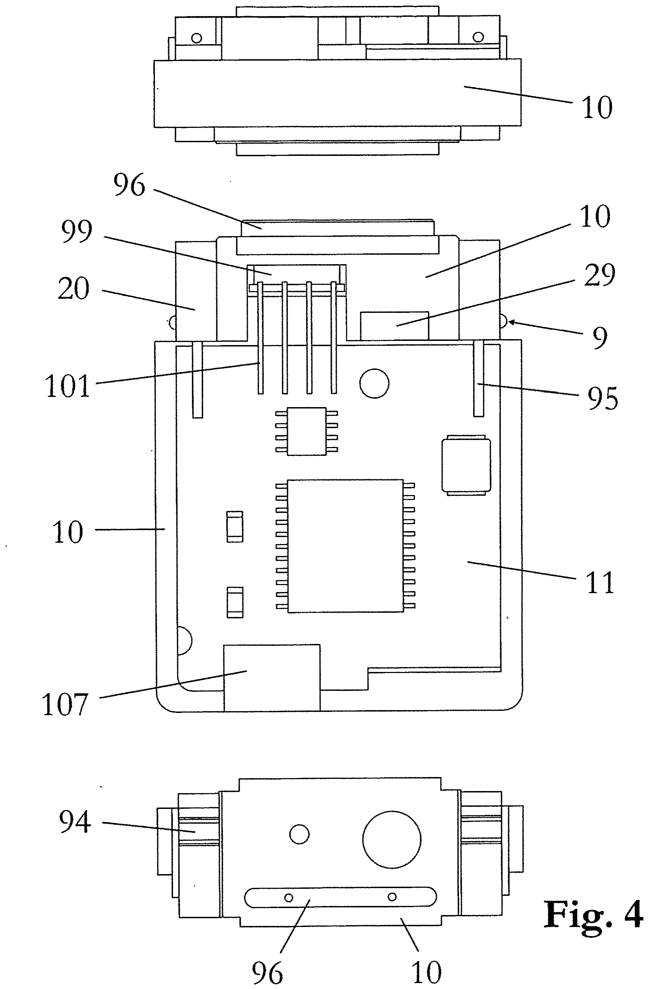

US 5,060,671 und US 5,095,921 (Mary E. Counts, D. Bruce Losee et al.) beschreiben einen Artikel 30 (Fig. 4), in welchem ein Aromen-freisetzendes Medium 111 durch elektrische Heizelemente 110 erhitzt wird, um inhalierbare Aromen in Dampf- oder Aerosolform darzureichen. Der Artikel beinhaltet mehrere Ladungen des Aromen-freisetzenden Mediums 111, welche sequenziell erhitzt werden und auf diese Weise einzelne Züge darbieten. Die mehreren Ladungen des Aromen-freisetzenden Mediums 111 werden auf den Heizelementen 110 vorzugsweise als Überzug, Beschichtung oder als dünner Film appliziert und können auch aerosolbildende Substanzen beinhalten. Die Haftung des Aromen-freisetzenden Mediums 111 auf den Heizelementen 110 kann durch ein haftvermittelndes Agens wie zum Beispiel Pektin verbessert werden. Die elektrischen Heizelemente 110 und die auf diesen aufgetragenen Ladungen des Aromen-freisetzenden Mediums 111 sind vorzugsweise in einer auswechselbaren Einheit 11 angeordnet, welche über elektrische Kontaktstifte mit einer wiederverwendbaren Einheit 31 verbunden ist. Die wiederverwendbare Einheit 31 beinhaltet eine elektrische Energiequelle 121 sowie eine elektronische Steuerschaltung 32. US 5,322,075 (Seetharama C. Deevi et al.) beschreibt einen ähnlichen Artikel.

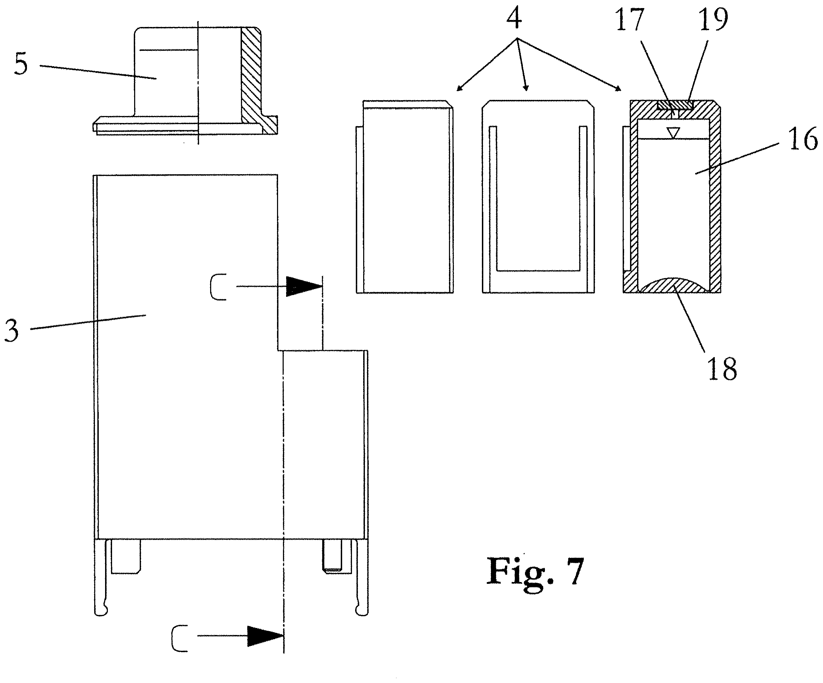

Obwohl dieser Artikel einige der Nachteile der zuvor beschriebenen Artikel (US 4,922,901, US 4,947,874 und US 4,947,875) behebt, erscheint die Konstruktion der auswechselbaren Einheit 11 noch komplexer, da im konkreten Fall eine Vielzahl von Heizelementen samt elektrischer Kontaktierung vorgesehen ist. Berücksichtigt man ferner, daß die komplexe auswechselbare Einheit 11 kaum mehr als 15 Züge zuläßt (vgl. Fig. 7A-7K), wird klar, daß der Gebrauch eines solchen Artikels teuer wäre. Weiters liegt im konkreten Fall das Aromenfreisetzende Medium 111 als relativ großflächige dünne Schicht vor, welche vor allem während der Lagerung der auswechselbaren Einheit 11 diversen Umgebungseinflüssen (Oxidation, etc.) ausgesetzt ist. Um diese Einflüsse zu abzuwenden, wäre eine aufwendige Verpackung vorzusehen, welche das Medium 111 gegenüber der Umgebung schützt aber möglichst nicht berührt. Auf diesen Aspekt gehen US 5,060,671 und US 5,095,921 nicht ein.

US 2005/0268911 (Steven D. Cross et al.) ist dem zuvor beschriebenen Artikel nach US 5,060,671 und US 5,095,921 sehr ähnlich und beschreibt ein Gerät zur Erzeugung und Abgabe mehrerer Dosen eines Kondensationsaerosols für die Inhalation von Medikamenten von hoher Reinheit und besteht im einfachsten Fall (Fig. 1A) aus einem Luftkanal 10 mit einem Einlaß und einem Auslaß, mehreren im Luftkanal angeordneten Trägern 28, welche jeweils eine bestimmte Dosis einer Substanz/ eines Medikaments tragen, und einer Einrichtung zur Verdampfung dieser diskreten Dosen. Der durch den Einlaß einströmende Luftstrom wird zu den Trägern 28 geleitet, wo schließlich das Kondensationsaerosol gebildet wird. Die Träger 28 beinhalten jeweils ein elektrisches Widerstandsheizelement - vorzugsweise bestehend aus einer Metallfolie 78 aus nichtrostendem Stahl. Die Metallfolien-Heizelemente 78 sind vorzugsweise auf einer Platine montiert (Fig. 4). Die Nachteile des Artikels nach US 5,060,671 und US 5,095,921 gelten gleichermaßen für das Gerät nach US 2005/0268911.

US 5,505,214 und US 5,865,185 (Alfred L. Collins et al.) beschreiben elektrische Rauchartikel bestehend aus (Fig. 4; US 5,505,214) einer auswechselbaren Einheit 21 und einem wiederverwendbaren Teil 20. Die auswechselbare Einheit 21 beinhaltet Tabakaromen 27, welche sich auf einem Träger 36 befinden. Der wiederverwendbare Teil 20 beinhaltet mehrere Heizelemente 23, welche von einer elektrischen Energiequelle - beispielsweise einem wiederaufladbaren Akku - über eine elektrische Steuerschaltung mit Strom bzw. Energie versorgt werden. Nach Einsetzen der auswechselbaren Einheit 21 in den wiederverwendbaren Teil 20 kommt der Träger 36 auf den Heizelementen 23 zu liegen. Während einer Inhalation bzw. eines Zuges wird jeweils ein einzelnes Heizelement von der Steuerschaltung aktiviert, wodurch der Träger 36 abschnittsweise erhitzt und die Tabakaromen 27 verdampft und ggf. als Aerosol freigesetzt werden. Im Ausführungsbeispiel nach Fig. 4 enthält der wiederverwendbare Teil 20 acht Heizelemente 23, wonach ähnlich wie bei einer Zigarette acht Inhalationen oder Züge ermöglicht werden. Danach ist die auswechselbare Einheit 21 durch eine neue Einheit zu ersetzen.

Gegenüber dem Artikel nach US 5,060,671 und US 5,095,921 weisen die Rauchartikel nach US 5,505,214 und US 5,865,185 den Vorteil auf, daß die Heizelemente 23 stationär im wiederverwendbaren Teil 20 angeordnet sind und somit mehrfach verwendet werden können. Elektrische Kontakte zwischen der auswechselbaren Einheit 21 und dem wiederverwendbaren Teil 20 sind nicht erforderlich. Nachteilig gegenüber dem Artikel laut US 5,060,671 und US 5,095,921 ist jedoch, daß neben den Heizelementen 23 zusätzlich der Träger 36 erhitzt werden muß; die hierfür erforderliche Wärme verschlechtert den Verdampfer-Wirkungsgrad. Die übrigen, bereits früher angeführten Nachteile des Artikels laut US 5,060,671 und US 5,095,921 gelten sinngemäß.

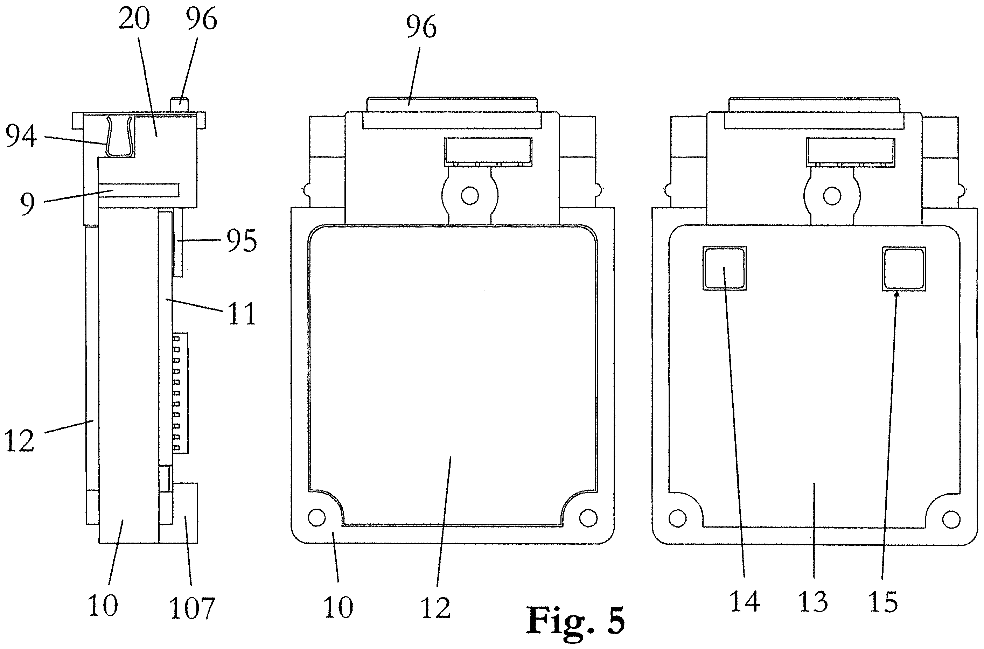

US 4,735,217 (Donald L. Gerth et al.) beschreibt eine Dosiereinheit zur Verabreichung von verdampften Medikamenten in Form von feinen Aerosolpartikeln, welche durch Inhalation in die Lunge gelangen. Die Dosiereinheit besteht in einem exemplarischen Ausführungsbeispiel (Fig. 4 und 5) aus einem folienartigen Nichrome ® Heizelement-Segment 72 (Länge x Breite x Dicke: 1 x 1/8 x 0,001 Zoll), welches in Serie mit einer Batterie 65 und einem Luftstrom- bzw. zugaktivierten Schalter (60,69) verbunden ist. Das zu verdampfende Medikament - beispielsweise Nikotin - liegt als festes Pellet 40 vor, welches das Heizelement 72 kontaktiert. Alternativ kann das zu verdampfende Medikament direkt auf die Heizelement-Oberfläche in Form einer Beschichtung oder eines Films aufgebracht werden.

Einige Nachteile dieser Dosiereinheit wurden zum Teil schon in US 4,922,901 erwähnt. Hinzu kommt, daß sich der Wärmeübergang vom Heizelement auf das Pellet sehr ungünstig darstellt. Ein Großteil des Heizelements 72 wird ohne Nutzen aufgeheizt, da die in peripheren Bereichen des Heizelements gebildete Wärme nur zu einem geringen Teil für das Pellet nutzbar ist. Grundsätzlich von Nachteil ist, daß zur Bildung des Pellets Feststoffe gebraucht werden, welche im Allgemeinen erst einmal geschmolzen werden müssen, bevor sie verdampft werden können, wodurch sich die Energiebilanz weiter verschlechtert.

EP 1,736,065 (Hon Lik) beschreibt eine "elektronische Zigarette" zur Zerstäubung einer Nikotinlösung und besteht im Wesentlichen aus einem Behälter 11 zur Aufnahme der zu zerstäubenden Flüssigkeit und einem Zerstäuber 9. Im Inneren des Zerstäubers 9 befindet sich eine Zerstäuberkammer 10, welche durch die Zerstäuberkammerwand 25 gebildet wird. Innnerhalb der Zerstäuberkammer 10 ist ein elektrisches Heizelement 26 beispielsweise in Form eines Widerstand-Heizdrahtes oder einer PTC-Keramik angeordnet. Im Zerstäuber bzw. in der Zerstäuberwand 25 sind weiters Auswurflöcher 24, 30 vorgesehen, welche in Richtung des Heizelements 26 weisen. Der Behälter 11 beinhaltet einen porösen Körper 28 - beispielsweise bestehend aus Kunststofffasern oder Schaumstoff, welcher mit der zu zerstäubenden Flüssigkeit getränkt ist. Die Zerstäuberkammerwand 25 ist ebenfalls von einem porösen Körper 27 - beispielsweise bestehend aus Nickelschaum oder einem Metallfilz umgeben. Der poröse Körper 27 steht mit dem porösen Körper 28 über eine Ausbuchtung 36 in Kontakt. Kapillarkräfte bewirken, daß der poröse Körper 27, welcher gleichzeitig die äußere Hülle des Zerstäubers 9 bildet, mit der zu zerstäubenden Flüssigkeit infiltriert wird. Der Zerstäuber umfaßt ferner ein piezoelektrisches Element 23.

Die "elektronische Zigarette" wird zugaktiviert betrieben. Während eines Zuges entsteht in der Zerstäuberkammer 10 ein Unterdruck, da diese mit dem Mundstück 15 in Verbindung steht. Dadurch strömt Luft aus der Umgebung über die Auswurflöcher 24, 30 in die Zerstäuberkammer. Die hohe Strömungsgeschwindigkeit in den Auswurflöchern 24, 30 bewirkt, daß Flüssigkeit aus dem porösen Körper 27 gesaugt und vom Luftstrom in Form von Tropfen mitgerissen wird (Venturi-Effekt). Die nikotinhältige Flüssigkeit gelangt in die Zerstäuberkammer 10, wo sie mittels des piezoelektrischen Elements 23 durch Ultraschall zerstäubt wird. Das Heizelement 26 soll eine zusätzliche Zerstäubung bzw. Verdampfung der Nikotinlösung bewirken. In einer alternativen Ausgestaltungsvariante erfolgt die Zerstäubung ausschließlich durch das Heizelement 26.

Die Anordnung weist funktionelle Ähnlichkeiten mit dem in US 4,848,374 (Brian C. Chard et al.) offenbarten Rauchgerät auf. In beiden Fällen nachteilig ist, daß die Dosierung der zu zerstäubenden Flüssigkeit bzw. des gebildeten Aerosols ähnlich wie bei einer Zigarette vom jeweiligen Zugprofil des Nutzers abhängt. Dies ist jedoch bei medizinischen oder therapeutischen Anwendungen unerwünscht. Hinzu kommt, daß die Zerstäubung mittels Ultraschall im Allgemeinen deutlich größere Aerosolpartikel erzeugt als Kondensationsaerosole üblicherweise aufweisen. Diese größeren Partikelfraktionen gelangen nicht bis zu den Lungenbläschen, sondern werden vielmehr schon in vorgelagerten Lungenabschnitten absorbiert, was sich im Fall von systemisch wirkenden Arzneimitteln wie Nikotin auf die Absorptionskinetik und die Effizienz der Wirkstoffzuführung sehr ungünstig auswirkt. Ferner muß insbesondere im Fall der alternativen Ausgestaltungsvariante ohne Ultraschall-Zerstäubung angezweifelt werden, ob das ähnlich einem Glühbirndraht ausgebildete elektrische Heizelement überhaupt in der Lage ist, die für die Verdampfung während eines Zuges erforderliche Heizenergie auf das flüssige Material zu übertragen. Dies wäre wohl nur durch Wärmestrahlung möglich, wozu das Heizelement sprichwörtlich auf Glühtemperatur gebracht werden müßte. Solch hohe Temperaturen sind grundsätzlich mit verschiedenen Gefahren und Nachteilen verbunden - unter anderem mit der Gefahr einer thermischen Zersetzung der zu zerstäubenden oder bereits zerstäubten Flüssigkeit. Schließlich ist als hohes Sicherheitsrisiko zu werten, daß der die sehr giftige Nikotinlösung enthaltende Behälter auf einer Stirnseite offen ist und ferner von der "elektrischen Zigarette" lösbar ist. Dieses Risiko wurde bereits erkannt, und wurde in einer Weiterbildung - wie in DE 202006013439U offenbart - insofern teilweise entschärft, als der Behälter durch eine hermetisch abgeschlossene Patrone gebildet wird, welche Patrone allerdings nachteiligerweise immer noch von der "elektrischen Zigarette" lösbar ist und beispielsweise von Kleinkindern verschluckt werden kann.

Aus der US 2007/107879 A1 ist ein Inhalator für intermittierende, inhalations- oder zugsynchrone Bildung eines Dampf-LuftGemisches oder/und Kondensationaerosols mit einem elektrischen Heizelement und einem Docht mit Kapillarstruktur, welcher das Heizelement nach der Verdampfung mit flüssigem Material versorgt, bekannt.

US2003/108342 A1 offenbart einen weiteren Aerosolgenerator umfassend eine Flüssigkeitszufuhr, die eine Flüssigkeit zu einem Flüssigkeitsdurchlass liefert, und eine Heizvorrichtung, die die Flüssigkeit in einen gasförmigen Zustand erhitzt, wobei der Flüssigkeitsdurchlass zwischen gegenüberliegenden Schichten eines Laminats angeordnet ist.

Abschließend sei angemerkt, daß einige der soeben dargestellten Dokumente, obwohl sie nicht zur eingangs bezeichneten Erfindungsgattung zählen, dennoch beschrieben wurden, da sie zumindest den weiteren Stand der Technik abbilden und insofern würdig sind, berücksichtigt zu werden.

Der Erfindung liegt die Aufgabe zugrunde, die zuvor aufgezeigten Nachteile der aus dem Stand der Technik bekannten Anordnungen zu beheben. Der Erfindung liegt insbesondere die Aufgabe zugrunde, eine Inhalatorkomponente der eingangs geschilderten Art so auszugestalten, daß die für den intermittierenden, inhalations- oder zugsynchronen Betrieb erforderliche hohe spezifische Verdampfungsleistung bei gleichzeitig hohem Verdampfer-Wirkungsgrad realisiert werden kann. Der erforderliche Leistungs- und Energiebedarf soll dabei durch einen Energiespeicher etwa im Format eines durchschnittlichen Mobiltelefon-Akkus gedeckt werden können. Das Auftreten einer Siedekrise im Docht soll vermieden werden, und das flüssige Material soll möglichst schonend, also ohne wesentliche thermische Zersetzung verdampft werden können.

Die Inhalatorkomponente soll ferner einen benutzerfreundlichen und sicheren Betrieb gestatten, und dabei möglichst kostengünstig hergestellt werden können, was konkret bedeutet: Der Verbund soll vom flüssigen Material möglichst rasch infiltriert werden, so daß zwischen zwei Inhalationen oder Zügen keine wesentlichen Wartezeiten einzuhalten sind. Die Inhalatorkomponente soll lageunabhängig betrieben werden können. Das Risiko, daß flüssiges Material - einschließlich flüssige Kondensatrückstände in die Umwelt gelangen oder die Funktion der Inhalatorkomponente beeinträchtigen, soll minimiert werden. Der Verbund soll möglichst kostengünstig hergestellt werden können. Die Inhalatorkomponente soll handlich und ergonomisch ausgestaltet und einfach zu bedienen sein.

Weiters sollen die Eigenschaften des gebildeten Dampf-Luft-Gemisches oder/und Kondensationsaerosols zumindest in gewissen Grenzen beeinflußbar sein - vor allem die Partikelgrößenverteilung des gebildeten Kondensationsaerosols sowie die organoleptischen Wirkungen desselben.

Schließlich soll die Inhalatorkomponente in zwei grundsätzlich verschiedenen Varianten ausgebildet werden, so daß eine Verwendung sowohl in klassischen Inhalatoren als auch in Zug-Inhalatoren möglich ist.

Die Erfindung wird in dem unabhängigen Anspruch 1 definiert. Die abhängigen Ansprüche betreffen bevorzugte Ausführungsformen.

In einer Variante ist der Verbund flächig ausgebildet, und zumindest ein beheizter Abschnitt des Verbundes berührungsfrei in der Kammer angeordnet ist, und die Kapillarstruktur des Dochts im besagten Abschnitt wenigstens auf einer Seite des flächigen Verbundes weitgehend freiliegt. In einer Weiterbildung der Erfindung liegt die Kapillarstruktur des. Dochts im besagten Abschnitt auf beiden Seiten des flächigen Verbundes weitgehend frei. Dadurch, daß die Kapillarstruktur des Dochts im besagten Abschnitt weitgehend freiliegt, kann der gebildete Dampf ungehindert aus dem Docht abströmen, wodurch die Verdampfungsleistung gesteigert bzw. eine Siedekrise im Docht vermieden werden kann.

Begriffserläuterungen:

"Flächiger Verbund" bedeutet, daß das Heizelement und der Docht in derselben Fläche oder/und in zueinander parallellen Flächen angeordnet und miteinander verbunden sind. Der kapillare Transport des flüssigen Materials im flächigen Verbund erfolgt primär in Flächenrichtung.

"Berührungsfrei" bedeutet, daß weder die Kammerwand noch sonstige Strukturelemente der Inhalatorkomponente berührt werden; durch die berührungsfreie Anordnung in der Kammer wird erreicht, daß die Wärmeleitungsverluste des Verbundes in diesem Abschnitt wesentlich herabgesetzt werden, und der Verbund soweit erhitzt wird, daß das im Docht gespeicherte flüssige Material verdampfen kann.

"Kammer" soll auch Kanäle mit einschließen; somit fällt auch ein rohrförmiger Kanal unter den Begriff "Kammer"; ein offenes Rohrende könnte in diesem Fall beispielsweise die Lufteinlaßöffnung bilden.

Der flächige Verbund weist in einer bevorzugten Ausgestaltung eine Dicke kleiner als 0,6mm, und in einer besonders bevorzugten Ausgestaltung eine Dicke kleiner als 0,3mm auf. Diese Dimensionierung hat zur Folge, daß die flächig eingebrachte Wärme durch Wärmeleitung effizient - d.h. bei kleinem Temperaturgradienten der freiliegenden Dochtoberfläche bzw. Kapillarstruktur zufließen kann, wo sie die Verdampfung des flüssigen Materials bewirkt. Bereits im Inneren des Dochts gebildeter Dampf kann außerdem leichter die freiliegende Dochtoberfläche erreichen. Diese Bedingungen ermöglichen eine weitere Steigerung der Verdampfungsleistung und tragen dazu bei, daß das flüssige Material besonders schonend verdampft wird. Es sei angemerkt, daß es sich hierbei nicht bloß um eine einfache Dimensionierung, sondern um ein wesentliches Erfindungsmerkmal handelt. Selbst der Erfinder war überrascht, als er in Experimenten fand, daß flächige Dochte mit einer freiliegenden Dochtoberfläche und einer Dicke <300$\mu$m noch eine Dochtwirkung in Flächenrichtung zeigen.

In einer Variante kann der Verbund plattenförmig, folienförmig, streifenförmig oder bandförmig ausgebildet sein. Diese flächigen Anordnungen machen Herstellverfahren nutzbar, welche eine besonders wirtschaftliche Massenherstellung erlauben.

In einer Variante enthält der flächige Verbund eine der folgenden Strukturen:

Gewebe, offenporige Faserstruktur, offenporige Sinterstruktur, offenporiger Schaum, offenporige Abscheidungsstruktur. Diese Strukturen eignen sich im Besonderen dafür, einen Dochtkörper mit einer hohen Porosität darzustellen. Eine hohe Porosität gewährleistet, daß die vom Heizelement erzeugte Wärme großenteils für die Verdampfung des in den Poren befindlichen flüssigen Materials genutzt, und ein hoher Verdampfer-Wirkungsgrad erzielt werden kann. Konkret kann mit diesen Strukturen eine Porosität größer als 50% realisiert werden. Die offenporige Faserstruktur kann beispielsweise aus einem Vlies bestehen, welches beliebig verdichtet, und zur Verbesserung des Zusammenhalts zusätzlich gesintert werden kann. Die offenporige Sinterstruktur kann beispielsweise aus einem, durch ein Foliengieß-Verfahren hergestellten, körnigen, faserigen oder flockigen Sinterverbund bestehen. Die offenporige Abscheidungsstruktur kann beispielsweise durch ein CVD-Verfahren, PVD-Verfahren, oder durch Flammspritzen erzeugt werden. Offenporige Schäume sind grundsätzlich kommerziell verfügbar und auch in dünner, feinporiger Ausführung erhältlich.

In einer Ausgestaltungsvariante der Erfindung weist der flächige Verbund mindestens zwei Lagen auf, wobei die Lagen mindestens eine der folgenden Strukturen enthalten: Platte, Folie, Papier, Gewebe, offenporige Faserstruktur, offenporige Sinterstruktur, offenporiger Schaum, offenporige Abscheidungsstruktur. Dabei können bestimmte Lagen dem Heizelement, und andere Lagen dem Docht zugeordnet sein. Beispielsweise kann das Heizelement durch einen elektrischen Heizwiderstand bestehend aus einer Metallfolie gebildet werden. Es ist aber auch möglich, daß eine Lage sowohl Heizelement- als auch Dochtfunktionen übernimmt; so kann eine solche Lage aus einem Metalldrahtgewebe bestehen, welches einerseits durch seinen elektrischen Widerstand einen Beitrag zur Beheizung leistet, und andererseits auf das flüssige Material eine Kapillarwirkung ausübt. Die einzelnen Lagen sind vorteilhafterweise, aber nicht notwendigerweise durch eine Wärmebehandlung wie Sintern oder Schweißen miteinander verbunden. Beispielsweise kann der Verbund als Sinterverbund, bestehend aus einer Edelstahlfolie und einer oder mehreren Lagen eines Edelstahl-Drahtgewebes (Material z.B. AISI 304 oder AISI 316) ausgebildet sein. Anstatt Edelstahl können beispielhaft auch Heizleiterlegierungen - insbesondere NiCr-Legierungen und CrFeAl-Legierungen ("Kanthal") Verwendung finden, welche im Vergleich zu Edelstahl einen noch höheren spezifischen elektrischen Widerstand aufweisen. Durch die Wärmebehandlung wird eine materielle Verbindung zwischen den Lagen erzielt, wodurch die Lagen den Kontakt miteinander beibehalten - auch unter widrigen Bedingungen, beispielsweise während der Beheizung durch das Heizelement und dadurch induzierten Wärmedehnungen. Würde der Kontakt zwischen den Lagen verloren gehen, könnte sich ein Spalt bilden, welcher einerseits die kapillare Kopplung und andererseits die Wärmeübertragung vom Heizelement auf das flüssige Material stören könnte.

In einer Variante ist vorgesehen, daß der Verbund linienförmig ausgebildet ist, und zumindest ein beheizter Abschnitt des Verbundes berührungsfrei in der Kammer angeordnet ist, und die Kapillarstruktur des Dochts im besagten Abschnitt weitgehend freiliegt. Dadurch, daß die Kapillarstruktur des Dochts im besagten Abschnitt freiliegt, kann der gebildete Dampf ungehindert aus dem Docht abströmen, wodurch die Verdampfungsleistung gesteigert bzw. eine Siedekrise im Docht vermieden werden kann. Der kapillare Transport des flüssigen Materials im linienförmigen Verbund erfolgt primär in Längsrichtung des linsenförmigen Verbundes. Die Begriffe "berührungsfrei" und "Kammer" wurden bereits früher erläutert.

Der linienförmige Verbund weist vorzugsweise eine Dicke kleiner als 1,0mm auf, wobei die Dicke definiert ist durch: $\sqrt{4*A/\pi}$ (A bezeichnet die Querschnittsfläche des Verbundes). Diese Dimensionierung hat zur Folge, daß die linienförmig eingebrachte Wärme durch Wärmeleitung effizient - d.h. bei kleinem Temperaturgradienten der freiliegenden Dochtoberfläche zufließen kann, wo sie die Verdampfung des flüssigen Materials bewirkt. Bereits im Inneren des Dochts gebildeter Dampf kann außerdem leichter die freiliegende Dochtoberfläche erreichen. Diese Bedingungen ermöglichen eine weitere Steigerung der Verdampfungsleistung.

Der linienförmige Verbund enthält mindestens eine der folgenden Strukturen: Draht, Garn, offenporige Sinterstruktur, offenporiger Schaum, offenporige Abscheidungsstruktur. Diese Strukturen eignen sich im Besonderen dafür, einen linienförmigen Verbund mit einer ausreichenden mechanischen Stabilität und mit einer hohen Porosität darzustellen.

In einer bevorzugten Ausgestaltung des flächigen oder linienförmigen Verbundes ist das Heizelement zumindest teilweise in den Docht integriert. Diese Anordnung hat den vorteilhaften Effekt, daß die Wärme direkt im Dochtkörper erzeugt und freigesetzt wird, und dort unmittelbar auf das zu verdampfende, flüssige Material übertragen wird. Beispielsweise kann das Heizelement aus einer elektrisch leitenden Dünnschicht aus Platin, Nickel, Molybdän, Wolfram, Tantal bestehen, welche Dünnschicht durch ein PVD- oder CVD-Verfahren auf die Dochtoberfläche aufgebracht wird. Der Docht besteht in diesem Fall aus einem elektrisch nicht leitenden Material - z.B. aus Quarzglas. In einer herstellungstechnisch einfacheren Ausgestaltung der Erfindung besteht der Docht selbst zumindest teilweise aus einem elektrischen Widerstandsmaterial, beispielsweise aus Kohlenstoff, aus einer elektrisch leitenden oder halbleitenden Keramik oder aus einem PTC-Material. Besonders günstig ist es, wenn das elektrische Widerstandsmaterial metallisch ist. Metalle weisen im Vergleich zu den zuvor genannten Materialien eine höhere Duktilität auf. Diese Eigenschaft erweist sich insofern als vorteilhaft, als der Verbund im Betrieb einer thermischen Wechselbelastung ausgesetzt ist, wodurch Wärmedehnungen induziert werden. Metalle können solche Wärmedehnungen besser kompensieren. Darüberhinaus weisen Metalle im Vergleich eine höhere Schlagzähigkeit auf. Diese Eigenschaft erweist sich dann als Vorteil, wenn die Inhalatorkomponente Stößen ausgesetzt ist. Geeignete metallische Widerstandsmaterialien sind beispielsweise: Edelstähle wie AISI 304 oder AISI 316 sowie Heizleiterlegierungen - insbesondere NiCr-Legierungen und CrFeAl-Legierungen ("Kanthal") wie DIN-Werkstoff-Nummer 2.4658, 2.4867, 2.4869, 2.4872, 1.4843, 1.4860, 1.4725, 1.4765, 1.4767.

In einer weiteren bevorzugten Ausgestaltung des flächigen oder linienförmigen Verbundes ist vorgesehen, daß die Verbindung zwischen dem Heizelement und dem Docht sich über die gesamte Ausdehnung des Dochts erstreckt. Dabei ist es unerheblich, ob das Heizelement auch über seine ganze Ausdehnung als solches genutzt - d.h. beheizt wird, oder nur abschnittsweise. Dies hängt von der jeweiligen Position der elektrischen Kontaktierung des Heizelements ab. Auch wenn diese Kontaktierung an den äußeren Enden des Heizelements erfolgt, muß das Heizelement nicht zwangsweise über seine gesamte Ausdehnung zur Verdampfung des flüssigen Materials beitragen. So kann das Heizelement abschnittsweise Strukturkomponenten berühren, welche die im Heizelement erzeugte Wärme weitgehend ableiten, so daß das flüssige Material im Docht zumindest in diesem Abschnitt praktisch nicht erwärmt wird. Diese abfließende Wärme wäre allerdings in der Energiebilanz als Verlust zu werten. Durch diese Ausgestaltung werden Herstellverfahren anwendbar, welche gegenüber dem Stand der Technik deutliche Kostenvorteile bieten und eine Massenherstellung erst wirtschaftlich machen. So kann der flächige Verbund in großer Stückzahl aus einem flächigen Vielfachnutzen gewonnen werden, indem der Verbund durch geeignete Trennverfahren wie Stanzen oder Laserschneiden aus diesem Vielfachnutzen gelöst wird. Der linienförmige Verbund kann vorteilhafterweise aus einem Endlosmaterial gewonnen werden. Der Begriff "Endlosmaterial" schließt auch ein Material mit einer endlichen Länge mitein, sofern diese Länge um ein Vielfaches größer ist als die Länge des linienförmigen Verbundes.

Wie bereits früher ausgeführt wurde, ist eine hohe Porosität des Dochts bzw. des Verbundes im Hinblick auf eine effektive Nutzung der vom Heizelement eingebrachten Wärmeenergie wünschenswert. Die Porosität kann zusätzlich gesteigert werden, indem der Verbund oder seine Produktions-Vorstufe - z.B. der Vielfachnutzen - geätzt wird. Beispielhaft kann ein Sinterverbund bestehend aus einer Edelstahlfolie und einer oder mehreren Lagen eines Edelstahlgewebes (z.B. AISI 304, AISI 316) in einem wässrigen Beizbad bestehend aus 50% Salpetersäure und 13% Flussäure entsprechend behandelt werden, wobei als Nebeneffekt auch der elektrische Widerstand des Heizelements bzw. Verbundes beeinflußt, nämlich vergrößert werden kann.

Bevorzugt kann die Oberfläche des Verbundes oder seiner Produktions-Vorstufe außerdem aktiviert werden. Diese Maßnahme schließt auch eine Reinigung der Oberfläche ein und bewirkt eine bessere Benetzung des Verbundmaterials durch das flüssige Material und damit verbunden eine schnellere Infiltration des Dochts. Für den zuvor beispielhaft angeführten Sinterverbund bestehend aus einer Edelstahlfolie und einer oder mehreren Lagen eines Edelstahlgewebes eignet sich beispielsweise eine Behandlung in einer 20%-igen Phosphorsäure sehr gut, um die zuvor erwähnten Effekte zu erzielen.

In einer vorteilhaften Ausgestaltung ist der Docht als arterieller Docht ausgebildet. Dieser Dochttyp findet vor allem in Wärmerohren Verwendung und ist in der einschlägigen Literatur genauer beschrieben - siehe z.B. ISBN 0080419038. Ein solcher Docht kann beispielsweise aus einem Bündel von Kanälen oder Kapillaren - sog. "Arterien" - bestehen, welche von einer feineren Porenstruktur umgeben sind bzw. von dieser gebildet werden. Im Vergleich zu einer homogenen Porenstruktur gleicher Kapillarität bzw. gleichen Kapillardrucks (kapillare Steighöhe) setzt das Bündel aus Kanälen oder Kapillaren dem flüssigen Material einen geringeren Strömungswiderstand entgegen, wodurch die Infiltration des Dochts mit dem flüssigen Material wesentlich beschleunigt werden kann.

In einer Ausgestaltungsvariante ist der Docht in Dickenrichtung gelocht. Die Lochung kann beispielsweise mittels Laser erfolgen und hat folgende Effekte: zum einen wird die Porosität weiter gesteigert; zum anderen wird der Strömungswiderstand in Dickenrichtung reduziert. Der letztere Effekt tritt insbesondere bei Verwendung eines arteriellen Dochts in Erscheinung, insofern, als das flüssige Material im Docht während der Verdampfung eine Drucksteigerung erfährt, und die Lochung als Druckentlastung wirkt. Dadurch wird vermieden, daß der im Docht gebildete Dampf das flüssige Material über die Arterien zurück zur Quelle des flüssigen Materials drückt, wodurch die Versorgung mit flüssigem Material empfindlich gestört werden kann.

Es wird ferner als bevorzugt angesehen, daß der flächige Verbund im Wesentlichen plan ausgebildet ist, und die Lufteinlaßöffnung als schlitzförmiger Kanal ausgebildet ist, und der schlitzförmige Kanal parallel zur planen Verbundfläche ausgerichtet ist. Es wird ferner als bevorzugt angesehen, daß der linienförmige Verbund im Wesentlichen geradlinig ausgebildet ist, und die Lufteinlaßöffnung als schlitzförmiger Kanal ausgebildet ist, und der schlitzförmige Kanal parallel zum geradlinigen Verbund ausgerichtet ist. Durch diese geometrisch einfachen Anordnungen können sehr günstige Mischungsbedingungen zwischen der einströmenden Luft und dem aus dem Docht austretenden Dampf geschaffen werden, welche Mischungsbedingungen darüber hinaus durch eine Änderung der Position des schlitzförmigen Kanals oder/und durch eine Änderung der Schlitzhöhe auf einfache Art variiert werden können; auf diese Weise ist es möglich, auf die Eigenschaften des gebildeten Aerosols - insbesondere auf die Größe der gebildeten Aerosolteilchen in gewissem Maße Einfluß zu nehmen.

Erfindungsgemäß ist vorgesehen, daß der Verbund die Kammer brückenartig durchsetzt und mit zwei Endabschnitten auf zwei elektrisch leitenden, plattenförmigen Kontakten lagert, und das Heizelement mit den Kontakten elektrisch kontaktiert ist. Berücksichtigt man, daß es sich beim Verbund um ein ausgesprochen kleines und mechanisch empfindliches Bauteil handelt, welches außerdem den Strömungskräften der in die Kammer einströmenden Luft sowie Kräften zufolge thermischer Ausdehnung ausgesetzt ist, wird klar, daß die soeben beschriebene Anordnung eine verhältnismäßig stabile und herstellungstechnisch einfache Verankerung und Kontaktierung des Verbundes ermöglicht. Die elektrische Kontaktierung des Heizelements besteht in einer bevorzugten Ausgestaltung der Erfindung aus einer Schweiß- oder Sinterungverbindung. Die Schweißverbindung kann durch Punktschweißen, Widerstandsschweißen, Ultraschallschweißen, Laserschweißen, Bonden oder sonstige geeignete Schweißverfahren hergestellt werden. Besonders günstig für die Verschweißung oder Versinterung ist es, wenn die plattenförmigen Kontakte aus demselben oder aus einem ähnlichen Material wie das Heizelement bestehen. In einer anderen vorteilhaften Ausgestaltung der Erfindung besteht die elektrische Kontaktierung des Heizelements aus einer Klebeverbindung mittels eines elektrisch leitenden Klebstoffes, zum Beispiel mittels eines silberhaltigen Klebers auf Epoxidbasis. In diesem Fall können die plattenförmigen Kontakte im Prinzip aus jedem beliebigen elektrischen Kontaktwerkstoff hergestellt werden, solange der Werkstoff mit dem verwendeten Klebstoff kompatibel ist; alternativ können die plattenförmigen Kontakte auch durch Leiterplatten oder eine gemeinsame Leiterplatte gebildet werden. Dickkupfer-Leiterplatten mit Kupfer-Schichtstärken im Bereich von 100-500 $\mu$m erhalten wegen der besseren Wärmeableitung den Vorzug. Selbstverständlich ist die Erfindung nicht auf die zuvor genannten Kontaktierungsverfahren beschränkt. So könnte die elektrische Kontaktierung alternativ auch durch eine mechanische Klemmung erfolgen. In einer Weiterbildung der Erfindung ragen die plattenförmigen Kontakte aus der äußeren Oberfläche des Gehäuses in Form zweier Steckkontakte heraus. Die beiden Steckkontakte sind dazu vorgesehen, dem Heizelement die erforderliche elektrische Energie zuzuführen.

In einer bevorzugten Ausbildung ragt der Verbund mit einem Ende in einen Kapillarspalt, dessen Strömungswiderstand kleiner ist als der Strömungswiderstand des Dochts. Der Kapillarspalt speist den Docht mit flüssigem Material; der im Vergleich zum Docht reduzierte Strömungswiderstand bewirkt, daß das flüssige Material schneller zur Verdampfungszone im Verbund gelangt. Dadurch verkürzt sich aber auch die Zeit, welche erforderlich ist, um den Docht nach einer Verdampfung von neuem vollständig mit flüssigem Material zu infiltrieren. Diese Zeit entspricht einer Wartezeit, welche zwischen zwei Zügen oder Inhalationen mindestens einzuhalten ist. Wird diese Wartezeit nicht eingehalten, kann dies zu einer Verringerung der emittierten Dampfmenge bzw. Arzneimittel-Dosis führen. Außerdem kann es dadurch, daß der Verbund abschnittsweise ohne flüssiges Material aufgeheizt wird, zu lokalen Überhitzungen kommen, welche den Verbund schädigen oder seine Lebensdauer verkürzen. In einer Weiterbildung der Erfindung ist vorgesehen, daß der Querschnitt des Kapillarspalts größer ist als der Querschnitt des Verbundes. Dies hat den Effekt, daß das flüssige Material den Docht teilweise Bypass-artig umgeht und auf diese Weise noch schneller zur Verdampfungszone im Verbund gelangt. In einer bevorzugten Ausgestaltung der Erfindung ist das Heizelement des Verbundes im Kapillarspalt elektrisch kontaktiert. Dadurch wird eine sehr platzsparende Anordnung erzielt.

Eine bevorzugte Ausführungsform betrifft eine Inhalatorkomponente mit einem im Gehäuse angeordneten oder mit dem Gehäuse verbundenen, das flüssige Material enthaltenden Flüssigkeitsbehälter samt einem öffenbaren Verschluß; erfindungsgemäß ist vorgesehen, daß der Flüssigkeitsbehälter aus dem Gehäuse weder entnehmbar noch vom Gehäuse trennbar ist, und das flüssige Material im Flüssigkeitsbehälter durch manuelles Öffnen des öffenbaren Verschlusses mit dem Kapillarspalt kapillar koppelbar ist. Der Flüssigkeitsbehälter kann also vom Benutzer aus der Inhalatorkomponente nicht entfernt werden, auch wenn das flüssige Material aufgebraucht ist, was insbesondere dann als Sicherheitsvorteil zu werten ist, wenn der Behälter Arzneimittel oder/ und Gifte wie beispielsweise Nikotin enthält. Das Gehäuse der Inhalatorkomponente ist zu groß, um von Kleinkindern verschluckt zu werden. Eine Wiederbefüllung des Flüssigkeitsbehälters ist nicht vorgesehen; vielmehr bildet die Inhalatorkomponente zusammen mit dem Flüssigkeitsbehälter einen Einwegartikel, welcher nach Aufbrauch des flüssigen Materials sachgerecht zu entsorgen ist. Das flüssige Material wird im Flüssigkeitsbehälter hermetisch dicht verwahrt. Ein Zutritt von Luft oder UV-Strahlen ist weitestgehend ausgeschlossen. Der Flüssigkeitsbehälter kann außerdem ein Schutzgas wie Argon, Stickstoff oder Kohlendioxid enthalten, welches das flüssige Material zusätzlich vor Oxidation schützt. Der öffenbare Verschluß des Flüssigkeitsbehälters wird zweckmäßigerweise erst kurz vor dem Gebrauch der Inhalatorkomponente geöffnet, wonach das flüssige Material über den Kapillarspalt zum Docht gelangt und diesen infiltriert. Das Öffnen des öffenbaren Verschlusses erfolgt auf einfache Weise manuell ohne Zuhilfenahme besonderer Hilfsmittel.

In einer ersten Ausgestaltungsvariante ist der Flüssigkeitsbehälter mit dem Gehäuse starr und permanent verbunden, oder bildet selbst einen Teil des Gehäuses. Der Flüssigkeitsbehälter kann beispielsweise als ein separates Teil ausgebildet sein, welches mit dem Gehäuse durch eine Klebeverbindung oder eine Schweißverbindung untrennbar verbunden ist. In einer Weiterbildung der ersten Ausgestaltungsvariante ist ein mit dem Kapillarspalt kommunizierendes Reservoir vorgesehen, welches an den Flüssigkeitsbehälter anschließt und von diesem durch den öffenbaren Verschluß getrennt ist. Das Reservoir dient dazu, bei geöffnetem Verschluß zumindest einen Teil des flüssigen Materials aus dem Flüssigkeitsbehälter aufzunehmen und die kapillare Kopplung mit dem Kapillarspalt zu gewährleisten. Geöffnet wird der öffenbare Verschluß vorzugsweise durch einen im Gehäuse axial verschiebbar gelagerten Stift, dessen erstes Ende gegen den öffenbaren Verschluß gerichtet ist, und dessen zweites Ende bei geschlossenem Verschluss aus der äußeren Oberfläche des Gehäuses fortsatzartig herausragt, indem auf das zweite Ende des Stiftes eine Druckkraft ausgeübt wird. Die Druckkraft wird vom Stift auf den öffenbaren Verschluß übertragen, wodurch dieser schließlich entlang einer Sollbruchstelle aufreißt. Die Druckkraft kann beispielsweise durch Fingerdruck erzeugt werden. Eine besonders günstige Ausgestaltung der Erfindung betrifft einen Inhalator, umfassend eine Inhalatorkomponente wie soeben beschrieben sowie ein wiederverwendbares Inhalatorteil, welches mit der Inhalatorkomponente koppelbar ist; erfindungsgemäß ist vorgesehen, daß das zweite Ende des Stiftes mit dem wiederverwendbaren Inhalatorteil während der Kopplung in einer stößelartigen Wirkverbindung steht, wodurch die zuvor beschriebene Druckkraft erzeugt wird. Das Koppeln der Inhalatorkomponente mit dem wiederverwendbaren Inhalatorteil und das Öffnen des Flüssigkeitsbehälters geschehen also gleichzeitig durch eine einzige Manipulation.

In einer Ausgestaltung kommuniziert das Reservoir über einen Belüftungskanal mit der Kammer, wodurch Luft in das Reservoir gelangt, und ein Druckausgleich bewirkt wird. Auf diese Weise wird jede Portion flüssigen Materials, welche in den Kapillarspalt gelangt, unmittelbar durch eine volumengleiche Portion Luft ersetzt. Wesentlich ist, daß der Belüftungskanal mit der Kammer verbunden ist, und nicht mit der äußeren Umgebung kommuniziert, da andernfalls der Saugdruck während einer Inhalation die Kapillarströmung überlagern würde, und flüssiges Material nach dem Strohhalm-Prinzip aus dem Flüssigkeitsbehälter gesaugt würde.

In einer zweiten Ausgestaltungsvariante ist der Flüssigkeitsbehälter im Gehäuse längs einer Verschiebeachse zwischen zwei Anschlagpositionen manuell verschiebbar angeordnet, und der Flüssigkeitsbehälter wirkt in der ersten Anschlagposition mit einer nicht entriegelbaren Blockiervorrichtung und in der zweiten Anschlagposition mit einem den öffenbaren Verschluß öffnenden Öffnungsmittel zusammen. Durch die Blockiervorrichtung wird eine Entnahme des Flüssigkeitsbehälters aus dem Gehäuse grundsätzlich verhindert. Der Flüssigkeitsbehälter kann also wie bei der ersten Ausgestaltungsvariante nicht aus dem Gehäuse entfernt werden - mit denselben Sicherheitsvorteilen wie bereits früher beschrieben. In einer Weiterbildung der zweiten Ausgestaltungsvariante umfaßt das Öffnungsmittel einen vom Kapillarspalt ausgebildeten ersten Dorn, welcher den öffenbaren Verschluß in der zweiten Anschlagposition durchdringt, wodurch die kapillare Kopplung mit dem flüssigen Material hergestellt wird. Weiters ist wieder ein Belüftungskanal vorgesehen, dessen erstes Ende mit der Kammer kommuniziert, und dessen zweites Ende als zweiter Dorn ausgebildet ist, welcher den öffenbaren Verschluß in der zweiten Anschlagposition durchdringt. Der erste und der zweite Dorn bilden also zusammen das Öffnungsmittel. Die Wirkung dieser Anordnung ist ähnlich der einer Kopplung zwischen einem Füllhalter und dessen Tintenpatrone. Selbstverständlich können der erste und der zweite Dorn auch zu einem einzigen gemeinsamen Dorn fusioniert sein. Die nicht entriegelbare Blockiervorrichtung kann in einfacher Weise aus einem beispielsweise vom Gehäuse oder vom Mundstück ausgebildeten Vorsprung bestehen, gegen den der Flüssigkeitsbehälter in der ersten Anschlagposition stößt. Schließlich bezieht sich die zweite Ausgestaltungsvariante auf eine Inhalatorkomponente, umfassend ein Mundstück mit einem Mundstückkanal, durch welchen ein Benutzer das Dampf-Luft-Gemisch oder/und Kondensationsaerosol dargeboten erhält, und es ist nach der Erfindung vorgesehen, daß die Verschiebeachse zumindest annähernd parallel zur Mittelachse des Mundstückkanals ausgerichtet ist, und der Flüssigkeitsbehälter zumindest in der ersten Anschlagposition mit einem Endabschnitt seitlich neben dem Mundstück aus dem Gehäuse ragt. Der verschiebbare Flüssigkeitsbehälter kann auf einfache Weise in seine zweite Anschlagposition verschoben werden, indem der Benutzer auf das herausragende Ende des Flüssigkeitsbehälters drückt. Das Mundstück und der Flüssigkeitsbehälter ragen auf derselben Stirnseite der Inhalatorkomponente aus dem Gehäuse, was die Inhalatorkomponente handlich und die Benutzung derselben ergonomisch macht.

In einer Ausgestaltungsvariante kann ferner ein Pufferspeicher vorgesehen sein, welcher mit dem Kapillarspalt kommuniziert und selbst aus Kapillaren besteht. Der Pufferspeicher hat die Fähigkeit, flüssiges Material aus dem Kapillarspalt aufzunehmen, und bei Bedarf das gepufferte flüssige Material lageunabhängig an den Docht über den Kapillarspalt wieder abzugeben. Dadurch kann die Inhalatorkomponente in jeder beliebigen Lage betrieben werden, zumindest solange wie flüssiges Material im Pufferspeicher vorrätig ist. Die Kapillaren können beispielsweise aus Schlitzen, Löchern, oder aus einem porösen Material bestehen, wobei darauf zu achten ist, daß deren Kapillarität bzw. Kapillardruck (kapillare Steighöhe) kleiner ist wie die Kapillarität des Dochts, da ansonsten keine kapillare Strömung zustande kommt.

Alternativ zu dem zuvor beschriebenen Flüssigkeitsbehälter kann die Inhalatorkomponente einen aus einem elastischen, offenporigen Material bestehenden und mit dem flüssigen Material getränkten Flüssigkeitsspeicher beinhalten; bevorzugt ist vorgesehen, daß der Verbund sandwichartig zwischen einem der beiden plattenförmigen Kontakte - wie zuvor bereits beschrieben - einerseits, und dem Flüssigkeitsspeicher andererseits eingeklemmt ist, wodurch der Docht mit dem flüssigen Material im Flüssigkeitsspeicher kapillar gekoppelt wird. Das elastische, offenporige Material kann beispielsweise aus einem Fasermaterial oder aus Schaumstoff bestehen. Das flüssige Material wird selbsttätig aus dem Flüssigkeitsspeicher in den Docht gesaugt und infiltriert diesen. Voraussetzung ist, daß die Kapillarität bzw. der Kapillardruck (kapillare Steighöhe) des Dochts größer ist wie die Kapillarität des Flüssigkeitsspeichers. Die sandwichartige Klemmung stellt eine konstruktiv einfache und in ihrer Herstellung kostengünstige Anordnung dar.

In einer Weiterbildung beinhaltet die Inhalatorkomponente eine Kondensatbindeeinrichtung zur Aufnahme und Speicherung von Kondensatrückständen, welche im Zuge der Erzeugung des Dampf-Luft-Gemisches oder/und Kondensationsaerosols gebildet werden; vor allem, wenn das zu verdampfende flüssige Material größere Anteile an niedrig siedenden Fraktionen mit hohem Dampfdruck enthält, z.B. Ethanol oder/und Wasser, können erhebliche Mengen an Kondensatrückständen anfallen. Solche Anteile an niedrig siedenden Fraktionen sind vor allem aus zwei Gründen vorteilhaft und im Fall der erfindungsgemäßen Inhalatorkomponente auch notwendig: zum einen verringern solche Anteile die Zähigkeit des flüssigen Materials, wodurch das flüssige Material den Docht schneller infiltrieren kann. Diese Wirkung erweist sich bei dem Verbund als besonders vorteilhaft, da die Dicke des Verbundes, und dadurch bedingt auch der mittlere Porendurchmesser des Dochts ausgesprochen klein sind. Zum anderen bewirken die niedrig siedenden Fraktionen, daß im flüssigen Material enthaltene Arzneimittel und andere Zusatzstoffe leichter verdampfen, sich weniger Verdampfungsrückstände bilden, und die thermische Zersetzung des flüssigen Materials reduziert wird. Um diese positiven Wirkungen in einem zufriedenstellenden Ausmaß nutzbar zu machen, sollte der Massenanteil der niedrig siedenden Fraktionen deutlich über 50% liegen. Folgedessen sind im Betrieb der erfindungsgemäßen Inhalatorkomponente erhebliche Mengen an Kondensatrückständen zu erwarten, welche zweckmäßig gebunden werden müssen.

Bevorzugt besteht die Kondensatbindeeinrichtung aus einem offenporigen, saugfähigen Körper, welcher beabstandet, aber in unmittelbarer Nähe zur im besagten Abschnitt freiliegenden Kapillarstruktur des Dochts angeordnet ist. Der offenporige, saugfähige Körper nimmt in seinen Poren aus der Dampfphase gebildete Kondensatablagerungen auf und wirkt insofern vom Prinzip her ähnlich wie ein Schwamm. Auch eine größere Kondensatmenge kann problemlos gebunden werden. Der offenporige, saugfähige Körper verhindert, daß sich in der Inhalatorkomponente insbesondere in der Kammer frei bewegliche Kondensatansammlungen bilden, welche die Funktion der Inhalatorkomponente beeinträchtigen können, aber auch ein Risiko für den Benutzer und die Umwelt darstellen, sofern diese Ansammlungen Arzneimittelreste oder Gifte wie Nikotin enthalten. Durch die spezielle Anordnung des offenporigen, saugfähigen Körpers in unmittelbarer Nähe zur Dampfbildungszone - d.h. in einem Bereich hoher Dampfdichte - wird bewirkt, daß die Kondensatrückstände in sehr hoher Konzentration und damit sehr effektiv absorbiert werden, und ihnen gar nicht erst die Gelegenheit geboten wird, in periphere Bereiche zu streuen. Besonders günstig ist es, wenn der offenporige, saugfähige Körper die im besagten Abschnitt freiliegende Kapillarstruktur des Dochts direkt überdeckt, da in dieser Zone die höchste Dampfdichte zu erwarten ist. In einer vorteilhaften Ausgestaltung umfaßt der offenporige, saugfähige Körper zwei zueinander beabstandet angeordnete Teile oder Abschnitte, und der Verbund ist zumindest abschnittsweise zwischen den beiden Teilen oder Abschnitten angeordnet. Des Weiteren gilt als bevorzugt, daß der offenporige, saugfähige Körper in der Kammer angeordnet ist und den überwiegenden Teil der Kammer ausfüllt. Auf diese Weise kann bei kompakter Bauweise eine besonders große Aufnahmekapazität für die flüssigen Kondensatrückstände realisiert werden. Günstig ist ferner, wenn der offenporige, saugfähige Körper aus einem formbeständigen Material besteht, welches auch nach vollständiger Infiltration mit den Kondensatrückständen seine Form weitgehend beibehält. Um festzustellen, ob ein konkretes Material formbeständig ist, reicht es aus, dieses mit einer Ethanol-Wasser-Lösung zu tränken, und nach drei Tagen Verweildauer die Formbeständigkeit zu überprüfen. Die Formbeständigkeit gewährleistet, daß die Strömungsverhältnisse in der Kammer, insbesondere um den Verbund, und damit die Bedingungen für die Bildung des Dampf-Luft-Gemisches oder/und Kondensationsaerosols konstant bleiben. Beispielhaft kann der offenporige, saugfähige Körper aus einem festen schaumartigen Material wie Metallschaum oder Keramikschaum, aus einem porösen Sinterformkörper, aus einem porösen Füll- oder Schüttmaterial ohne Blähneigung, beispielsweise aus einer Trocknungsmittel-Granulat-Schüttung, oder aus einem porösen Faserverbund, beispielsweise gebildet aus thermisch oder mit Hilfe eines Bindemittels miteinander verbundenen Natur- oder Chemiefasern, bestehen. Wesentlich ist außerdem, daß das Material gegenüber den Kondensatrückständen weitestgehend chemisch inert ist.

Gemäß einer bevorzugten Ausführungsform wird der offenporige, saugfähige Körper vom Gehäuse weitgehend umschlossen und ist mit dem Gehäuse untrennbar verbunden. Damit soll erreicht werden, daß der offenporige, saugfähige Körper nicht direkt mit der Umwelt in Kontakt kommen kann, und eine Entfernung desselben aus dem Gehäuse nur durch Gewalteinwirkung und Zerstörung der Inhalatorkomponente möglich ist. Diese Schutzmaßnahme erweist sich vor allem dann als vorteilhaft, wenn das Kondensat Arzneimittelreste oder/und Gifte wie Nikotin enthält. Die Inhalatorkomponente bildet zusammen mit dem offenporigen, saugfähigen Körper einen Einwegartikel, welcher nach Erreichen der vorgesehenen Lebensdauer sachgerecht zu entsorgen ist.

In einer vorteilhaften Weiterbildung ist eine zweistufige Kondensat-Abscheidevorrichtung vorgesehen, bestehend erstens aus dem offenporigen, saugfähigen Körper und zweitens aus einem vom gebildeten Dampf-Luft-Gemisch oder/und Kondensationsaerosol durchströmbaren Kühler. Diese Weiterbildung eignet sich insbesondere dafür, in Zug-Inhalatoren verwendet zu werden. Der Kühler kühlt das durchströmende Dampf-Luft-Gemisch oder/und Kondensationsaerosol und entzieht ihm hierbei noch weiteres Kondensat. Der Kühler kann beispielsweise durch einen durchströmbaren und für die Partikel des gebildeten Kondensationsaerosols weitgehend durchlässigen Porenkörper gebildet werden. Der Porenkörper bewirkt neben der Kühlung auch eine innige Durchmischung des durchströmenden Dampf-Luft-Gemisches bzw. Kondensationsaerosols, wodurch dessen Eigenschaften homogenisiert werden, zum Beispiel Konzentrationsspitzen abgebaut werden. Der Porenkörper besteht typischerweise aus einem weitporigen Material, beispielsweise aus einem offenzelligen Schaummaterial, aus einem grobporigen, porösen Füllmaterial oder aus einem vliesartigen Fasermaterial. Als Beispiel für ein vliesartiges Fasermaterial seien aus Polyolefinfasern (PE, PP) oder Polyesterfasern gefertigte Synthetikfaservliese genannt. Der Porenkörper kann auch aus einem Regeneratormaterial bestehen. Das Regeneratormaterial ist in der Lage, bei einer großen Oberfläche bzw. Wärmeaustauschfläche ohne wesentliche Strömungsverluste schnell und viel Wärme aufzunehmen. Typische Regeneratormaterialien sind: Metallwolle, Metallspäne, Metallgewebe, Drahtgestricke, Metallfaservliese, offenzellige Metallschäume, Schüttungen aus metallischem oder keramischem Granulat. Schließlich kann der Kühler auch mehrstufig aufgebaut sein, indem verschiedene poröse Materialien miteinander kombiniert werden. Selbstverständlich ist die Erfindung nicht auf die zuvor aufgezählten Kühler-Materialien beschränkt. Durch die Kühlung und Homogenisierung können die organoleptischen Eigenschaften des vom Benutzer aufgenommenen Dampf-Luft-Gemisches oder/und Kondensationsaerosols deutlich verbessert werden.

In einer besonders bevorzugten Ausgestaltung wird der Kühler durch eine Tabakfüllung gebildet. Die Tabakfüllung bewirkt neben der Kühlung/ Kondensation und Homogenisierung zusätzlich eine Aromatisierung des durch sie hindurchströmenden Dampf-Luft-Gemisches bzw. Kondensationsaerosols und bietet sich vor allem dann an, wenn das flüssige Material Nikotin als Arzneimittel enthält. Bei labormäßigen Versuchen mit nach dem Zug-Inhalator-Prinzip arbeitenden Prototypen und mit nikotinhaltigen Arzneimittel-Zubereitungen als flüssiges Material wurden darüberhinaus noch weitere günstige Effekte festgestellt: beispielsweise konnte die Inhalierbarkeit des nikotinhaltigen Dampf-Luft-Gemisches und Kondensationsaerosols verbessert werden, was zum Teil gewiß auf die oben beschriebenen Effekte zurückzuführen ist. Es besteht jedoch die Hypothese, daß zusätzliche Wirkmechanismen beteiligt sind - insbesondere Diffusions- und Adsorptionsprozesse das freie, unprotonisierte Nikotin betreffend, welche im Detail noch zu erforschen wären. Die Fülldichte der Tabakfüllung ist nach oben hin dadurch beschränkt, daß die Füllung einerseits für die durchströmenden Aerosolpartikel möglichst durchlässig sein muß, und andererseits der induzierte Strömungswiderstand nicht größer sein sollte als jener von Zigaretten. Die Tabakfüllung kann aus Schnitttabak, Feinschnitt-Tabak, Stopftabak, aus einem zigarrenartigen Tabakwickel oder aus vergleichbaren oder ähnlichen Tabakformen gebildet werden. Als Tabak eignen sich insbesondere getrockneter fermentierter Tabak, rekonstituierter Tabak, expandierter Tabak oder Mischungen derselben. Der Tabak kann zusätzlich gesoßt, gewürzt, aromatisiert oder/und parfümiert werden. Die Verwendung einer Tabakfüllung als Kühler kann außerdem den Umstieg von Tabakwaren auf die erfindungsgemäße Inhalatorkomponente attraktiver machen oder/und erleichtern. In einer bevorzugten Weiterbildung ist vorgesehen, daß das Volumen der Tabakfüllung größer als 3cm3 ist. In eigenen labormäßigen Versuchen hat sich gezeigt, daß die oben genannten Wirkungen der Tabakfüllung erst ab dem zuvor spezifizierten Mindestvolumen in einem für den Benutzer zufriedenstellenden Maß zum Tragen kommen.

Gemäß einer weiteren Ausführungsform umfaßt die Inhalatorkomponente eine von einem Mundstück gebildete Mundstücköffnung, welche mit der Kammer kommuniziert, und durch welche ein Benutzer das Dampf-Luft-Gemisch oder/und Kondensationsaerosol dargeboten erhält, wobei sich im Zuge der Inhalation zwischen der Lufteinlaßöffnung und der Mundstücköffnung eine Strömung in Richtung der Mundstücköffnung ausbildet, welche Strömung zumindest abschnittsweise den Verbund passiert. Es ist auch vorgesehen, daß stromabwärts vom Verbund mindestens eine Luft-Bypassöffnung angeordnet ist, durch welche zusätzlich Luft aus der Umgebung in die Strömung eingespeist wird, und der wirksame Strömungsquerschnitt der Luft-Bypassöffnung mindestens 0,5cm2 beträgt. Diese Anordnung macht die Inhalatorkomponente auch für klassische Inhalatoren verwendbar, welche grundsätzlich einen möglichst kleinen Strömungswiderstand voraussetzen. Die durch die Luft-Bypassöffnung zusätzlich einströmende Luft ("Bypass-Luft") passiert selbst nicht den Verbund und hat dadurch auch keinen unmittelbaren Einfluß auf die Bildung des Dampf-Luft-Gemisches oder/und Kondensationsaerosols bzw. auf dessen Eigenschaften. Es besteht jedoch ein mittelbarer Einfluß insofern, als die Bypass-Luft die durch die Lufteinlaßöffnung einströmende Luftmenge ("Primärluft") reduziert, wenn man eine konstante Inhalationsluftmenge voraussetzt. Auf diese Weise kann die Primärluftmenge beliebig verringert werden. Eine Verringerung der Primärluftmenge führt unter anderem zu einer Vergrößerung der gebildeten Aerosolpartikel; gleichzeitig nimmt allerdings auch die Menge an gebildeten Kondensatrückständen zu, welchem Umstand jedoch durch die Anordnung einer Kondensatbindeeinrichtung - wie zuvor beschrieben - begegnet werden kann. Eine weitere Reduktion des Strömungswiderstandes und eine weitere Verringerung der Primärluftmenge werden erfindungsgemäß dadurch erzielt, daß die Luft-Bypassöffnung aus zwei Bypassöffnungen besteht, welche in gegenüberliegenden Gehäuseabschnitten angeordnet sind.

Es ist ferner vorgesehen, daß an die beiden Bypassöffnungen zwei Leitschaufeln anschließen, welche in Richtung der Mundstücköffnung weisen und aufeinander zustreben, und deren freie Enden eine düsenförmige Mündungsöffnung bilden, durch welche das gebildete Dampf-Luft-Gemisch oder/und Kondensationsaerosol aus der Kammer ausströmt und sich anschließend mit der aus den Bypassöffnungen einströmenden Luft mischt. Die beiden Leitschaufeln haben den Effekt, daß sie die Kammer nach außen hin weitgehend abdecken, wodurch die Gefahr eines Eintritts z.B. von Regenwasser oder Speichel in die Kammer deutlich reduziert wird. Zusätzlich wird auch der Luftaustausch zwischen der Kammer und der Umgebung eingeschränkt, wodurch die natürliche Verdunstung von Anteilen des flüssigen Materials im Docht vermindert wird. Eine solche Verdunstung kann sich insbesondere während längerer Perioden der Nichtbenutzung der Inhalatorkomponente insofern als ungünstig erweisen, als sich die Zusammensetzung des flüssigen Materials ändern kann, und im Fall von Arzneimitteln deren Dosierung vom Zielwert abweichen kann.

Es wird außerdem als bevorzugt betrachtet, daß stromabwärts der Luft-Bypassöffnung ein Strömungshomogenisator angeordnet ist, dessen Strömungswiderstand kleiner als 1 mbar bei einem Luftdurchsatz von 250 mL/sec ist. Der Strömungshomogenisator wird sowohl vom gebildeten Dampf-Luft-Gemisch oder/und Kondensationsaerosol als auch von der durch die Luft-Bypassöffnung einströmenden Bypass-Luft durchströmt und bewirkt eine Durchmischung und Homogenisierung dieser beiden Strömungsanteile. Konzentrationsspitzen werden abgebaut, und das aus der Mundstücköffnung austretende, homogenisierte Gemisch ist für den Benutzer angenehmer zu inhalieren. Der Strömungshomogenisator kann beispielhaft aus einem vlies- oder schaumartigen Material bestehen; ein solches Material ist geeignet, ausreichend Strömungsturbulenzen und Verwirbelungen zu erzeugen, ohne den angeführten Grenzwert für den Strömungswiderstand zu überschreiten. Nur so ist die soeben beschriebene Ausgestaltung für einen klassischen Inhalator nutzbar. In einer optionalen Ausgestaltung sind mehrere nebeneinander angeordnete Verbunde mit unterschiedlichen Wärmekapazitäten vorgesehen. In einer weiteren optionalen Ausgestaltung sind mehrere nebeneinander angeordnete Verbunde mit unterschiedlichen Heizelement-Eigenschaften vorgesehen. In einer weiteren optionalen Ausgestaltung sind mehrere nebeneinander angeordnete Verbunde mit unterschiedlich ansteuerbaren elektrischen Heizelementen vorgesehen. In einer weiteren optionalen Ausgestaltung sind mehrere nebeneinander angeordnete Verbunde vorgesehen, und den einzelnen Verbunden flüssige Materialen unterschiedlicher Zusammensetzung zur Verdampfung zugeordnet, indem deren Dochte von Quellen mit unterschiedlichem flüssigen Material gespeist werden. Die vorgenannten Ausgestaltungsoptionen, welche im Übrigen auch beliebig miteinander kombiniert werden können, ermöglichen es, den Verdampfungsprozeß räumlich wie zeitlich variabler zu gestalten. Diese Variabilität erlaubt es, selbst die komplexen Verhältnisse in der Destillationszone einer Zigarette annähernd nachzubilden.

In einer speziellen Ausgestaltung sind mehrere nebeneinander angeordnete Verbunde vorgesehen, deren Heizelemente aus elektrischen Heizwiderständen bestehen; erfindungsgemäß sind die Heizwiderstände miteinander in Reihe geschaltet. Diese spezielle Ausgestaltung erweist sich als besonders vorteilhaft, wenn die Heizwiderstände aus einem metallischen Widerstandsmaterial wie beispielsweise Edelstahl oder Heizleiterlegierungen bestehen, da durch die Reihenschaltung und durch die mit dieser einhergehenden Widerstanderhöhung der Heizstrom auf ein Maß begrenzt werden kann, welches von der elektronischen Ansteuerung und vom Energiespeicher noch gut beherrschbar ist. Durch die Widerstanderhöhung kann ferner die Leistungsdichte im Verbund nach Bedarf gedrosselt werden, sodaß in jedem Fall eine stabile Verdampfung gewährleistet werden kann.

Zweckmässige und vorteilhafte Ausführungsbeispiele der Erfindung sind in den Zeichnungen dargestellt und werden in der nachfolgenden Beschreibung näher erläutert.

Es zeigen:

Fig.1 einen erfindungsgemäßen Inhalator in einer ersten Ausführungsform, ausgebildet als Zug-Inhalator, in verschiedenen Ansichten;

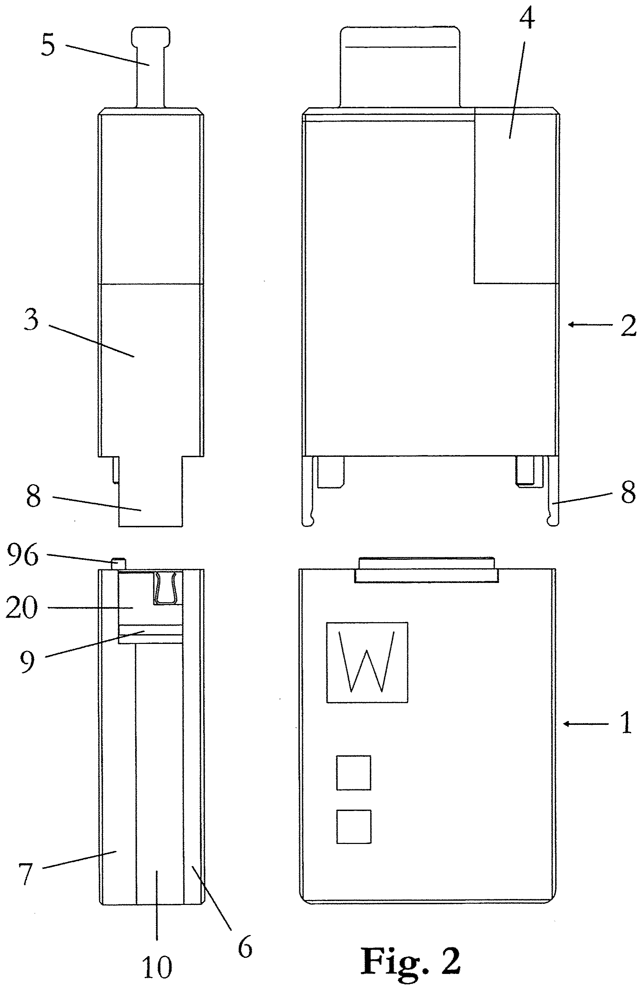

Fig. 2 einen Inhalator nach Fig. 1 mit einem wiederverwendbaren Inhalatorteil und einer auswechselbaren Inhalatorkomponente im entkoppelten Zustand;

Fig. 3 das wiederverwendbare Inhalatorteil in verschiedenen Ansichten;

Fig. 4 und Fig. 5 das wiederverwendbare Inhalatorteil ohne Batteriedeckel und ohne Schaltkreisdeckel in verschiedenen Ansichten;

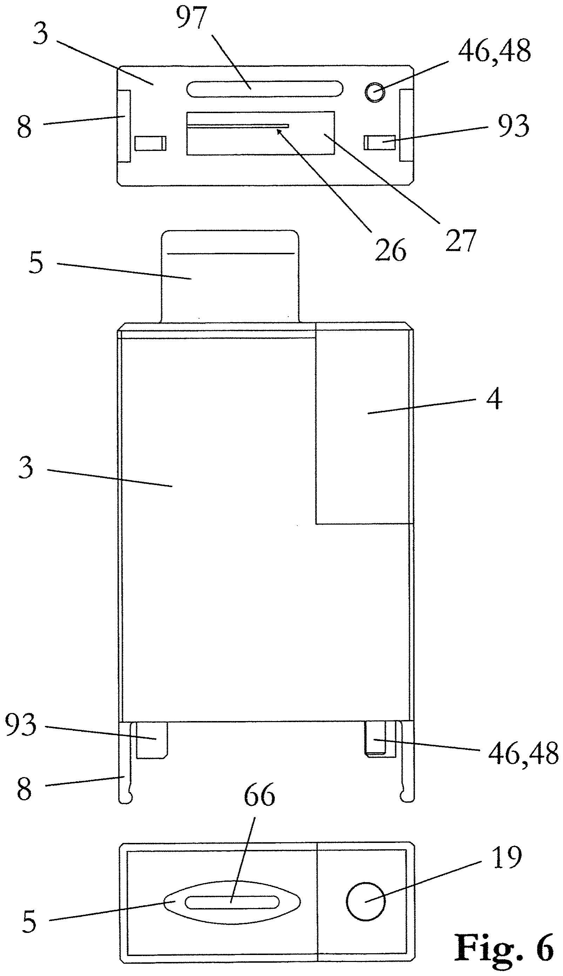

Fig. 6 die auswechselbare Inhalatorkomponente in verschiedenen Ansichten;

Fig. 7 die auswechselbare Inhalatorkomponente mit getrennt dargestelltem Flüssigkeitsbehälter und Mundstück;

Fig. 8 den Inhalator nach Fig. 1 ohne Schaltkreisdeckel;

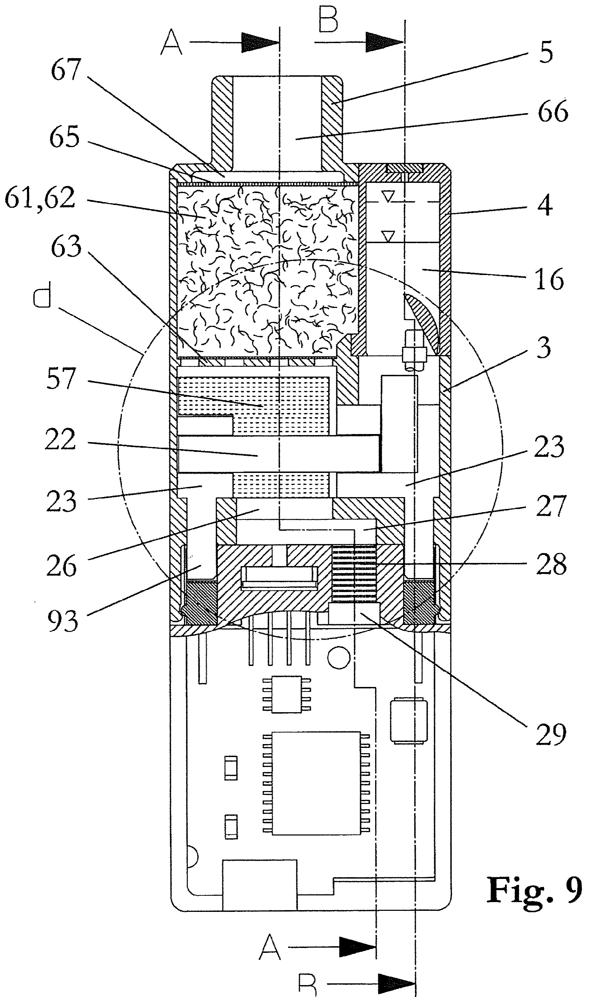

Fig. 9 einen Längsschnitt durch den Inhalator nach Fig. 8 in Höhe des flächigen Verbundes, wobei die Schnittführung abseits des Verbundes zweckmäßig angepaßt wurde;

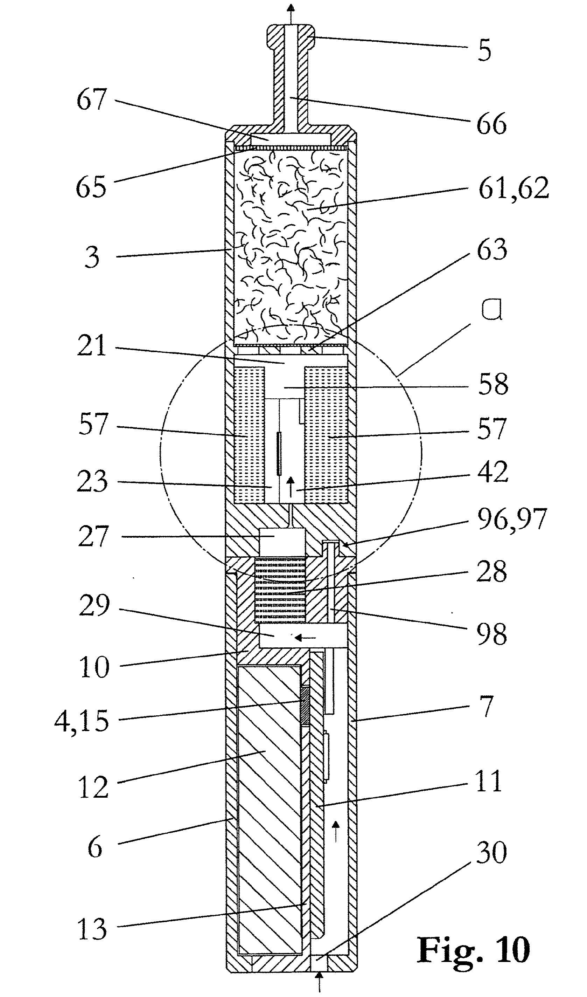

Fig. 10 eine Schnittansicht des Inhalators längs der Linie A-A in Fig. 9 mit Schaltkreisdeckel;

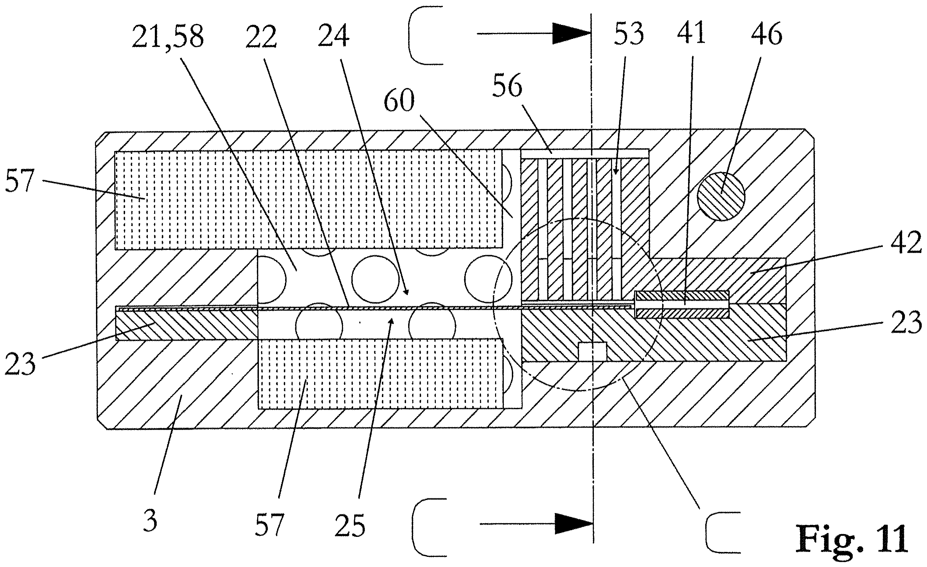

Fig. 11 einen Querschnitt des Inhalators nach Fig. 1 in Höhe des flächigen Verbundes;

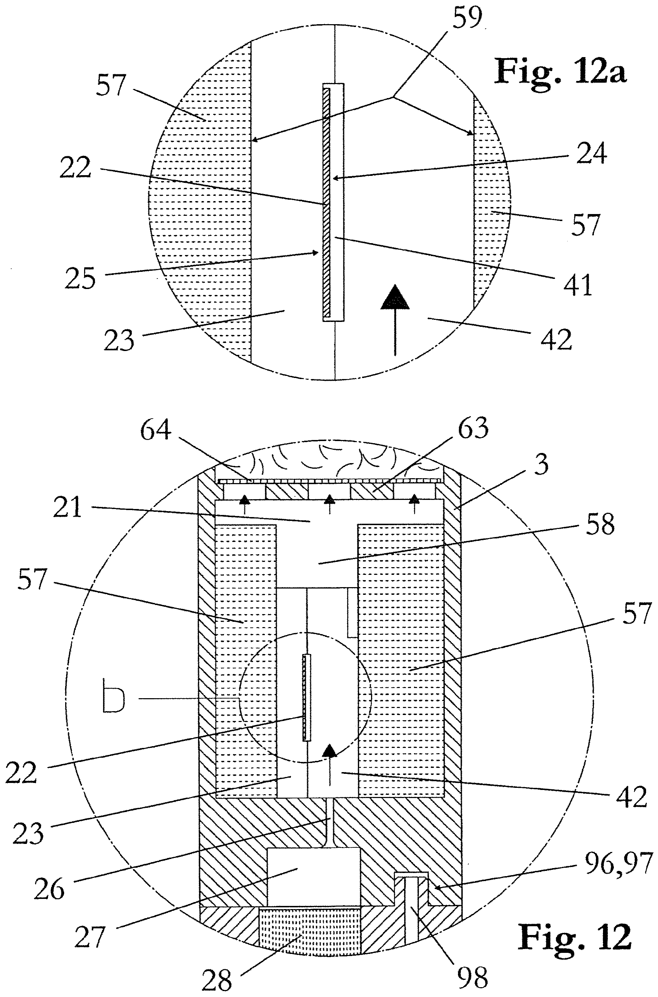

Fig. 12 das Detail a aus Fig. 10 in einer vergrößerten Darstellung;

Fig. 12a das Detail b aus Fig. 12 in einer vergrößerten Darstellung;

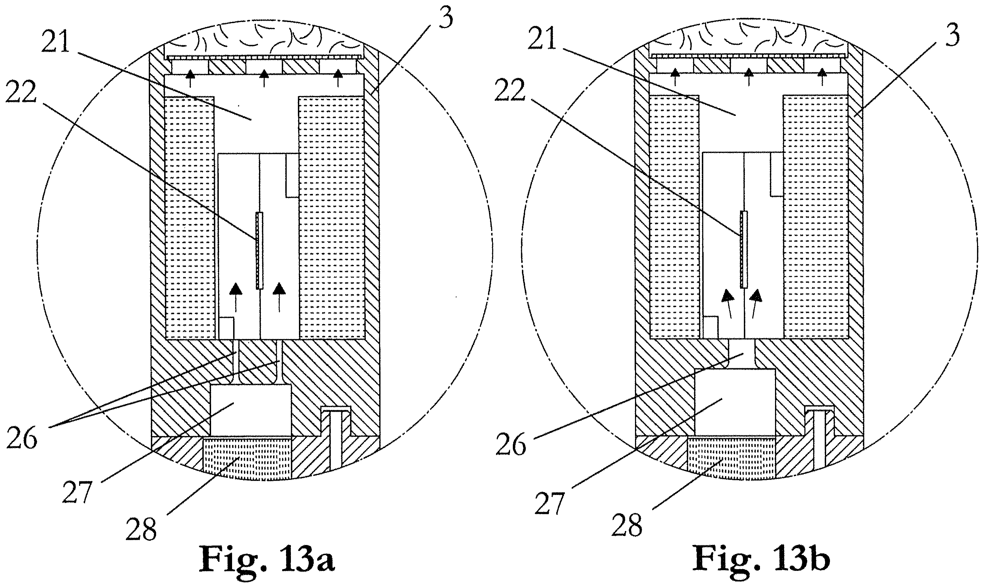

Fig. 13a und Fig. 13b alternative Ausführungsvarianten betreffend das Detail a;

Fig. 14a, Fig. 14b sowie Fig. 15a, Fig. 15b und Fig. 15c Querschnitte flächiger Verbunde in verschiedenen Ausführungsformen in vergrößerter Darstellung;

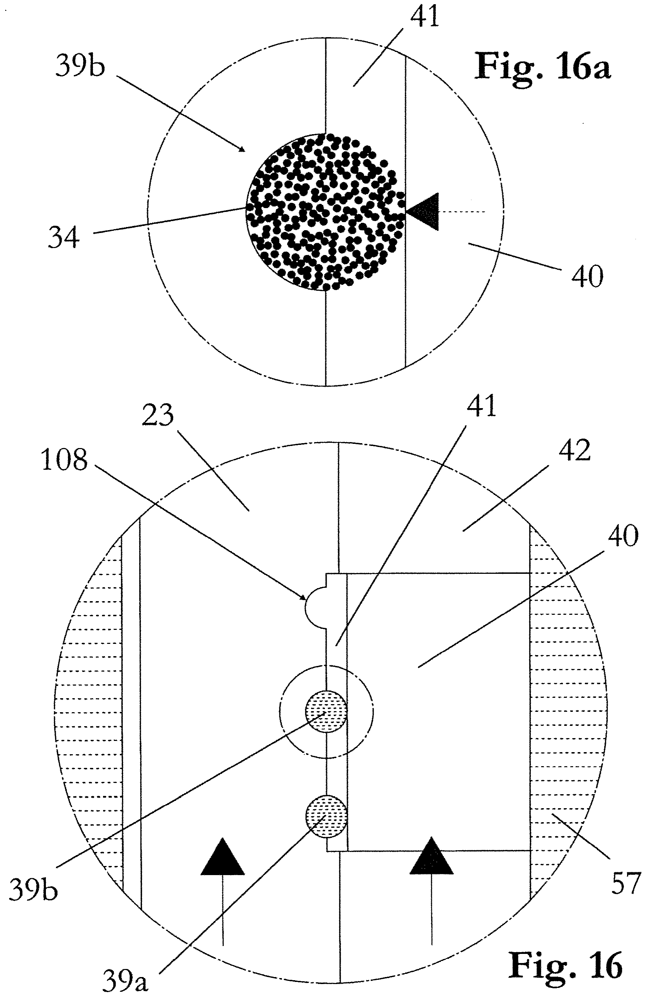

Fig. 16 eine Ausgestaltungsvariante betreffend das Detail b aus Fig. 12 mit drei nebeneinander angeordneten linienförmigen Verbunden;

Fig. 16a einen Querschnitt eines einzelnen linienförmigen Verbundes nach Fig. 16 in einer vergrößerten Darstellung;

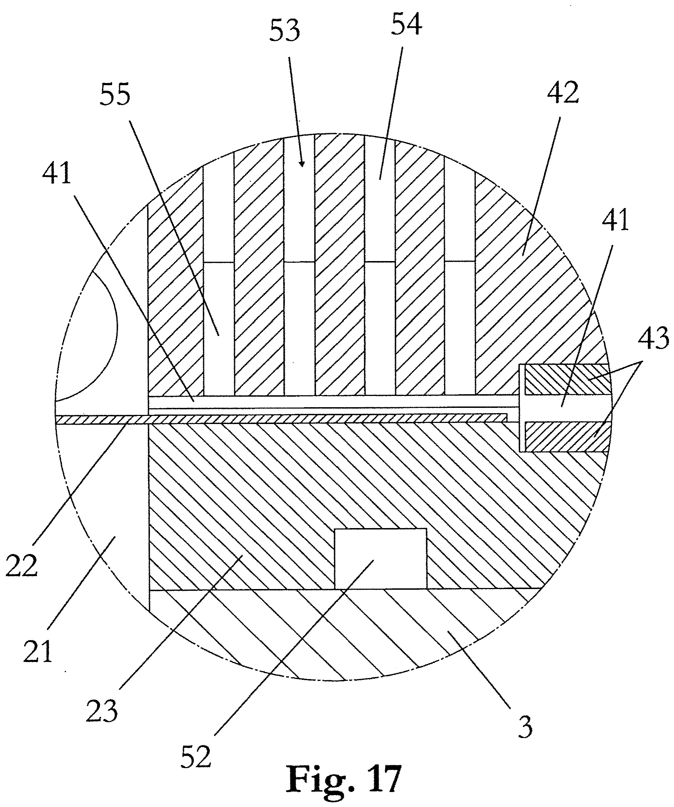

Fig. 17 das Detail c aus Fig. 11 in einer vergrößerten Darstellung;

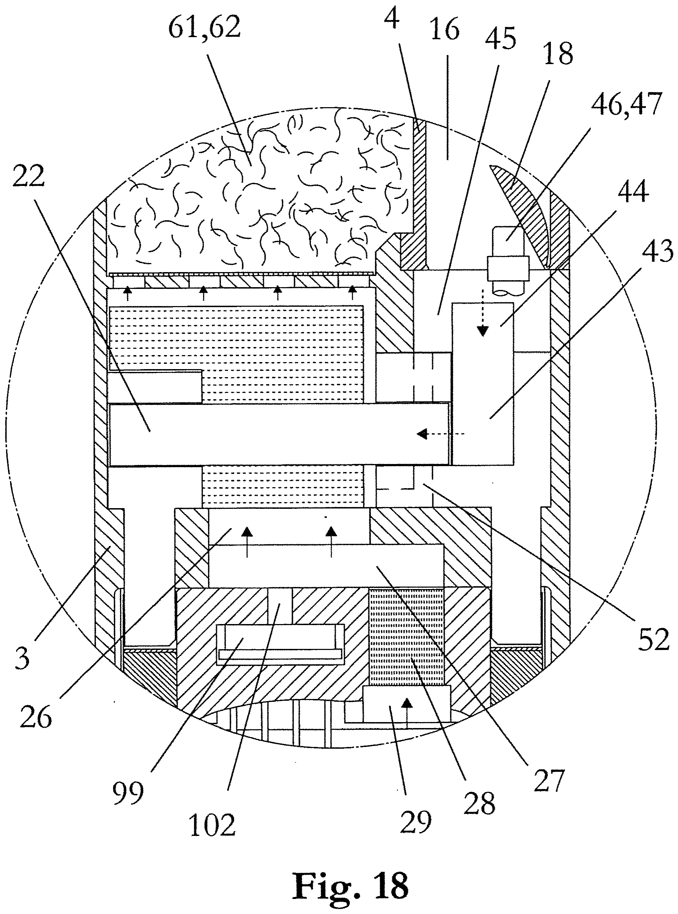

Fig. 18 das Detail d aus Fig. 9 in einer vergrößerten Darstellung;

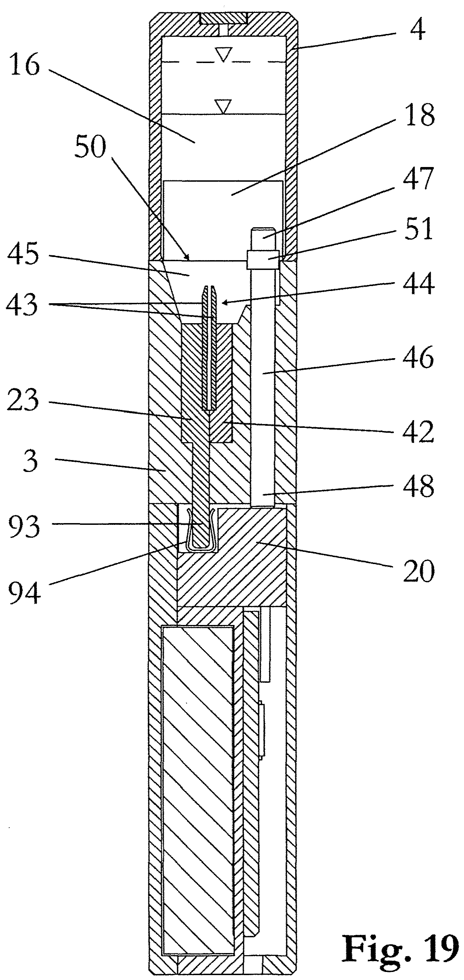

Fig. 19 eine Schnittansicht des Inhalators längs der Linie B-B in Fig. 9 mit Schaltkreisdeckel;

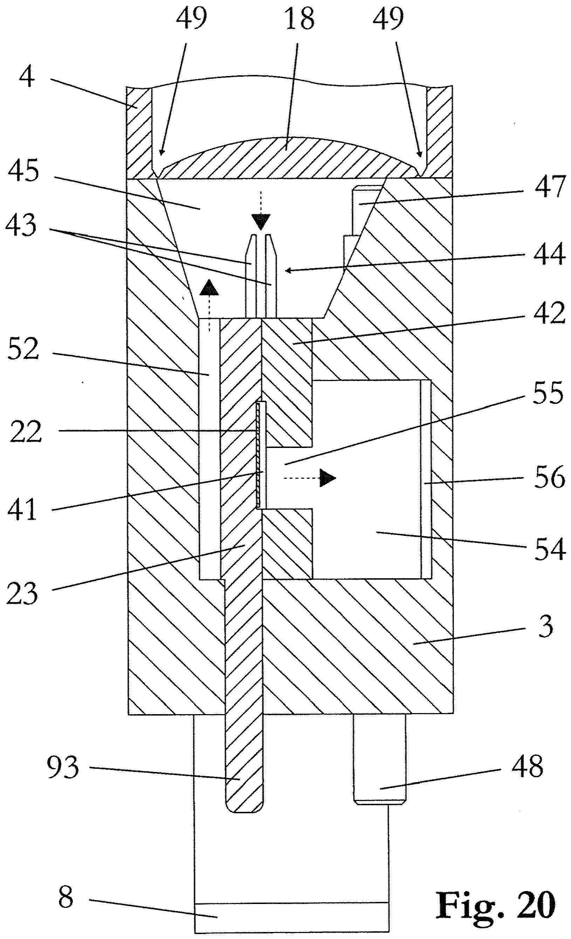

Fig. 20 eine Schnittansicht der auswechselbaren Inhalatorkomponente längs der Linie C-C in Fig. 7 und Fig. 11 mit angedeutetem Flüssigkeitsbehälter;

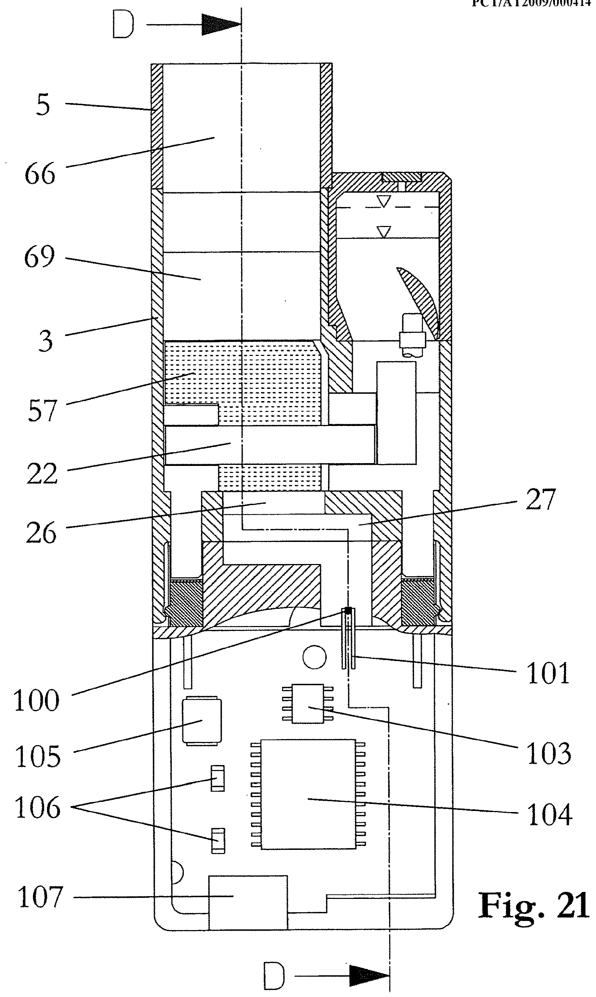

Fig. 21 einen erfindungsgemäßen Inhalator in einer zweiten Ausführungsform, ausgebildet als klassischer Inhalator in einer Ansicht analog zu Fig. 9;

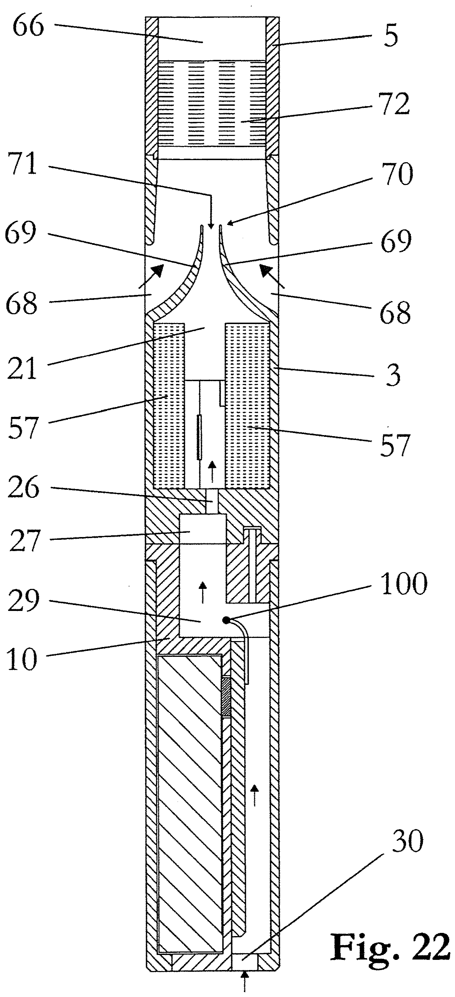

Fig. 22 eine Schnittansicht des Inhalators nach Fig. 21 längs der Linie D-D in Fig. 21 mit Schaltkreisdeckel;

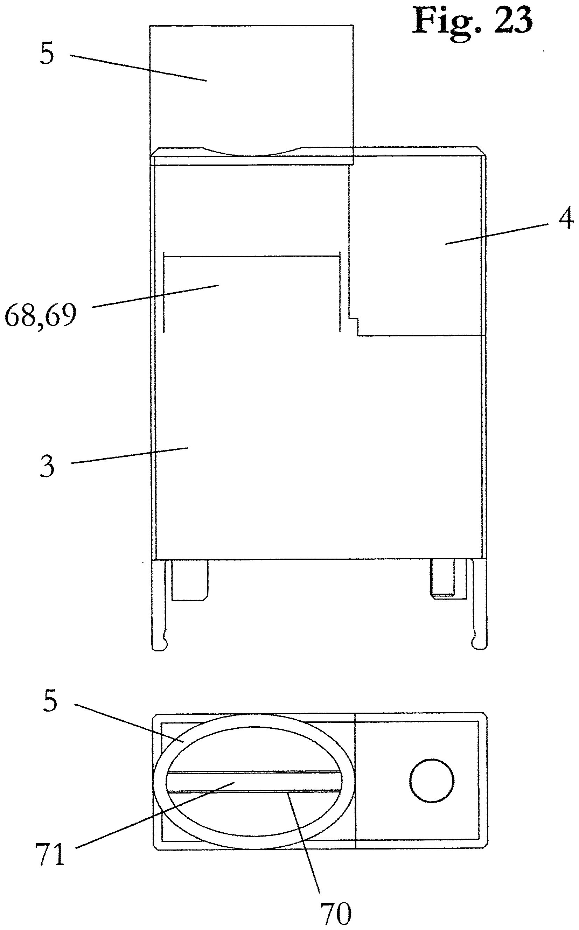

Fig. 23 die auswechselbare Inhalatorkomponente des Inhalators nach Fig. 21 in zwei Ansichten;

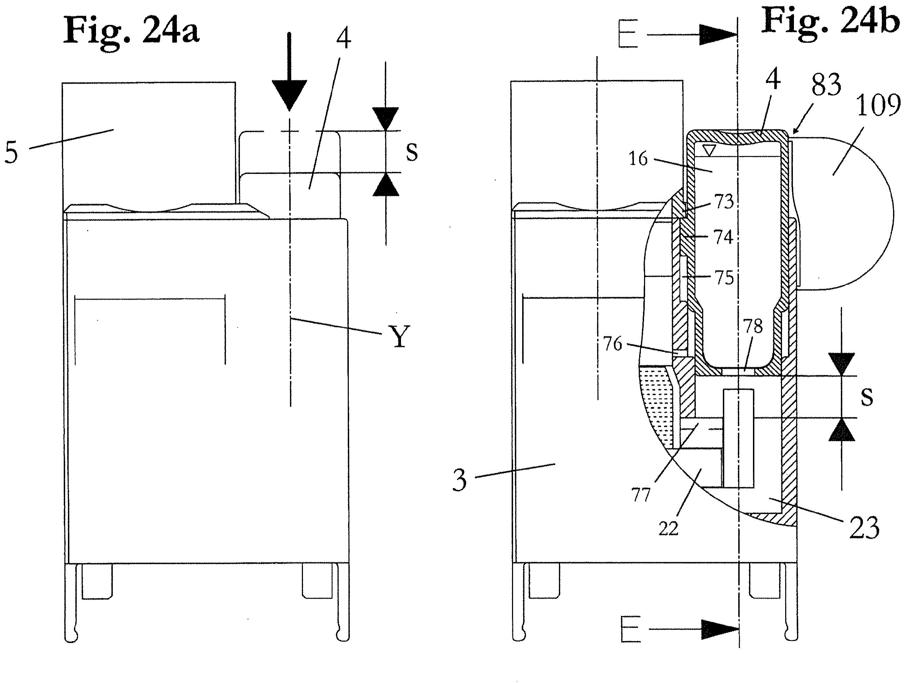

Fig. 24a und Fig. 24b eine auswechselbare Inhalatorkomponente mit einem alternativen Flüssigkeitsbehälter-System, wobei die Inhalatorkomponente nach Fig. 24b um den Flüssigkeitsbehälter aufgerissen dargestellt ist;

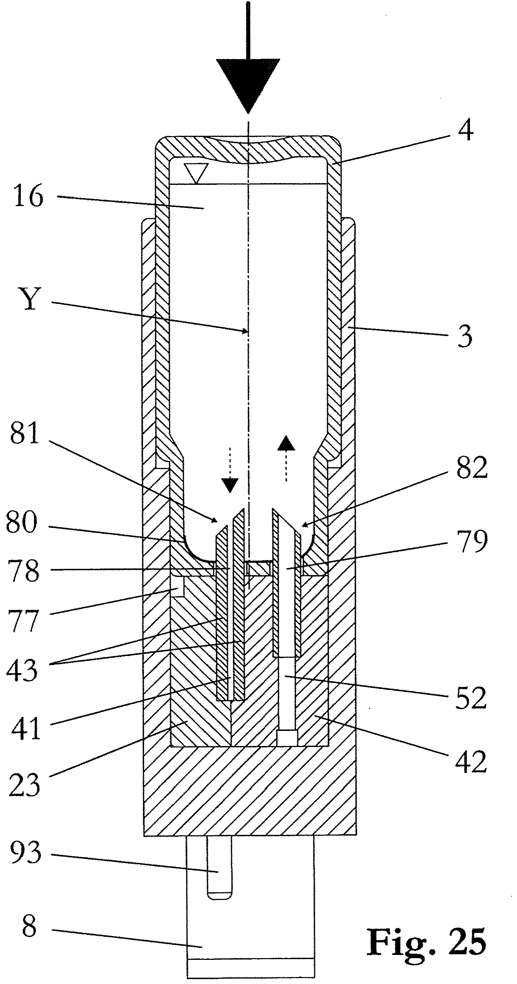

Fig. 25 eine Schnittansicht des Inhalators längs der Linie E-E in Fig. 24b;

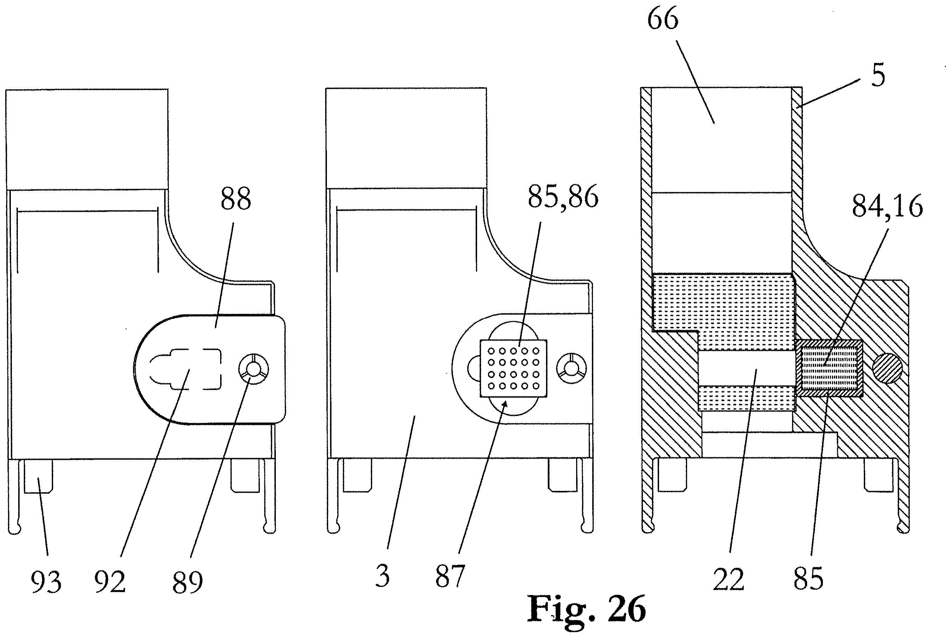

Fig. 26 eine auswechselbare Inhalatorkomponente mit einem weiteren alternativen Flüssigkeitsspeicher-System;

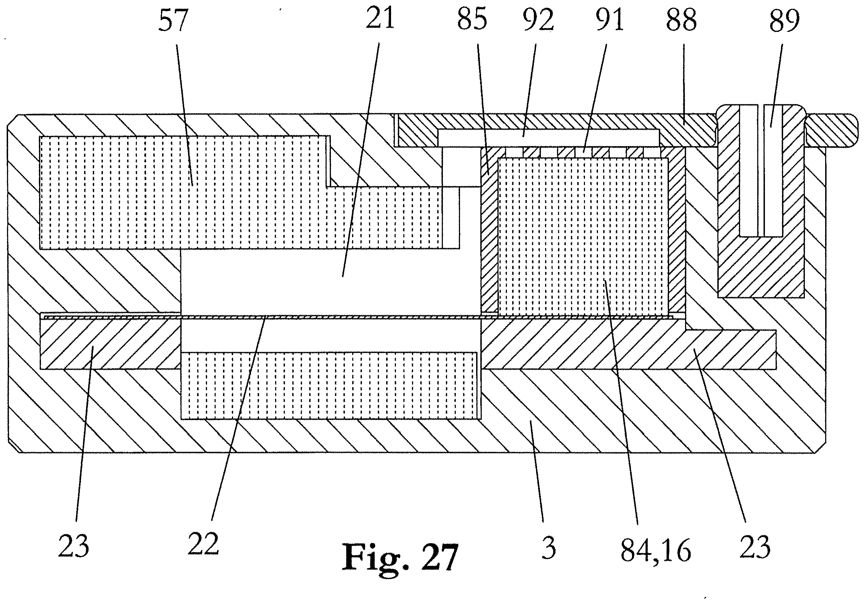

Fig. 27 einen Querschnitt der Inhalatorkomponente nach Fig. 26 in Höhe des flächigen Verbundes;

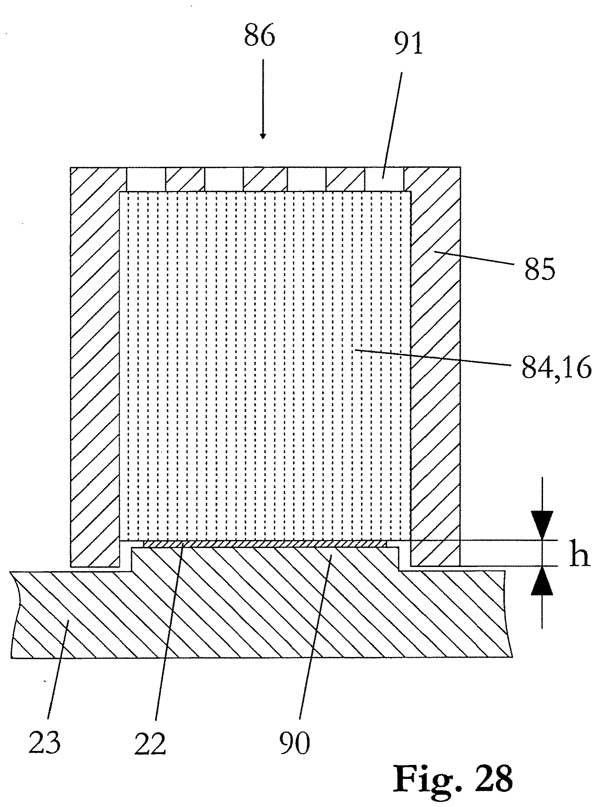

Fig. 28 einen Schnitt durch den Flüssigkeitsspeicher nach Fig. 26 quer zum flächigen Verbund;

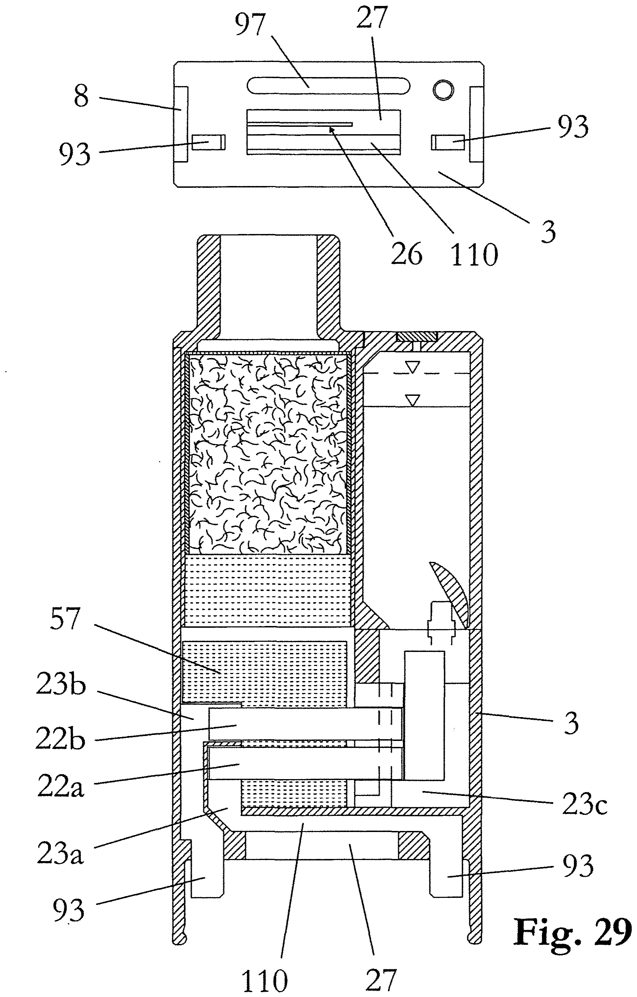

Fig. 29 eine auswechselbare Inhalatorkomponente mit zwei nebeneinander angeordneten flächigen Verbunden in einer Schnittansicht, wobei der Schnitt in Höhe der flächigen Verbunde verläuft, und in einer Seitenansicht.

Fig. 1 zeigt ein erstes Ausführungsbeispiel eines erfindungsgemäßen Inhalators, welcher Inhalator im konkreten Beispiel als Zug-Inhalator ausgebildet ist, und dessen Form und Größe derart gestaltet sind, daß der Inhalator von Benutzern einfach und bequem gehandhabt werden kann. Volumenmäßig ist der Inhalator nur etwa halb so groß wie eine Zigarettenschachtel. Der beispielhaft dargestellte Inhalator besteht grundsätzlich aus zwei Teilen, nämlich aus einem Inhalatorteil 1 und einer Inhalatorkomponente 2. Die Inhalatorkomponente 2 besteht aus einem Gehäuse 3 und umfaßt unter anderem einen Flüssigkeitsbehälter 4 und ein tabakpfeifenartiges Mundstück 5. Der Flüssigkeitsbehälter 4 beinhaltet ein flüssiges Material, welches in der Inhalatorkomponente 2 verdampft und in ein inhalierbares Dampf-Luft-Gemisch oder/und Kondensationsaerosol übergeführt wird. Das gebildete Dampf-Luft-Gemisch oder/und Kondensationsaerosol wird dem Benutzer über das Mundstück 5 dargeboten. Als flüssiges Material kommen grundsätzlich alle Stoffe und Zubereitungen in Betracht, welche unter atmosphärischen Bedingungen weitgehend rückstandsfrei verdampfbar sind. Diese Bedingung ist auch schon erfüllt, wenn der jeweilige Stoff oder die jeweilige Zubereitung verdünnt, beispielsweise in Wasser oder/ und Ethanol gelöst vorliegt und die Lösung weitgehend rückstandsfrei verdampft. Durch eine hinreichend hohe Verdünnung in einem leicht flüchtigen Lösungsmittel wie Ethanol oder/und Wasser können auch sonst schwer verdampfbare Stoffe die zuvor genannte Bedingung erfüllen, und eine thermische Zersetzung des flüssigen Materials vermieden oder deutlich verringert werden.

Das flüssige Material enthält vorzugsweise ein Arzneimittel, Die durch Kondensation erzeugten Aerosolteilchen weisen in der Regel einen massemedianen aerodynamischen Durchmesser (MMAD) kleiner als $2\mu$m auf und erreichen dadurch auch die Alveolen. Der erfindungsgemäße Inhalator eignet sich insbesondere für die Verabreichung von systemisch wirkenden Arzneimitteln wie beispielsweise solchen Arzneimitteln, welche ihre Hauptwirkung im zentralen Nervensystem entfalten. Als Beispiel sei Nikotin erwähnt, dessen Siedepunkt bei 246°C liegt. Die das Arzneimittel beinhaltenden Aerosolpartikel werden vorwiegend in den Alveolen niedergeschlagen, wo das Arzneimittel blitzartig in den Blutkreislauf übergeht. Am Beispiel des Nikotins sei angemerkt, daß dieses bereits etwa 7-10 Sekunden nach der Inhalation sein Zielorgan - nämlich das zentrale Nervensystem in gebündelter Konzentration erreicht. Selbstverständlich könnte der gegenständliche Inhalator auch ohne Arzneimittel, beispielsweise nur mit Aromastoffen betrieben werden - auch in Form nicht-medizinischer Anwendungen.

Das Inhalatorteil 1 beinhaltet, wie nachfolgend noch im Detail erläutert wird, zumindest einen Energiespeicher und einen elektrischen Schaltkreis, wobei der Energiepeicher durch einen Batteriedeckel 6 und der Schaltkreis durch einen Schaltkreisdeckel 7 geschützt sind.

Wie die Fig. 2 zeigt, sind das Inhalatorteil 1 und die Inhalatorkomponente 2 im konkreten Ausführungsbeispiel voneinander lösbar ausgeführt. Die lösbare Kopplung besteht aus einer Schnappverbindung, gebildet aus zwei Schnapphaken 8 und zwei mit diesen zusammenwirkenden Rastnasen 9. Diese Anordnung macht das Inhalatorteil 1 wiederverwendbar, was grundsätzlich sinnvoll ist, wenn man in Betracht zieht, daß erstens das Inhalatorteil 1 mit dem flüssigen Material nicht in Berührung kommt, also nicht mit dem flüssigen Material kontaminiert wird, und zweitens Komponenten beinhaltet, welche langlebiger sind als die Bestandteile der Inhalatorkomponente 2. Die Inhalatorkomponente 2 wird, nachdem das flüssige Material im Flüssigkeitsbehälter 4 aufgebraucht ist, vom Benutzer als Ganzes sachgerecht entsorgt, und durch eine neue Inhalatorkomponente 2 ersetzt. Die Inhalatorkomponente 2 stellt insofern einen auswechselbaren Einwegartikel dar. Eine sachgerechte Entsorgung ist vor allem dann angezeigt, wenn das flüssige Material Arzneimittel enthält, weil sich im Inneren des Gehäuses 3 der Inhalatorkomponente 2 im Zuge der Bildung des Dampf-Luft-Gemisches oder/und Kondensationsaerosols stets Kondensatrückstände bilden und ablagern. Auch im Flüssigkeitsbehälter 4 bleiben stets Reste des flüssigen Materials zurück. Grundsätzlich wäre es natürlich auch denkbar, das Inhalatorteil 1 und die Inhalatorkomponente 2 einteilig, also voneinander untrennbar auszuführen. Diese Ausführungsform dürfte jedoch unwirtschaftlicher sein, weil in diesem Fall alle Teile und Komponenten des Inhalators, also der Inhalator als Ganzes einen Einwegartikel zur einmaligen Benutzung bildet. Selbstverständlich schließt die gegenständliche Erfindung auch diese Ausführungsform mit ein, wobei in diesem Fall der ganze Inhalator als Inhalatorkomponente aufzufassen ist.

Die Fig. 3 bis 5 zeigen verschiedene Ansichten des wiederverwendbaren Inhalatorteils 1 mit und ohne Deckel. Das wiederverwendbare Inhalatorteil 1 setzt sich im Wesentlichen aus folgenden drei Gehäuseteilen zusammen: dem Batteriedeckel 6, dem Schaltkreisdeckel 7 und einem dazwischen angeordneten Trägergehäuse 10. Die drei Gehäuseteile sind aus Gewichtsgründen vorzugsweise aus Kunststoff gefertigt. Das Trägergehäuse 10 beherbergt den elektrischen Schaltkreis 11 und den Energiespeicher 12 und umfaßt eine Trennwand 13, welche den Schaltkreis 11 und den Energiespeicher 12 voneinander trennt. Der elektrische Schaltkreis 11 ist im Ausführungsbeispiel als einseitig bestückte Leiterplatte ausgebildet, welche auf der Trennwand 13 beispielsweise durch eine Klebeverbindung befestigt ist. Der Energiespeicher 12 besteht vorzugsweise aus einer wiederaufladbaren Batterie, beispielsweise aus einem Lithium-Ionen-Akkumulator oder einem Lithium-Polymer-Akkumulator, vorzugsweise in Flachbauweise. Diese Akku-Typen stellen gegenwärtig die höchsten Energiedichten und -ströme zur Verfügung und werden seit längerem vielfältig eingesetzt, wobei an erster Stelle die breite Verwendung in Mobiltelefonen zu nennen ist. Die Stromzufuhr von der Batterie 12 zur Platine 11 erfolgt über zwei Flachkontakte 14, welche auf der Rückseite der Platine 11 aufgelötet sind - siehe auch Fig. 10. Die Flachkontakte 14 ragen durch zwei etwas größere Fenster 15 in der Trennwand 13 hindurch. Die Batterie 12 umfaßt zwei korrespondierende Kontake (nicht dargestellt), welche gegen die Flachkontakte 14 gedrückt werden, wodurch ein lösbarer elektrischer Kontakt hergestellt wird. Die für diesen Zweck erforderliche Druckkraft wird vorzugsweise durch eine zwischen der Batterie 12 und dem Batteriedeckel 6 angeordnete Blattfeder (nicht dargestellt) erzeugt. Der Batteriedeckel 6 ist mit dem Trägergehäuse 10 lösbar verbunden - im Ausführungsbeispiel mittels einer Schraubverbindung (siehe Fig. 1). Selbstverständlich könnte der Batteriedeckel 6 alternativ auch als ein verrastbarer Schiebedeckel ausgebildet sein. Der Schaltkreisdeckel 7 ist mit dem Trägergehäuse 10 vorzugsweise untrennbar verbunden, zum Beispiel mittels einer Klebe- oder Schweißverbindung. Auf diese Weise soll einer unbefugten Manipulation des Schaltkreises 11 entgegengewirkt werden. Im normalerweise seltenen Fall eines Schaltkreisdefektes ist das ganze Inhalatorteil 1 mit Ausnahme der Batterie 12 zu ersetzen. Weitere Bestandteile und Eigenschaften des wiederverwendbaren Inhalatorteils 1 werden später noch näher beschrieben.

Die Fig. 6 und 7 zeigen verschiedene Ansichten der auswechselbaren Inhalatorkomponente 2. Wie schon erwähnt wurde, wird die auswechselbare Inhalatorkomponente 2 im Wesentlichen vom Gehäuse 3 ausgebildet, und beinhaltet unter anderem den Flüssigkeitsbehälter 4 und das tabakpfeifenartige Mundstück 5. Der Flüssigkeitsbehälter 4 und das Mundstück 5 sind untrennbar mit dem Gehäuse 3 verbunden. Herstellungstechnisch ist es günstig, den Flüssigkeitsbehälter 4 und das Mundstück 5 als separate Teile zu fertigen, und erst in einem Folgeschritt mit dem Gehäuse 3 beispielsweise durch eine Klebe- oder Schweißverbindung zu verbinden - siehe Fig. 7. Grundsätzlich ist es natürlich auch denkbar, den Flüssigkeitsbehälter 4 oder/und das Mundstück 5 mit dem Gehäuse 3 einteilig auszubilden. Das Gehäuse 3, der Flüssigkeitsbehälter 4 und das Mundstück 5 sind aus Gewichtsgründen vorzugsweise aus Kunststoff gefertigt, wobei bei der Materialauswahl für den Flüssigkeitsbehälters 4 die Eigenschaften des flüssigen Materials 16 zu berücksichtigen sind. Enthält das flüssige Material 16 beispielsweise Nikotin, können Kunststoffe gemäß US 5,167,242 (James E. Turner et al.) und US 6,790,496 (Gustaf Levander et al.) Verwendung finden.

Die Befüllung des Flüssigkeitsbehälters 4 mit dem flüssigen Material 16 erfolgt über ein Füllloch 17 vorzugsweise unter Schutzgasatmosphäre wie Argon oder Stickstoff. An einer Stirnseite des Flüssigkeitsbehälters 4 befindet sich ein klappenartiger, öffenbarer Verschluß 18, welcher vom Benutzer vor der Verwendung der Inhalatorkomponente 2 durch Eindrücken geöffnet wird. Der öffenbare Verschluß 18 wird später noch im Detail beschrieben. Der Flüssigkeitsbehälter 4 wird niemals vollständig mit dem flüssigen Material 16 gefüllt. Eine vollständige Befüllung würde wegen der Inkompressibilität des flüssigen Materials 16 dazu führen, daß sich der klappenartige, öffenbare Verschluß 18, welcher stets eine gewisse Elastizität aufweist, nicht mehr eindrücken und öffnen ließe. Nach der Befüllung wird das Füllloch 17 mit einem Verschlußdeckel 19 luftdicht verschlossen. Der Verschlußdeckel 19 kann beispielsweise aufgeklebt oder aufgeschweißt werden, wobei eine Hitzeeinwirkung auf das flüssige Material 16 möglichst vermieden werden sollte. Alternativ kann das Füllloch 17 als Kapillarbohrung ausgebildet sein, und die Befüllung mit dem flüssigen Material 16 über einer Injektionsnadel erfolgen. In diesem Fall könnte der Verschlußdeckel 19 entfallen, und die Kapillarbohrung selbst zugeschmolzen werden. Weitere Bestandteile und Eigenschaften der auswechselbaren Inhalatorkomponente 2 werden später noch im Detail beschrieben.

Fig. 8 zeigt den Inhalator nach Fig. 1 mit abgehobenem Schaltkreisdeckel 7. Unter anderem zeigt die Fig. 8 die Schnappverbindung, bestehend aus den zwei Schnapphaken 8 und den korrespondierenden Rastnasen 9, im gekoppelten, eingerasteten Zustand. Dabei sind die Schnapphaken 8 als Fortsätze des Gehäuses 3 ausgebildet, während die Rastnasen 9 von Kontaktelementen 20 gebildet werden. Die Kontaktelemente 20 sind am Trägergehäuse 10 des wiederverwendbaren Inhalatorteils 1 durch eine Klebeverbindung befestigt und erfüllen noch weitere Funktionen, welche später noch im Detail beschrieben werden.

Die Fig. 9 bis 13 geben nähere Aufschlüsse über das Innenleben des Inhalators und über dessen grundsätzliche Funktionsweise. Demnach bildet das Gehäuse 3 der auswechselbaren Inhalatorkomponente 2 im Inneren eine Kammer 21. Die Kammer 21 wird, wie Fig. 11 am besten zeigt, von einem erfindungsgemäßen flächigen Verbund 22 brückenartig und damit berührungsfrei durchsetzt. Der flächige Verbund 22 hat eine folien- bzw. streifenförmige plane Form und besteht aus einem Heizelement und einem Docht. Die Kapillarstruktur des Dochts ist dazu geeignet, flüssiges Material 16 aufzusaugen. Das Heizelement und der Docht können auf verschiedenste Weise ausgebildet und miteinander verbunden sein. Beispielhafte Ausbildungsformen werden später noch im Detail beschrieben. Der flächige Verbund 22 lagert mit zwei Endabschnitten auf zwei elektrisch leitenden, plattenförmigen Kontakten 23, auf deren Oberfläche er auch gleichzeitig elektrisch kontaktiert ist. Die Kontaktierung erfolgt vorzugsweise entweder durch eine flächige Klebeverbindung mittels eines leitfähigen Klebstoffes - z.B. Klebstoffe der Firma Epoxy Technology, www.epotek.com - oder durch eine Schweißverbindung. Im Fall einer Schweißverbindung ist darauf zu achten, daß der Docht bzw. dessen Kapillarstruktur durch die Schweißung nach Möglichkeit nicht beeinträchtigt wird. Bei Bedarf ist die Schweißung nur punktuell auszuführen. Bezüglich der Materialauswahl für die plattenförmigen Kontakte 23 wurden bereits früher Hinweise gegeben.

Der Bereich zwischen den beiden plattenförmigen Kontakten 23 definiert im Ausführungsbeispiel jenen beheizten Abschnitt des flächigen Verbundes 22, welcher berührungsfrei in der Kammer 21 angeordnet ist. Die berührungsfreie Anordnung führt dazu, daß die Wärmeleitungsverluste in Dickenrichtung des flächigen Verbundes 22 gleich null sind. Dadurch kann sich dieser Abschnitt so weit aufheizen, daß das im Docht gespeicherte flüssige Material 16 Siedetemperatur erreicht und verdampft. Erfindungsgemäß liegt die Kapillarstruktur des Dochts im besagten Abschnitt zumindest auf einer Seite des flächigen Verbundes weitgehend frei. Diese Seite ist, wie später noch im Zuge der Beschreibung beispielhafter Ausbildungsformen des Verbundes verständlich gemacht wird, vorzugsweise die den plattenförmigen Kontakten 23 abgewandte Seite 24 des flächigen Verbundes 22. Der im Zuge der Verdamfung des flüssigen Materials gebildete Dampf kann also weitflächig und ohne wesentliche Behinderung aus der freiliegenden Kapillarstruktur des Dochts ausströmen. In einer zweiten Ausgestaltung des flächigen Verbundes, welche ebenfalls später noch anhand von Beispielen beschrieben wird, liegt die Kapillarstruktur des Dochts im besagten Abschnitt zusätzlich auf der der Seite 24 gegenüberliegenden Seite 25 des flächigen Verbundes 22 weitgehend frei, so daß sich die Verdampfungsfläche und folglich auch die maximal erzielbare Verdampfungsleistung gegenüber dem zuerst genannten Fall verdoppelt. Die maximal erzielbare Verdampfungsleistung sei durch das erstmalige Auftreten einer Siedekrise im Docht definiert.

Das Gehäuse 3 bildet ferner eine Lufteinlaßöffnung 26 für die Zufuhr von Luft aus der Umgebung in die Kammer 21. Die zugeführte Luft mischt sich in der Kammer 21 mit dem aus der freiliegenden Kapillarstruktur des Dochts ausströmenden Dampf, im Zuge dessen sich das Dampf-Luft-Gemisch oder/und Kondensationsaerosol ausbildet. Die Lufteinlaßöffnung 26 ist als schlitzförmiger Kanal ausgebildet. Der schlitzförmige Kanal ist parallel zum flächigen Verbund 22 ausgerichtet. Im Ausführungsbeispiel nach Fig. 10 bzw. Fig. 12 ist der schlitzförmige Kanal etwas seitlich versetzt zum flächigen Verbund 22, nämlich auf jener Seite des flächigen Verbundes angeordnet, auf welcher die Kapillarstruktur des Dochts weitgehend freiliegt. Durch diese Anordnung wird erreicht, daß die, durch den schlitzförmigen Kanal 26 in die Kammer 21 einströmende Luft die freiliegende Kapillarstruktur des Dochts komplett überströmt, und sich homogene Mischungsbedingungen einstellen können. Durch Variation der Schlitzhöhe des schlitzförmigen Kanals 26 kann, wenn man ein konstantes Zugprofil (Zugvolumen, Zugdauer) voraussetzt, die Strömungsgeschwindigkeit der einströmenden Luft verändert, und auf diese Weise die Dynamik der Aerosolbildung und damit in Verbindung die Eigenschaften des erzeugten Aerosols in gewissen Grenzen beeinflußt werden. Eine Verringerung der Strömungsgeschwindigkeit läßt die Aerosolteilchen im Mittel an Größe zunehmen. Auch die geometrische Lage des schlitzförmigen Kanals 26 in Bezug auf den flächigen Verbund 22 hat einen Einfluß auf die Aerosolbildung.

Die Fig. 13a und 13b zeigen alternative Anordungen der Lufteinlaßöffnung 26; demnach wird die Lufteinlaßöffnung 26 im Beispiel nach Fig. 13a durch zwei schlitzförmige Kanäle 26 gebildet, welche auf gegenüberliegenden Seiten des flächigen Verbundes 22 angeordnet sind. Der flächige Verbund 22 wird also auf beiden Seiten von der in die Kammer 21 einströmenden Luft umströmt. Im Beispiel nach Fig. 13b ist der schlitzförmige Kanal 26 zentrisch zum flächigen Verbund angeordnet; der flächige Verbund 22 liegt in diesem Fall also in der Ebene des schlitzförmigen Kanals und wird von der einströmenden Luft direkt angeströmt, wobei der Luftstrom vom flächigen Verbund in zwei Teile aufgespalten wird, und der Verbund folglich wie im Beispiel zuvor auf beiden Seiten umströmt wird. Die Anordnungen nach Fig. 13a und 13b eignen sich vor allem für die Ausführungsvariante des flächigen Verbundes 22, bei welcher die Kapillarstruktur des Dochts beidseitig freiliegt, da in diesem Fall von beiden Seiten 24 und 25 des flächigen Verbundes 22 Dampf abströmt. Sie sind jedoch ebenso für die Ausführungsvariante des flächigen Verbundes 22 mit nur einseitig freiliegender Kapillarstruktur geeignet, insofern als der zweite Luftstromanteil, welcher den Verbund quasi passiv umströmt, den ersten, die Aerosolbildung bewirkenden Luftstromanteil abschwächt, wodurch wiederum Einfluß auf die Eigenschaften des gebildeten Aerosols genommen werden kann.

Die als schlitzförmiger Kanal ausgebildete Lufteinlaßöffnung 26 bezieht die Luft aus einer Plenumkammer 27, welche dazu dient, die Luft gleichmäßig auf den schlitzförmigen Kanal 26 zu verteilen, sodaß im schlitzförmigen Kanal im Wesentlichen allseits gleiche Strömungsbedingungen herrschen. Stromaufwärts der Plenumkammer 27 befindet sich eine Strömungsdrossel 28. Die Strömungsdrossel 28 hat den Zweck, einen Strömungswiderstand zu erzeugen, welcher dem einer Zigarette ähnlich ist, so daß der Benutzer während eines Zuges einen ähnlichen Zugwiderstand verspürt wie bei einem Zug an einer Zigarette. Konkret sollte der Strömungswiderstand bei einem Durchfluß von 1,05 L/min im Bereich 12-16mbar liegen und eine möglichst lineare Charakteristik aufweisen. Die Strömungsdrossel 28 kann beispielsweise aus einem offenporigen Sinterkörper aus Metall oder Kunststoff gebildet sein, dessen Poren von der Luft durchströmt werden. In Prototypen haben sich beispielsweise poröse Kunststofformkörper der Firma Porex, www.porex.com bewährt. Im Ausführungsbeispiel ist die Plenumkammer 27 Teil der auswechselbaren Inhalatorkomponente 2 und die Strömungsdrossel 28 Teil des wiederverwendbaren Inhalatorteils 1. Grundsätzlich wäre es auch möglich, die Plenumkammer 27 und die Strömungsdrossel 28 in der auswechselbaren Inhalatorkomponente 2 anzuordnen, oder alternativ beide im wiederverwendbaren Inhalatorteil 1 anzuordnen.

Die Fig. 10 zeigt den weiteren Verlauf der Luftströmung stromaufwärts der Strömungsdrossel 28. Die Strömung ist durch Pfeile angedeutet. Demnach bezieht die Strömungsdrossel 28 die Luft aus einem Querkanal 29, welcher seinerseits in den Raum zwischen der Platine 11 und dem Schaltkreisdeckel 7 mündet. Die eigentliche Zufuhr der Luft aus der Umgebung erfolgt über eine vom Schaltkreisdeckel 7 gebildete Speiseöffnung 30. Die Speiseöffnung 30 ist auf der dem Mundstück 5 gegenüberliegenden Stirnseite des Inhalators angeordnet. Diese Lage schützt am ehesten vor dem Eintritt von Regenwasser.

Die Fig. 14a, 14b und 15a, 15b, 15c zeigen beispielhafte Ausbildungsformen des flächigen Verbundes 22 anhand von Querschnittsdarstellungen, wobei unter "Querschnitt" ein Schnitt normal zur Längsrichtung des Verbundes verstanden wird (vgl. Fig. 9). Konkret zeigen die Fig. 14a und 14b Ausführungsformen mit nur einseitig freiliegender Kapillarstruktur, während die Fig. 15a bis 15c Ausführungsformen zeigen, bei welchen die Kapillarstruktur des Dochts auf beiden Seiten des flächigen Verbundes freiliegt. Nach der Ausführungsform gemäß Fig. 14a besteht der flächige Verbund 22 aus vier Lagen: nämlich aus einer Metallfolie 31 und drei darauf aufgesinterten Metalldrahtgeweben 32. Das Metall besteht aus Edelstahl (z.B. AISI 304 oder AISI 316), oder aus einer Heizleiterlegierung - insbesondere aus der Gruppe der NiCr-Legierungen oder CrFeAl-Legierungen ("Kanthal"). Bei Verwendung von Edelstahl wird kohlenstoffreduzierten Chargen (z.B. AISI 304L oder AISI 316L) der Vorzug gegeben, weil diese weniger anfällig für interkristalline Korrosion sind. Die Metallfolie 31 kann in Edelstahlausführung beispielsweise von der Firma Record Metall-Folien GmbH, www.recordmetall.de bezogen werden. Die Drahtgewebe können beispielsweise von den Firmen Haver & Boecker, www.haverboecker.com oder Spörl KG, www.spoerl.de bezogen werden. Die vier Lagen sind durch eine Sinterung miteinander verbunden. Die Sinterung wird vorzugsweise im Vakuum oder unter Wasserstoff-Schutzgas durchgeführt. Solche Sinterungen zählen zum Stand der Technik und werden beispielsweise von der Firma GKN Sinter Metals Filters GmbH, www.gkn-filters.com sowie von der Firma Spörl KG, www.spoerl.de routinemäßig durchgeführt. Die Sinterung erfolgt vorteilhafterweise in Form eines Vielfachnutzens; das heißt, daß nicht einzelne flächige Verbunde gesintert werden, sondern größere flächige Nutzen beispielsweise im Format 200x200mm. Die einzelnen Verbunde werden nach der Sinterung durch Laserschneiden oder Stanzen aus dem Vielfachnutzen gewonnen und anschließend optional in einem Beizbad geätzt.

Tabelle 1 zeigt beispielhaft die Spezifikationen von in Prototypen verwendeten flächigen Verbunden 22,

Tabelle1:

| | | |
|---|---|---|
| Metallfolien-Dicke: | 10$\mu$m | |
| Metallfolien-Material: | AISI 304 | |
| 1. Drahtgewebelage: | 36x90$\mu$m | Drahtdurchmesser x Maschenweite |
| 2. Drahtgewebelage: | 30x71$\mu$m | Drahtdurchmesser x Maschenweite |
| 3. Drahtgewebelage: | 20x53$\mu$m | Drahtdurchmesser x Maschenweite |
| Drahtgewebe-Material: | AISI 316L | |
| Verbund-Spannweite: | 14mm | |
| Verbund-Breite: | 2-5mm | |
| Verbund-Dicke: | 140-160$\mu$m | |
| Ätzrate: | bis 50% | mit Badbeize Avesta 302 *) |
| Porosität: | 65-80% | abhängig von der Ätzrate |
| *) Hersteller: Avesta Finishing Chemicals, www.avestafinishing.com | | |

Die Verbund-Spannweite entspricht jener Strecke in der Kammer 21, welche der Verbund 22 berührungsfrei überbrückt; im konkreten Ausführungsbeispiel entspricht diese Strecke dem Abstand zwischen den beiden plattenförmigen Kontakten 23. Die Verbund-Spannweite und die Verbund-Breite haben einen entgegengesetzten Einfluß auf den resultierenden Heizelement-Widerstand. Die Ätzrate definiert den insgesamt durch die Ätzung erzielten Massenverlust. Die erste Drahtgewebelage liegt direkt auf der Metallfolie 31 auf. Die dritte Drahtgewebelage bildet die Decklage und gleichzeitig die freiliegende Kapillarstruktur des flächigen Verbundes 22. Der flächige Verbund 22 lagert vorzugsweise mit der Metallfolie 31 auf den plattenförmigen Kontakten 23. Die elektrische Kontaktierung der Metallfolie 31 erfolgt vorzugsweise über eine flächige Klebeverbindung zwischen der Matallfolie 31 und den elektrisch leitenden, plattenförmigen Kontakten 23. Grundsätzlich könnte die Kontaktierung auch durch eine Schweißverbindung hergestellt werden. Ein auf solche Weise kontaktierter flächiger Verbund 22 mit den Spezifikationen gemäß Tabelle 1, mit einer Verbund-Breite von 2mm und einer Ätzrate von 35% weist einen Heizelement-Widerstand von etwa 310mOhm auf. Bei Verwendung von Heizleiterlegierungen anstatt Edelstahl kann der Heizelement-Widerstand deutlich gesteigert werden, konkret bei Verwendung von DIN-Werkstoff-Nummer 2.4872 (NiCr20AlSi) im Vergleich zu AISI 304/ AISI 316 um den Faktor 1,8 und bei Verwendung von DIN-Werkstoff-Nummer 1.4765 (CrAl255) gar um den Faktor 2,0. Folgedessen hätte ein flächiger Verbund mit einer Verbund-Breite von 5mm in Ausführung DIN-Werkstoff-Nummer 2.4872 aber ansonsten gleichen Spezifikationen, wie zuvor angeführt, einen Heizelement-Widerstand von etwa 225 mOhm. Erfolgt die Energiezufuhr auf Basis einer Lithium-Polymer-Zelle mit einer Nenn- oder Leerlaufspannung von 3,7V und einer Nutzspannung unter Last von ca. 3,1V, errechnet sich auf Basis des Ohmschen Gesetzes der Strom, welcher durch den flächigen Verbund fließt, zu 10A (für 310mOhm) bzw. 13,8A (für 225mOhm). Diese Stromstärken können aus heutigen Lithium-Polymer-Zellen problemlos bezogen werden. In einem weiteren Schritt errechnet sich die elektrische Nennleistung, das ist gleichzeitig die maximal realisierbare Heizleistung zu 31W (für 310mOhm) bzw. 42,7W (für 225mOhm). Wie später noch beschrieben wird, können diese Leistungen durch den elektrischen Schaltkreis 11 beliebig reduziert werden.

Auf Basis der zuvor angeführten Spezifikationen eines beispielhaften flächigen Verbundes mit einer Verbund-Breite von 5mm und einer Ätzrate von 35% errechnet sich das Porenvolumen des flächigen Verbundes 22 im Abschnitt der Verbund-Spannweite (Verdampfungsabschitt) zu etwa 7,5μL. Dieses Volumen wird vom zu verdampfenden flüssigen Material 16 ausgefüllt und entspricht jener Menge an flüssigem Material, welche pro Zug bzw. Inhalation (intermittierender Inhalatorbetrieb) maximal verdampft werden kann. Enthält das flüssige Material beispielsweise Nikotin als Arzneimittel in einer Konzentration von typischerweise 1,5 Vol.-%, so resultiert daraus theoretisch pro Verdampfung bzw. Zug eine maximal freigesetzte Nikotindosis von 110μg bzw. aufgerechnet auf 10 Inhalationen eine Gesamtdosis von 1,1mg. Real wird die maximal erzielbare Dosis aus verschiedenen Gründen etwas unter den berechneten Werten liegen. Wesentlich ist jedoch die Tatsache, daß mit dem erfindungsgemäßen Inhalator die Nikotin-Dosen von heutigen Zigaretten (0,1-1,0mg) problemlos verabreicht werden können. Wesentlich ist ferner, daß sich die Wirkstoff-Dosis beliebig reduzieren läßt, sei es durch eine Verringerung der Wirkstoffkonzentration im flüssigen Material, sei es durch die Wahl einer kleineren Verbund-Breite, oder sei es durch eine Drosselung der zugeführten Heizleistung mittels des elektrischen Schaltkreises 11. Die letztere Maßnahme wirkt außerdem einer thermischen Zersetzung des flüssigen Materials 16 entgegen, da der Verbund 22 nicht so hoch aufgeheizt wird.

Es sei angemerkt, daß sowohl die Metallfolie 31 als auch die auf die Folie aufgesinterten Metalldrahtgewebe 32 einen Beitrag zum elektrischen Heizwiderstand leisten. Der elektrische Heizwiderstand kann insofern als Parallelschaltung von diesen Einzelwiderständen interpretiert werden. Ebenso ist auch die Kapillarwirkung des Dochts im Zusammenwirken der Drahtgewebe 32 mit der Metallfolie 31 begründet, wobei auch schon eine einzelne Drahtgewebelage in Kombination mit der Metallfolie 31 einen Kapillareffekt erzeugen kann. Selbstverständlich ist die Erfindung nicht auf die zuvor genannten Spezifikationen beschränkt. Es wäre auch möglich, anstatt der Metalldrahtgewebe 32 sonstige offenporige Strukturen aus Metall auf der Metallfolie 31 anzuordnen; desweiteren könnten erfindungsgemäß auch ein Gewebe oder sonstige offenporige Strukturen aus einem elektrisch nichtleitenden Material, wie bevorzugt Quarzglas, auf der Metallfolie 31 angeordnet bzw. auf diese aufgefrittet werden.

Fig. 14b zeigt eine zweite exemplarische Ausführungsform eines flächigen Verbundes 22 mit nur einseitig freiliegender Kapillarstruktur. Diese Ausführungsform unterscheidet sich von jener nach Fig. 14a nur dadurch, daß anstatt der äußeren zwei Drahtgewebelagen ein Faserverbund in Form eines Vlieses 33 vorgesehen ist, welches auf die erste Drahtgewebelage 32 aufgesintert ist. Solche Vliese 33 können in Edelstahlausführung beispielsweise von der Firma GKN Sinter Metals Filters GmbH, www.gkn-filters.com nach Kundenspezifikation angefertigt werden. Das Vlies 33 hat vorzugsweise eine Dicke von 100-300μm und eine Porosität >70%. Das die freiliegende Kapillarstruktur des Dochts bildende Vlies 33 weist im Vergleich zu den Drahtgeweben 32 eine deutlich größere Oberfläche auf; die größere Oberfläche wirkt sich günstig auf den Verdampfungsprozess aus. Das Vlies 33 kann selbstverständlich auch aus einer Heizleiterlegierung - insbesondere aus der Gruppe der NiCr-Legierungen oder CrFeAl-Legierungen ("Kanthal") hergestellt werden; zu diesem Zweck sind lediglich die das Vlies 33 bildenden Rohfasern in diesen Werkstoffspezifikationen zu erzeugen. Der flächige Verbund 22 kann nach der Sinterung optional wieder geätzt werden.

Fig. 15a zeigt eine Ausführungsform eines flächigen Verbundes 22 mit beidseitig freiliegender Kapillarstruktur. Der flächige Verbund besteht demnach aus einer offenporigen Sinterstruktur gebildet aus einem homogenen körnigen, faserigen oder flockigen Sinterverbund 34. Die Herstellung von dünnen porösen Sinterverbunden ist seit langem bekannt. US 3,433,632 (Raymond J. Elbert) beschreibt beispielsweise ein Verfahren zur Herstellung von dünnen porösen Metallplatten mit einer Dicke ab 75μm und einem Porendurchmesser zwischen 1-50μm. Unter anderem wurden Pulver aus Nickel sowie Edelstahl (AISI 304) verarbeitet. Es werden Porositäten bis 60%, und in einer Variante mit einem mehrlagigen Aufbau sogar Porositäten bis 90% (allerdings nur in den Decklagen) erreicht. US 6,652,804 (Peter Neumann et al.) beschreibt ein ähnliches Verfahren. JP 2004/332069 (Tsujimoto Tetsushi et al., Mitsubishi Materials Corporation) beschreibt ein weiterentwickeltes Verfahren zur Herstellung von dünnen porösen Sinterverbunden aus Metall im bevorzugten Dickenbereich von 50-300μm, welches sich dadurch auszeichnet, daß dem zu verarbeitenden Metallpulver entfernbare Füllstoffe, im konkreten Fall Acrylharz-Kügelchen beigemischt werden. Die Acrylharz-Kügelchen sind Platzhalter, welche im Zuge einer Wärmebehandlung noch vor der eigentlichen Sinterung bei etwa 500°C im Vakuum praktisch rückstandslos sublimieren und Hohlräume zurücklassen, welche Hohlräume auch während und nach der Sinterung bestehen bleiben. Auf diese Weise wurden fläche Verbunde bestehend aus Edelstahl der Spezifikation AISI 316L mit Porositäten von typischerweise 70-90% hergestellt. Das Institut für Energieforschung (IEF) des Forschungszentrums Jülich, www.fz-juelich.de/ief/ief-1 ist ebenfalls in der Lage, dünne poröse Metallfolien bis zu einer Dicke von 500μm herzustellen. Die Herstellmethode basiert wie auch das zuvorgenannte Verfahren auf dem sogenannten Doctor-Blade- Foliengießverfahren.

Grundsätzlich können alle genannten Verfahren für die Herstellung eines erfindungsgemäßen flächigen Sinterverbundes 22, 34 angewandt werden, wobei dem Verfahren nach JP 2004/332069 wegen der hohen erzielten Porosität der Vorzug gegeben wird. Zu beachten ist lediglich, daß der mittlere Porendurchmesser im homogenen Sinterverbund möglichst >10μm ist, um eine ausreichend schnelle Infiltration des Dochts mit dem flüssigen Material 16 sicherzustellen.

Die Korngröße der zu verarbeitenden Metallpulver und der Acrylharz-Kügelchen sind auf diese Bedingung abzustimmen. Der im Verfahren nach JP 2004/332069 angeführte, bevorzugte Dickenbereich von 50-300 $\mu$m deckt sich mit dem für den flächigen Verbund 22 besonders bevorzugten Dickenbereich.

Die genannten Verfahren eignen sich neben der Verarbeitung von Edelstahl auch für die Verarbeitung von pulverförmigen Heizleiterlegierungen sowie pulverförmigen keramischen Widerstandsmaterialien.

Fig. 15b zeigt eine Weiterbildung bzw. Modifikation eines flächigen Verbundes gemäß der Ausführung nach Fig. 15a, indem im flächigen Verbund 22 in Längsrichtung des Verbundes ausgerichtete Kanäle bzw. Arterien 35 angeordnet sind, deren vorteilhafte Effekte bereits früher beschrieben wurden. Die Herstellung dieser Kanäle 35 erfordert eine Adaption der zuvor genannten Herstellungsverfahren, indem in den Foliengießschlicker durch Oxidation, Sublimation oder chemische Zersetzung entfernbare Fäden, z.B. sublimierbare Acrylharz-Fäden eingebracht werden. Die Fäden sind Platzhalter, welche im Zuge ihrer Entfernung Kanäle 35 bildende Hohlräume zurücklassen. Hierbei wird am besten in drei Verfahrensschritten vorgegangen: zuerst wird eine erste Folienlage gegossen. Auf diese wird eine Lage von zueinander parallel ausgerichteten Fäden aufgelegt, welche später die Arterien 35 bilden. Schließlich wird eine zweite Folienlage gegossen, welche gleichzeitig die Decklage bildet. Zur besseren Handhabung werden die Fäden vor ihrer Applikation in einen Hilfsrahmen gespannt. Die Korngröße der zu verarbeitenden Metallpulver und ggf. Acrylharz-Kügelchen liegt bei dieser modifizierten Ausführungsform vorzugsweise im Bereich 1-10 $\mu$m, während der bevorzugte Durchmesser-Bereich der Fäden 20-150 $\mu$m ist. In einem an das Foliengießen und Sintern anschließenden optionalen Verfahrensschritt wird der flächige Sinterverbund 22, 34 in Dickenrichtung gelocht, wodurch Löcher 36 gebildet werden. Die Lochung kann beispielsweise mittels eines Lasers erfolgen. Das Lochraster sollte möglichst uneinheitlich gewählt werden; bei einem einheitlichen Raster könnte nämlich der ungünstige Fall eintreten, daß sämtliche Löcher 36 zwischen den Arterien 35 zu liegen kommen, und die Arterien nicht geschnitten werden. Die früher bereits beschriebenen, vorteilhaften Effekte der Lochung würden in diesem Fall nur teilweise in Erscheinung treten.

Zur weiteren Steigerung der Porosität und des elektrischen Widerstandes können die Verbunde gemäß den Ausführungen nach Fig. 15a und 15b nach der Sinterung optional wieder geätzt werden. Die Befestigung und Kontaktierung des flächigen Sinterverbundes 22, 34 auf den plattenförmigen Kontakten 23 erfolgt vorzugsweise durch eine Schweißverbindung. Eine Klebeverbindung ist nur möglich, wenn der verwendete Klebstoff eine hinreichend pastöse oder zähflüssige Konsistenz aufweist. Andernfalls bestünde die Gefahr, daß der Klebstoff in die Porenstruktur des Verbundes eintritt und die Kapillarwirkung des Dochts beeinträchtigt. Gegebenenfalls kann es von Vorteil sein, die Lochung des Verbundes im Bereich der Klebeverbindung auszusetzen.

Fig. 15c zeigt schließlich eine weitere Ausführungsform eines flächigen Verbundes 22 mit beidseitig freiliegender Kapillarstruktur. Demnach besteht der flächige Verbund 22 aus einem offenporigen Schaum 37 gebildet aus einem elektrischen Widerstandsmaterial. Die Herstellung von schaumartigen Verbunden ist seit langem bekannt. So beschreibt US 3,111,396 (Burton B. Ball) bereits ein Verfahren zur Herstellung von Metallschäumen, Keramikschäumen und Graphitschäumen. Das Verfahren beruht darauf, daß eine organische poröse Struktur mit einem, das schaumbildende Material enthaltenden Schlicker imprägniert wird, und im Zuge einer nachfolgenden Wärmebehandlung sich die organische Struktur zersetzt. Auf diese Weise wurden unter anderem Schäume aus Nickel und Nickel-Basis-Legierungen hergestellt. Für einen erfindungsgemäßen flächigen Verbund 22 sind dünne, folienartige Schäume mit einer Dicke im Bereich 100-500 $\mu$m, einem bevorzugten Porendurchmesser im Bereich 20-150 $\mu$m und einer Porosität >70% erforderlich. Ein solches Schaummaterial kann in Edelstahlausführung (z.B. AISI 316L) von der Firma Mitsubishi Materials Corporation, www.mmc.co.jp bezogen werden. Hierbei wird von einem standardmäßigen Schaummaterial mit einer Dicke von 0,5mm, einem Porendurchmesser im Bereich 50-150 $\mu$m und einer Porosität von zirka 90% ausgegangen, welches Material durch Walzen in der Dicke beliebig bis auf etwa 100 $\mu$m verdichtet werden kann. Das verdichtete Material kann anschließend optional noch gesintert werden. Durch die Verdichtung sinkt natürlich auch die Porosität, welche aber bei Bedarf im Zuge einer abschließenden Ätzbehandlung wieder gesteigert werden kann. Die Herstellungsmethode des standardmäßigen Schaummaterials basiert zwar auch auf der Verarbeitung eines Schlickers, unterscheidet sich jedoch von dem zuvor beschriebenen Verfahren nach US 3,111,396 dadurch, daß die eigentliche Schaumbildung durch ein Schäum- bzw. Treibmittel erfolgt, welches dem Schlicker zugesetzt wird. Heizleiterlegierungen - insbesondere aus der Gruppe der NiCr-Legierungen und CrFeAl-Legierungen ("Kanthal") können selbstverständlich auch verarbeitet werden. Der flächige Verbund 22 kann aus einer einzigen Schaumlage oder aus mehreren, miteinander versinterten Schaumlagen bestehen. Zur Steigerung der Stabilität und Festigkeit des flächigen Verbundes 22 kann der Schaum 37 optional auf eine dünne Trägerlage 38, beispielsweise auf ein Drahtgewebe, bestehend aus Edelstahl oder einer Heizleiterlegierung, aufgesintert werden. Bezüglich der Befestigung und Kontaktierung des Schaums 37 auf den plattenförmigen Kontakten 23 gilt Gleiches, wie schon in Zusammenhang mit den Ausführungsformen gemäß Fig. 15a und 15b ausgeführt wurde.

Alle zuvor beschriebenen Ausbildungsformen des flächigen Verbundes 22 stellen wohlgemerkt nur Ausführungsbeispiele dar. Die Erfindung ist auf diese Ausführungsbeispiele keineswegs beschränkt. So könnte beispielsweise ein flächiges Schaummaterial auf eine Metallfolie aufgesintert werden. Ferner könnte eine offenporige poröse Abscheidungsschicht auf eine Metallfolie aufgebracht werden - z.B. in Anlehnung an das Verfahren nach DE 1,950,439 (Peter Batzies et al.). Schließlich könnte der flächige Verbund selbstverständlich auch aus nichtmetallischen Materialien wie Kohlenstofffasern oder Graphitfasern, z.B. in Form von Geweben und Vliesen, oder aus Quarzglas, z.B. in Form eines körnigen oder faserigen Sinterverbundes gebildet werden, wobei im letzteren Fall eine auf die Glasoberfläche aufgebrachte leitende Dünnschicht die elektrische Widerstandsheizung bewirken könnte. Quarzglas zeichnet sich durch eine hohe Chemikalienbeständigkeit und Temperaturwechselbeständigkeit aus.

Fig. 16 und Fig. 16a zeigen eine beispielhafte Ausführungsform eines linienförmigen Verbundes 39, wobei im gegenständlichen Ausführungsbeispiel gleich drei zueinander parallel angeordnete linienförmige Verbunde 39a, 39b, 39c (39c ist nicht dargestellt) vorgesehen sind. Durch das Vorsehen mehrerer linienförmiger Verbunde kann die Verdampfungsfläche im Vergleich zu einem einzelnen linienförmigen Verbund deutlich gesteigert werden, wenn man von gleichen Summenquerschnitten ausgeht. Die einzelnen Verbunde müssen nicht zwingend identische Eigenschaften aufweisen. So ist es beispielsweise möglich, den einzelnen Verbunden 39a, 39b, 39c unterschiedliche Wärmekapazitäten oder/und unterschiedliche Heizelement-Eigenschaften zuzuordnen. Die sich daraus ergebenden Effekte wurden bereits früher dargestellt.

Die linienförmigen Verbunde sind im konkreten Beispiel als drahtförmige Sinterverbunde mit einer offenporigen Sinterstruktur 34 ausgebildet. Die drahtförmigen Sinterverbunde 39a, 39b, 39c lagern auf den plattenförmigen Kontakten 23 in Ausnehmungen 108, wodurch die drahtförmigen Sinterverbunde positioniert werden. Die elektrische Kontaktierung erfolgt im konkreten Ausführungsbeispiel durch eine Klemmung, indem die drahtförmigen Sinterverbunde 39a, 39b, 39c durch einen schafottartigen Preßstempel 40 gegen die plattenförmigen Kontakte 23 gedrückt werden (siehe Pfeil in Fig. 16a). Die Herstellung der drahtförmigen Sinterverbunde 39a, 39b, 39c erfolgt vorzugsweise mittels eines Extrudierverfahrens, z.B. gemäß AU 6,393,173 (Ralph E. Shackleford et al.). AU 6,393,173 beschreibt die Herstellung von Edelstahldrähten mit einem Drahtdurchmesser von 0,3-2,0mm. Dieser Durchmesserbereich deckt jedenfalls auch den bevorzugten Durchmesserbereich für den erfindungsgemäßen linienförmigen Verbund ab. Das Herstellungsverfahren basiert konkret auf der Extrusion einer Mixtur bestehend aus einem Metallpulver, einem Bindemittel und einem Plastifizierungsmittel, und der Sinterung des Extrudats. Das Metallpulver kann in einer körnigen, faserigen oder flockigen Form vorliegen. Um einen offenporigen, porösen Sinterkörper zu erhalten, ist das Verfahren zu adaptieren. Die Adaption besteht darin, daß der besagten Mixtur ein entfernbarer Füllstoff, z.B. sublimierbare Acrylharz-Kügelchen beigemischt werden. Die Acrylharz-Kügelchen sind Platzhalter, welche im Zuge einer Wärmebehandlung noch vor der eigentlichen Sinterung bei etwa 500°C praktisch rückstandslos sublimieren und Hohlräume zurücklassen. Gegebenenfalls sind das Binde- und Plastifizierungsmittel in Art und Menge der Füllstoffzugabe anzupassen. Die Partikelgröße der zu verarbeitenden Metallpulver und der Acrylharz-Kügelchen ist so abzustimmen, daß der mittlere Porendurchmesser des resultierenden homogenen Sinterverbundes möglichst >10$\mu$m ist; dadurch wird eine ausreichend schnelle Infiltration des Dochts mit dem flüssigen Material 16 sichergestellt. Statt Edelstahlpulver können selbstverständlich auch Pulver aus Heizleiterlegierungen - insbesondere aus der Gruppe der NiCr-Legierungen und CrFeAl-Legierungen ("Kanthal") verfahrensgemäß extrudiert und versintert werden.

Allgemein gilt, daß die Verbunde 22 und 39 vor ihrer Montage gereinigt, und die Oberfläche der Kapillarstruktur aktiviert werden sollte. Diese Maßnahme bewirkt eine verbesserte Benetzung des Verbundmaterials durch das flüssige Material 16 und damit verbunden eine schnellere Infiltration des Dochts. Im Fall von Edelstahl reicht beispielsweise eine Behandlung mit einer 20%-igen Phosporsäure aus, um die zuvor erwähnten Effekte zu erzielen.

Im Folgenden soll die Versorgung des Verbundes 22, 39 mit dem flüssigen Material 16 näher beschrieben werden. Die folgenden Ausführungen gelten für flächige wie linienförmige Verbunde 22, 39 gleichermaßen, auch wenn die Figuren auf die Darstellung nur einer Ausführungsform des Verbundes beschränkt sind. Wie Fig. 12a und Fig. 17 sowie Fig. 16 und Fig. 16a zeigen, ragt der Verbund 22, 39 mit einem Ende in einen Kapillarspalt 41. Der Kapillarspalt 41 speist den Docht des Verbundes mit dem flüssigen Material 16; wie den Figuren entnommen werden kann, ist der Querschnitt des Kapillarspalts 41 größer als der Querschnitt des Verbundes 22, 39. Dies hat den Effekt, daß das flüssige Material 16 hauptsächlich durch den lichten Querschnitt des Kapillarspalts 41 zur Verdampfungszone strömt, wodurch der Docht schneller infiltriert werden kann, und die Wartezeit zwischen zwei Zügen bzw. Inhalationen verkürzt werden kann. Der Effekt wirkt zumindest bis zur Mündung des Kapillarspalts 41 in die Kammer 21. Ab diesem Punkt ist allein der Docht des Verbundes 22, 39 für den Flüssigkeitstransport verantwortlich. Der Kapillarspalt 41 wird im Grunde durch einen der beiden plattenförmigen Kontakte 23 und ein auf diesen flächig aufgesetztes Oberteil 42 gebildet, indem in das Oberteil 42 und in den plattenförmigen Kontakt 23 entprechende, den Kapillarspalt 41 bildende Ausnehmungen eingearbeitet sind - siehe Fig. 12a und Fig. 17. Es sei angemerkt, daß auch schon eine einzelne Ausnehmung, sei sie im Oberteil 42 oder im plattenförmigen Kontakt 23 angeordnet, ausreichen würde, um einen Kapillarspalt 41 auszubilden. Bei Verwendung eines flächigen Verbundes 22 ist es jedenfalls vorteilhaft, die Ausnehmung im plattenförmigen Kontakt 23 anzuordnen, da in diesem Fall die Ausnehmung gleichzeitig als Positionierhilfe für den Verbund 22 genutzt werden kann. Das Oberteil 42 ist mit dem plattenförmigen Kontakt 23 vorzugweise durch eine Klebeverbindung gefügt und besteht aus einem mit dem flüssigen Material 16 gut benetzbaren Werkstoff, vorzugsweise aus Leichtmetall oder aus einem benetzbaren Kunststoff; die Benetzbarkeit und im Übrigen auch die Verklebbarkeit von Kunststoffen kann durch eine Oberflächenaktivierung, beispielsweise durch eine Plasma-Behandlung mit Sauerstoff als Prozessgas erheblich verbessert werden.

Weiter stromaufwärts wird der Kapillarspalt 41 durch zwei zueinander parallel und beabstandet angeordnete dünne Platten 43 gebildet (siehe Fig. 17), wobei eine Platte mit dem Oberteil 42 und die andere Platte mit dem plattenförmigen Kontakt 23 vorzugsweise durch eine Klebeverbindung verbunden sind. Die Platten 43 können beispielsweise aus einem Edelstahlband gestanzt werden. Wie Fig. 18-20 am besten zeigen, ragen die den Kapillarspalt 41 bildenden Platten 43 über einen Fortsatz 44 in ein Reservoir 45 hinein. Das Reservoir 45 schließt direkt an den Flüssigkeitsbehälter 4 an und ist von diesem nur durch den klappenartigen, öffenbaren Verschluß 18 getrennt. Der öffenbare Verschluß 18 wird mit Hilfe eines Stiftes 46 geöffnet. Der Stift 46 ist im Gehäuse 3 axial verschiebbar gelagert und besteht vorzugsweise aus Edelstahl. Ein erstes Ende 47 des Stiftes 46 ist gegen den öffenbaren Verschluß 18 gerichtet. Ein zweites Ende 48 ragt bei noch geschlossenem Verschluß 18 aus der äußeren Oberfläche des Gehäuses 3 fortsatzartig heraus. Das zweite Ende 48 des Stiftes 46 steht mit einem der beiden Kontaktelemente 20 des Inhalatorteils 1 in einer stößelartigen Wirkverbindung, indem das Kontaktelement 20 im Zuge der Kopplung der Inhalatorkomponente 2 mit dem Inhalatorteil 1 gegen das zweite Ende 48 des Stiftes 46 drückt, und der Stift 46 dadurch in das Gehäuse 3 verschoben wird. Die vom Kontaktelement 20 ausgeübte Druckkraft wird vom Stift 46 auf den öffenbaren Verschluß 18 übertragen. Der öffenbare Verschluß 18 weist auf seinem Umfang eine Materialschwächung 49 auf, welche so dimensioniert ist, daß sie bei Druckbeaufschlagung durch den Stift 46 gleich einer Sollbruchstelle über einen weiten Umfangsbereich aufreißt, jedoch auf einer Seite ein Scharnier 50 ausbildet. Auf diese Weise wird bewirkt, daß sich der öffenbare Verschluß 18 wie eine Klappe öffnet. Der Stift 46 weist nahe dem ersten Ende 47 eine Querschnittserweiterung 51 auf, welche in der Art eines Anschlages verhindert, daß der Stift aus dem Gehäuse 3 herausgleiten oder entnommen werden kann.

Die Versorgung des Verbundes 22, 39 mit dem flüssigen Material 16 soll im Folgenden zusammenfassend erläutert werden, wobei die Fig. 18 und Fig. 20 die Strömungsverhältnisse durch Pfeile veranschaulichen: im Zuge der Kopplung der Inhalatorkomponente 2 mit dem wiederverwendbaren Inhalatorteil 1 wird der klappenartige Verschluß 18 über den Stift 46 geöffnet, und in der Folge das Reservoir 45 vom flüssigen Material 16 unter Schwerkrafteinfluß geflutet. In Fig. 19 sind die Flüssigkeitsstände vor und nach dem Fluten eingezeichnet. Der Kapillarspalt 41 saugt das flüssige Material 16 über den Fortsatz 44 an und führt es dem Verbund 22, 39 zu, wodurch schließlich der Docht vollständig mit dem flüssigen Material 16 infiltriert wird. Der durch die Platten 43 gebildete Fortsatz 44 soll vermeiden, daß sich im Mündungsbereich des Kapillarspalts 41 Gasblasen ansiedeln, welche die kapillare Kopplung behindern könnten. Desweiteren ist in den plattenförmigen Kontakt 23 ein Belüftungskanal 52 eingearbeitet, welcher das Reservoir 45 mit der Kammer 21 verbindet. Die Funktion des Belüftungskanals 52 wurde bereits früher erläutert. Der Belüftungskanals 52 mündet in die Kammer 21 vorzugsweise an einer Stelle stromaufwärts des Verbundes 22, 39, da in diesem Bereich der Kammer 21 kaum Kondensatablagerungen zu erwarten sind; solche Kondensatablagerungen könnten nämlich den Belüftungskanals 52 blockieren oder über den Belüftungskanals 52 in das Reservoir 45 gelangen und das dort gespeicherte flüssige Material 16 kontaminieren. Schließlich ist in das Oberteil 42 ein Pufferspeicher 53 integriert - siehe auch Fig. 11 und Fig. 17, dessen Wirkung ebenfalls bereits früher erläutert wurde. Der Pufferspeicher 53 besteht im gegenständlichen Ausführungsbeispiel aus parallel zueinander angeordneten Schlitzen 54, welche in das Oberteil 42 eingearbeitet sind. Die Schlitze 54 kommunizieren einerseits über Öffnungen 55 mit dem Kapillarspalt 41 und andererseits über einen Belüftungsspalt 56 mit der Kammer 21. Die Kapillarität der Schlitze 54 bewirkt, daß das flüssige Material 16 aus dem Reservoir 45 über den Kapillarspalt 41 und über die Öffnungen 55 in die Schlitze 54 strömt, wo es zwischengespeichert wird, und vom Docht nach Bedarf wieder abgezogen werden kann.

Fig. 9-12 zeigen ferner eine in der Kammer 21 angeordnete Kondensatbindeeinrichtung bestehend aus zwei offenporigen, saugfähigen Körpern oder Schwämmen 57. Die Wirkungen der Kondensatbindeeinrichtung sowie deren Notwendigkeit für die erfindungsgemäße Inhalatorkomponente wurden bereits früher eingehend erläutert. Die beiden Schwämme 57 sind plattenförmig ausgebildet und beabstandet und parallel zueinander angeordnet, wobei der Verbund 22 von den beiden Schwämmen 57 beidseitig überdeckt wird. Zwischen den beiden Schwämmen 57 bildet sich ein Strömungskanal 58 aus, in welchem die Bildung des Dampf-Luft-Gemisches oder/und Kondensationsaerosols stattfindet. Der Hauptanteil der Kondensatrückstände wird an den, den Strömungskanal 58 bildenden Wandabschnitten 59 der Schwämme 57 abgeschieden und sofort von der offenen Porenstruktur der Schwämme aufgesaugt. Die Schwämme 57 sind an zwei gegenüberliegenden Wänden der Kammer 21 zum Beispiel mittels einer Klebeverbindung befestigt, füllen den überwiegenden Teil der Kammer 21 aus und bestehen vorzugsweise aus einem hochporösen, formbeständigen und möglichst feinporigen Material. Bei Verwendung von grobporigem Material besteht nämlich die Gefahr, daß bei abrupten Bewegungen bzw. Beschleunigungen der Inhalatorkomponente 2 die Kapillarkräfte des Schwammmaterials nicht mehr ausreichen, um das flüssige Kondensat zurückzuhalten, und Teile des Kondensats aus den Schwämmen 57 herausgeschleudert werden. Als Schwammmaterial besonders geeignet erweisen sich Faserverbunde, gebildet aus thermisch oder mit Hilfe eines Bindemittels miteinander verbundenen Natur- oder Chemiefasern. Die Firma Filtrona Richmond Inc., www.filtronaporoustechnologies.com, ist auf die Herstellung solcher Faserverbunde spezialisiert, wobei sowohl mittels Triacetin gebundene Celluloseacetat-Fasern als auch thermisch gebundene Polyolefin- und Polyesterfasern verarbeitet weden.

Die Schwämme 57 sind zum Oberteil 42 und zu dem mit dem Oberteil 42 verbundenen plattenförmigen Kontakt 23 etwas beabstandet angeordnet, sodaß sich ein Spalt 60 bildet. Der Spalt 60 gewährleistet, daß der Belüftungskanal 52 sowie der Belüftungsspalt 56 ungehindert mit der Kammer 21 kommunizieren können. Die Schwämme 57 sind so zu dimensionieren, daß deren Porenvolumen die zu erwartende Menge an gebildeten Kondensatrückständen aufzunehmen vermag. Die Kondensatmenge hängt in erster Linie vom Anteil des flüssigen Materials 16 an niedrig siedenden Fraktionen mit hohem Dampfdruck sowie vom Luftdurchsatz durch die Lufteinlaßöffnung 26 bzw. durch den Strömungskanal 58 ab. Je weniger Luft durchgesetzt wird, umso weniger Dampf kann die Luft bis zur Sättigung aufnehmen.

Wie Fig. 9-10 und Fig. 12 zeigen, ist den Schwämmen 57 stromabwärts vom Verbund 22 ein Kühler 61 nachgeordnet, welcher im konkreten Ausführungsbeispiel aus einem porösen Füllmaterial 61 besteht, dessen Poren vom gebildeten Dampf-Luft-Gemisch oder/und Kondensationsaerosol durchströmt werden. Die wesentlichen Wirkungen des Kühlers bzw. Füllmaterials 61 wurden bereits früher eingehend erläutert. Das Füllmaterial 61 befindet sich in einem Füllraum 62, welcher strömungseingangsseitig durch eine Lochwand 63, strömungsausgangsseitig durch das Mundstück 5, und mantelseitig durch das Gehäuse 3 und durch eine Wand des Flüssigkeitsbehälters 4 begrenzt ist. Die Lochwand 63 stützt das Füllmaterial 61 und steift gleichzeitig das Gehäuse 3 aus. Die Lochwand 63 ist etwas beabstandet zu den Schwämmen 57 angeordnet - siehe Fig. 12. Dadurch wird erreicht, daß sich das aus dem Strömungskanal 58 austretende Dampf-Luft-Gemisch oder/und Kondensationsaerosol noch vor der Lochwand 63 gleichmäßig über den ganzen Querschnitt des Füllmaterials 61 verteilen kann, und das Füllmaterial 61 gleichmäßig durchströmt wird. Damit das Füllmaterial 61 nicht aus den Löchern der Lochwand 63 austreten kann, ist zwischen dem Füllmaterial 61 und der Lochwand 63 ein erstes Drahtgewebe 64 angeordnet. Mundstückseitig wird das Füllmaterial 61 durch ein zweites Drahtgewebe 65 begrenzt, welches verhindert, daß das Füllmaterial in den Mundstückkanal 66 oder gar in die Mundhöhle des Benutzers gelangen kann. Zwischen dem zweiten Drahtgewebe 65 und dem Mundstückkanal 66 bildet das Mundstück eine Sammelkammer 67 aus, welche bewirkt, daß das Füllmaterial 61 auch im Endabschnitt gleichmäßig durchströmt wird. Das zweite Drahtgewebe 65 ist vorteilhafterweise direkt am Mundstück 5 befestigt, z.B. auf dieses aufgeschmolzen. Im Zuge der Montage wird zuerst das erste Drahtgewebe 64 auf die Lochwand 63 aufgelegt. Danach wird eine vordefinierte Menge Füllmaterial 61 in den Füllraum 62 eingebracht, wobei das Befüllen auch mehrstufig erfolgen kann, und das Füllmaterial 61 nach jeder Teilbefüllung zwischenverdichtet wird. Auf diese Weise kann eine homogene Fülldichte erzielt werden. Alternativ könnte das Füllmaterial 61 bereits außerhalb der Inhalatorkomponente 2 beispielsweise in Papierzylindern mit an den Füllraum 62 angepaßtem Querschnitt vorgepackt werden, und die Packung in den Füllraum 62 eingesetzt werden. Solche Packungen können auf wirtschaftliche Weise aus einem endlosen Strang gewonnen werden. Schließlich wird das Mundstück 5 montiert, und der Füllraum 62 verschlossen.

Das Füllmaterial kann beispielsweise aus einem Regeneratormaterial bestehen. Vor allem wenn das flüssige Material 16 Nikotin enthält, erweist es sich als besonders vorteilhaft, als Füllmaterial 61 Tabak zu verwenden. In Prototypen wurden auf Basis von Feinschnitt-Tabak und einem Füllvolumen von etwa 7cm3 hinsichtlich der organoleptischen Wirkungen des verabreichten Dampf-Luft-Gemisches bzw. Kondensationsaerosols ausgezeichnete Ergebnisse erzielt. Der Tabak kann zusätzlich aromatisiert werden, indem ihm aromatische Zusätze und ätherische Öle wie beispielsweise Tabakextrakt, Tabakaromaöle, Menthol, Kaffeeextrakt, Tabakrauch-Kondensat oder eine flüchtige aromatische Fraktion eines Tabakrauch-Kondensats zugesetzt werden. Selbstverständlich ist die Erfindung nicht auf diese Auswahl beschränkt.

Die Fülldichte des Füllmaterials 61 bestimmt den Strömungswiderstand, welchen das Füllmaterial 61 dem Dampf-Luft-Gemisch bzw. Kondensationsaerosol entgegensetzt; die Fülldichte ist mit dem Strömungswiderstand der Strömungsdrossel 28 so abzustimmen, daß der resultierende Strömungswiderstand innerhalb des bereits genannten Bereiches von 12-16mbar bei einem Luftdurchsatz von 1,05L/min liegt. Grundsätzlich ist es auch möglich, auf die Strömungsdrossel 28 ganz zu verzichten, und den gewünschten Strömungswiderstand allein durch das Füllmaterial 61 zu erzeugen, indem dessen Fülldichte entsprechend erhöht wird. Generell sollte jedoch beachtet werden, daß eine Filterwirkung unerwünscht ist; die in der Kammer 21 erzeugten Aerosolpartikel sollten das Füllmaterial 61 möglichst ohne Verluste durchsetzen können. Die alternative Ausführungsvariante ohne Strömungsdrossel 28 hat außerdem Auswirkungen auf die sensortechnische Erfassung des Zugbeginns, welche Auswirkungen später noch näher erläutert werden. Enthält das Füllmaterial 61 Tabak oder/und Aromastoffe, sollte die Inhalatorkomponente 2 bis zu ihrer Verwendung in einer luftdichten Verpackung aufbewahrt werden, um ein Entweichen von Aromastoffen zu verhindern. Auch noch nach der Kopplung der Inhalatorkomponente 2 mit dem Inhalatorteil 1 besteht die Möglichkeit, durch Verschließen des Mundstückkanals 66 beispielsweise mittels einer Kappe oder eines Stöpsels (nicht dargestellt) ein Entweichen von Aromastoffen sowie eine Verdunstung und ein Entweichen von Fraktionen des im Docht gespeicherten flüssigen Materials 16 weitgehend auszuschließen.

Fig. 21-22 zeigen ein zweites Ausführungsbeispiel eines Inhalators, und Fig. 23 zeigt eine auswechselbare Inhalatorkomponente für diesen Inhalator. Der Inhalator ist im konkreten Beispiel als klassischer Inhalator ausgebildet und basiert weitgehend auf der Anordnung nach Fig. 9-10, unterscheidet sich von dieser aber dadurch, daß eine bedeutend größere Luftmenge durchgesetzt werden kann, wodurch eine direkte Lungeninhalation in einem einzigen Schritt ermöglicht wird. Konkret unterscheidet sich der Inhalator von der Anordnung gemäß Fig. 9-10 dadurch, daß sowohl die Strömungsdrossel 28 als auch der zweite offenporige Körper 61 entfallen, und der Mundstückkanal 66 einen wesentlich größeren Querschnitt aufweist. Auf diese Weise wird der Strömungswiderstand maßgebend reduziert. Ein weiterer wesentlicher Unterschied besteht darin, daß der Hauptanteil der durchgesetzten Luft den Verbund 22, 39 gar nicht passiert, sondern erst stromabwärts von diesem in den Inhalator einströmt. Zu diesem Zweck sind stromabwärts vom Verbund 22, 39 auf gegenüberliegenden Seiten des Gehäuses 3 zwei Bypassöffnungen 68 angeordnet, deren gemeinsamer Querschnitt wesentlich größer ist als der Querschitt der Lufteinlaßöffnung 26. An die beiden Bypassöffnungen 68 schließen zwei vom Gehäuse 3 ausgebildete Leitschaufeln 69 an, welche in Richtung des Mundstückkanals 66 weisen und aufeinander zustreben, und deren freie Enden bzw. Spitzen 70 eine düsenförmige Mündungsöffnung 71 bilden, durch welche das gebildete Dampf-Luft-Gemisch oder/und Kondensationsaerosol aus der Kammer 21 ausströmt und sich anschließend mit der aus den Bypassöffnungen 68 einströmenden Luft mischt. Die Wirkungen der Leitschaufeln 69 wurden bereits früher erläutert. Zur besseren Durchmischung des Dampf-Luft-Gemisches oder/und Kondensationsaerosols mit der durch die Bypassöffnungen 68 einströmenden Bypass-Luft kann im Mundstückkanal 66 optional ein Strömungshomogenisator 72 angeordnet sein - siehe Fig. 22. Der Strömungshomogenisator 72 kann beispielsweise aus einem vliesartigen Synthetikfaser-Material gefertigt werden. Die Firma Freudenberg Vliesstoffe KG, www.freudenberg-filter.com, bietet ein solches Material in Form von Matten/ Platten unter der Bezeichnung Viledon®-Filtermatten an. Das Material kann nach Kundenspezifikation angefertigt werden. Insbesondere können die Materialeigenschaften so abgestimmt werden, daß das Endfabrikat für die feinen Partikel des erzeugten Kondensationsaerosols weitestgehend durchlässig ist, und der Strömungswiderstand im bereits früher spezifizierten Sollbereich liegt. Die Matten/ Platten werden aus Polyolefinfasern (PE, PP) oder Polyesterfasern gefertigt und können durch Stanzen weiterverarbeitet werden.

Fig. 24-25 zeigen eine auswechselbare Inhalatorkomponente 2 eines Inhalators mit einem alternativen Flüssigkeitsbehälter-System. Obwohl die auswechselbare Inhalatorkomponente 2 im konkreten Beispiel eine Inhalatorkomponente für die Verwendung in einem klassischen Inhalator darstellt, kann das dargestellte alternative Flüssigkeitsbehälter-System genauso in einer Inhalatorkomponente eines Zug-Inhalators, wie zuvor beschrieben, Verwendung finden. Wie die Figuren zeigen, ist der Flüssigkeitsbehälter 4 im Gehäuse 3 längs einer Verschiebeachse Y zwischen zwei Anschlagpositionen manuell verschiebbar angeordnet. Die Fig. 24b zeigt den Flüssigkeitsbehälter 4 in der ersten Anschlagposition, welche gleichzeitig seine Ausgangsstellung festlegt. Die erste Anschlagposition wird durch einen vom Mundstück 5 gebildeten Vorsprung 73 im Zusammenwirken mit einem vom Flüssigkeitsbehälter 4 ausgebildeten Zapfen 74 definiert. Der Vorsprung 73 macht es unmöglich, den gegebenenfalls Arzneimittel oder/und Gifte enthaltenden Flüssigkeitsbehälter 4 aus der Inhalatorkomponente 2 zu entnehmen. Der Zapfen 74 bewirkt gleichzeitig eine Verdrehsicherung des Flüssigkeitsbehälters 4, indem der Zapfen 74 in eine korrespondierende Nut 75 im Gehäuse 3 eingreift. Der Flüssigkeitsbehälter 4 ragt in der Ausgangsstellung mit einem Endabschnitt seitlich neben dem Mundstück 5 aus dem Gehäuse 3 heraus. Der verschiebbare Flüssigkeitsbehälter 4 kann auf einfache Weise in seine zweite Anschlagposition verschoben werden, indem der Benutzer auf das herausragende Ende des Flüssigkeitsbehälters 4 drückt. Der Flüssigkeitsbehälter 4 wird hierbei um die Strecke s verschoben. Der zweite Anschlag wird durch das Oberteil 42 und dem mit diesem verbundenen plattenförmigen Kontakt 23 gebildet. Die Entüftungsöffnung 76 und der Entlüftungskanal 77 verhindern, daß sich während des Verschiebevorgangs störende Luftpolster bilden. Der Flüssigkeitsbehälter 4 weist auf der dem zweiten Anschlag zugewandten Stirnseite zwei Öffnungen 78, 79 auf, welche auf der Behälterinnenseite mittels einer Foliendichtung 80 verschlossen sind. Der Kapillarspalt 41 ist mit der früher bereits beschriebenen Anordnung im Wesentlichen ident. Die Platten 43 bilden wieder einen Fortsatz in Form eines ersten Dorns 81. Der erste Dorn 81 ist so positioniert, daß er mit der ersten Öffnung 78 fluchtet, und diese in der zweiten Anschlagposition durchdringt. Das schräg angespitzte Ende des ersten Dorns 81 durchschneidet gleichzeitig die Foliendichtung 80 und tritt mit dem flüssigen Material 16 in Kontakt, wodurch schließlich die kapillare Kopplung mit dem Kapillarspalt 41 hergestellt wird. Analog verhält es sich mit dem Belüftungskanal 52: dieser ist im konreten Ausführungsbeispiel im Gegensatz zu der früher beschriebenen Anordnung in das Oberteil 42 integriert und bildet wie der Kapillarspalt 41 an dem, dem Flüssigkeitsbehälter 4 zugewandten Ende einen Fortsatz oder zweiten Dorn 82 aus, welcher so positioniert ist, daß er mit der zweiten Öffnung 79 im Flüssigkeitsbehälter 4 fluchtet, und diese in der zweiten Anschlagposition durchsetzt. Das zweite Ende des Belüftungskanals kommuniziert wiederum mit der Kammer 21 (nicht dargestellt). Die Versorgung des Verbundes 22, 39 mit dem flüssigen Material 16 funktioniert genauso wie bereits früher beschrieben. Im Auslieferungszustand der Inhalatorkomponente 2 befindet sich der Flüssigkeitsbehälter 4 in seiner Ausgangsstellung, also in der ersten Anschlagposition. Der Flüssigkeitsbehälter 4 wird vorzugsweise erst kurz vor der Benutzung der Inhalatorkomponente 2 in die zweite Anschlagposition verschoben und mit dem Kapillarspalt 41 gekoppelt. Um eine vorzeitige, unbeabsichtigte Kopplung auszuschließen, ist der Flüssigkeitsbehälter 4 in seiner Ausgangsstellung fixiert. Die Fixierung kann, wie Fig. 24b zeigt, beispielsweise mittels eines halbkreisrunden Arretier-Plättchens 109 erfolgen, welches über Mikrostege 83 einerseits mit dem Flüssigkeitsbehälter 4 und andererseits mit dem Gehäuse 3 verbunden ist. Das Arretier-Plättchen 109 stellt auf diese Weise eine starre Verbindung zwischen dem Flüssigkeitsbehälter 4 und dem Gehäuse 3 her. Durch manuelle Krafteinwirkung auf das Arretier-Plättchen 109 - beispielsweise durch mehrmaliges Verbiegen desselben - können die Mikrostege 83 gebrochen werden, und die Fixierung des Flüssigkeitsbehälters 4 aufgehoben werden. Alternativ kann der Flüssigkeitsbehälter 4 auf einfache Weise mittels eines Klebebandes fixiert werden (nicht dargestellt). Bezüglich der Materialauswahl für den Flüssigkeitsbehälters 4 wurden bereits früher Hinweise gegeben, welche gleichermaßen für das konkrete Ausführungsbeispiel gelten.

Fig. 26-27 zeigen eine auswechselbare Inhalatorkomponente 2 eines Inhalators mit einem weiteren alternativen Flüssigkeitsspeicher-System. Obwohl die auswechselbare Inhalatorkomponente 2 im konkreten Beispiel eine Inhalatorkomponente für die Verwendung in einem klassischen Inhalator darstellt, kann das dargestellte alternative Flüssigkeitsspeicher-System genauso in einer Inhalatorkomponente eines Zug-Inhalators, wie früher beschrieben, Verwendung finden. Der Flüssigkeitsspeicher beinhaltet im konkreten Ausführungsbeispiel einen mit dem flüssigen Material 16 getränkten offenporigen Schaumstoff 84. Der Verbund 22, 39 ist sandwichartig zwischen dem Schaumstoff 84 und einem der beiden plattenförmigen Kontakte 23 eingeklemmt, wodurch der Docht mit dem flüssigen Material 16 kapillar gekoppelt wird. Der Schaumstoff 84 wird von einem Patronengehäuse 85 gehalten, mit welchem er zusammen eine auswechselbare Patrone 86 bildet. Die Patrone 86 wird in eine entsprechende Ausnehmung 87 im Gehäuse 3 eingesetzt. Die Ausnehmung 87 ist durch einen Deckel 88 nach außen hin luftdicht verschlossen.

Der Deckel 88 ist mittels einer Schnappverbindung 89 am Gehäuse 3 fixiert. Diese Fixierung bewirkt außerdem, daß der Deckel 88 auf die Patrone 86 eine Druckkraft in Richtung des Verbundes 22, 39 ausübt. Wie Fig. 28 näher zeigt, lagert der Verbund 22, 39 auf einer Erhebung 90 des plattenförmigen Kontaktes 23. Die Erhebung 90 bewirkt zusammen mit der auf die Patrone einwirkenden Druckkraft eine Kompression des Schaumstoffs 84 - siehe Kompressionshub h. Die Kompression hat den Effekt, daß eine kleine Menge des flüssigen Materials 16 im Kontaktbereich zum Verbund aus dem Schaumstoff 84 herausgedrückt wird, welche Menge ausreicht, um eine kapillare Kopplung zwischen einer neu eingesetzten Patrone 86 und dem Docht sicherzustellen. Das Patronengehäuse 85 ist auf der dem Deckel 88 zugewandten Seite gelocht. Die Belüftungslöcher 91 kommunizieren über eine Aussparung 92 im Deckel 88 mit der Kammer 21 und bewirken auf diese Weise einen Druckausgleich zwischen dem in den Poren des Schaumstoffs 84 gebundenen flüssigen Material 16 und der Kammer 21. Der Schaumstoff 84 besteht vorzugsweise aus einem feinporigen Polyether-PUR-Schaummaterial, welches zusätzlich verdichtet sein kann. In Prototypen wurde zwei- bis dreifach verdichtetes Schaummaterial mit der Bezeichnung "Jet 6" des Herstellers Fritz Nauer AG, www.foampartner.com mit Erfolg verwendet. Das soeben dargestellte Flüssigkeitsspeicher-System hat den Nachteil, daß die Patrone 86 aus der Inhalatorkomponente 2 entfernt werden kann. Damit sind natürlich Gefahren verbunden, beispielsweise die Gefahr, daß die relativ kleine Patrone 86 von Kleinkindern verschluckt wird. Das Flüssigkeitsspeicher-System eignet sich daher nicht für die Speicherung von Arzneimitteln oder/und Giften wie beispielsweise Nikotin.

Im Folgenden sollen noch weitere, allgemeine Bestandteile des Inhalators näher beschrieben werden, welche Bestandteile in allen Ausführungsbeispielen vorhanden sind: wie Fig. 6, Fig. 9 und Fig. 19 zeigen, ragen die plattenförmigen Kontakte 23 der auswechselbaren Inhalatorkomponente 2 aus der äußeren Oberfläche des Gehäuses 3 in Form zweier Steckkontakte 93 heraus. Die Steckkontakte 93 bilden mit korrespondierenden Federkontakten 94 im Zuge der Kopplung der Inhalatorkomponente 2 mit dem Inhalatorteil 1 elektrische Kontakte aus, über welche dem Heizelement die elektrische Energie zur Verdampfung des flüssigen Materials 16 zugeführt wird. Die Federkontakte 94 sind Teil der Kontaktelemente 20 und vorzugsweise durch eine Schweißverbindung mit diesen verbunden - siehe auch Fig. 4-5. Die Kontaktelemente 20 bestehen vorzugsweise aus einem metallischen Kontaktwerkstoff und können beispielsweise von der Firma Ami Doduco GmbH, www.amidoduco.com gefertigt werden. Für den Fall, daß für die plattenförmigen Kontakte 23 aus den bereits genannten Gründen dasselbe oder ein ähnliches Material verwendet wird wie für das Heizelement - z.B. Edelstahl, ist es aufgrund der unzureichenden Leitfähigkeit dieses Materials erforderlich, die plattenförmigen Kontakte 23 zumindest im Bereich der Steckkontakte 93 beispielsweise galvanisch mit einer Leitschicht aus Gold, Silber, Palladium oder/und Nickel zu überziehen, wodurch der elektrische Kontaktwiderstand wesentlich verringert wird. Die Kontaktelemente 20 beziehen die elektrische Energie über zwei Drähte 95, welche die Kontaktelemente 20 mit der Platine 11 verbinden - siehe Fig. 4-5. Die Drähte 95 sind vorzugsweise beidseitig durch eine Lötung befestigt. Zusammenfassend sei nochmals darauf hingewiesen, daß die Kontaktelemente 20 bis zu drei verschiedene Aufgaben erfüllen: erstens übertragen sie, wie zuvor gerade beschrieben, die elektrische Energie von der Platine 11 auf die plattenförmigen Kontakte 23. Zweitens bilden sie seitliche Rastnasen 9 aus, welche *mit* den Schnapphaken 8 des Gehäuses 3 zusammenwirken, wodurch die Schnappverbindung zwischen der Inhalatorkomponente 2 und dem Inhalatorteil 1 verwirklicht wird. Und drittens bildet eines der beiden Kontaktelemente 20 einen Anschlag für den Stift 46 aus, wodurch die stößelartige Wirkverbindung zur Öffnung des Flüssigkeitsbehälters 4 hergestellt wird. Die letztere Aufgabe tritt wohlgleich nur in einer Ausführungsvariante des Inhalators und seines Flüssigkeitsbehälter-Systems in Erscheinung.

Zur lagegenauen Kopplung der Inhalatorkomponente 2 mit dem Inhalatorteil 1 ist eine *Positioniereinrichtung* vorgesehen, welche aus einem am Trägergehäuse 10 angeordneten Zentriervorsprung 96, und einer mit diesem korrespondierenden, und am Gehäuse 3 angeordneten Zentrierausnehmung 97 besteht - siehe Fig. 3, Fig. 6, Fig. 10 und Fig. 12. Der Zentriervorsprung 96 weist zwei Entlüftungslöcher 98 auf, welche die Zentrierausnehmung 97 im Zuge der Kopplung entlüften.

Fig. 29 zeigt eine auswechselbare Inhalatorkomponente 2 eines Inhalators, welche sich von den zuvor dargestellten Inhalatorkomponenten dadurch unterscheidet, daß sie zwei nebeneinander angeordnete flächige Verbunde 22a und 22b aufweist. Die flächigen Verbunde 22a und 22b können beispielsweise einen Aufbau aufweisen, wie er bereits in den Figuren 14-15 im Detail beschrieben wurde. Die flächigen Verbunde 22a und 22b bzw. deren Heizwiderstände sind elektrisch miteinander in Reihe geschaltet. Die Reihenschaltung bewirkt, daß sich der resultierende Heizwiderstand bei unveränderter Verbund-Spannweite verdoppelt, wenn man gleichgroße Einzelwiderstände der Verbunde 22a und 22b zugrundelegt. Die vorteilhaften Effekte dieser Widerstandserhöhung wurden bereits früher dargelegt. Grundsätzlich ließe sich der Heizwiderstand des Verbundes auch durch eine Vergrößerung der Verbund-Spannweite steigern. Dies hätte jedoch sehr nachteilige Auswirkungen auf die Infiltrationsdauer, das ist jene Dauer, welche das flüssige Material 16 benötigt, um den Docht nach einer Verdampfung von neuem vollständig zu infiltrieren. Die Infiltrationsdauer würde sprunghaft zunehmen. Geht man beispielhaft von den Verbund-Spezifikationen nach Tabelle 1 aus, und schaltet man zwei Verbunde 22a und 22b mit einer Verbund-Breite von jeweils 4mm und einer Ätzrate von 25% in Reihe, so resultiert hieraus ein Heizelement-Widerstand von etwa 275mOhm. Bei diesem Widerstandswert bietet es sich an, die Verbund-Spannweite im Hinblick auf eine kurze Infiltrationsdauer noch weiter zu verkleinern, z.B. auf 12mm, wodurch der Heizelement-Widerstand auf einen Wert von etwa 235mOhm absinken würde. Die beiden Verbunde 22a und 22b können optional auch verschiedene Widerstandswerte aufweisen, was sich am einfachsten dadurch realisieren läßt, daß den beiden Verbunden unterschiedliche Verbund-Breiten zugeordnet werden. Auf diese Weise kann der Verdampfungsprozeß räumlich variiert werden. Ferner können die beiden Verbunde 22a und 22b optional von unterschiedlichen Quellen flüssigen Materials gespeist werden. Mittels der beiden letzteren Ausgestaltungsoptionen ist es möglich, auf den Aerosolbildungsprozeß und letztlich auf die Eigenschaften des gebildeten Kondensationsaerosols noch gezielter Einfluß zu nehmen. Beispielsweise kann auf diese Weise der Verdampfungsprozeß in der Destillationszone einer Zigarette räumlich wie zeitlich näherungsweise nachgebildet werden.

Die Verbunde 22a und 22b lagern wiederum mit ihren Endabschnitten auf elektrisch leitenden, plattenförmigen Kontakten, und ihre Heizelemente sind mit den Kontakten elektrisch kontaktiert. Im Unterschied zu den früher beschriebenen Ausführungsbeispielen sind die plattenförmigen Kontakte auf einer Seite in zwei Kontaktteile 23a und 23b gesplittet, welche voneinander elektrisch isoliert sind.

Der erste flächige Verbund 22a lagert mit einem Endabschnitt auf dem Kontaktteil 23a, und der zweite flächige Verbund 22b lagert mit einem Endabschnitt auf dem Kontaktteil 23b. Auf der gegenüberliegenden Seite lagern die beiden Verbunde 22a und 22b mit ihren Endabschnitten auf einem gemeinsamen plattenförmigen Kontakt 23c. Der plattenförmige Kontakt 23c verbindet die beiden Verbunde 22a und 22b elektrisch miteinander. Der plattenförmige Kontakt 23c bewirkt die eigentliche elektrische Reihenschaltung, während die Zuleitung der elektrischen Energie zu den Verbunden 22a und 22b über die Kontaktteile 23a und 23b erfolgt. Die elektrische Kopplung mit dem wiederverwendbaren Inhalatorteil 1 erfolgt wieder über die Steckkontakte 93, deren Anordnung identisch ist mit dem Kopplungsschema der zuvor dargestellten Ausführungsbeispiele, vgl. Fig. 6, Fig. 9 und Fig. 19. Um dieses Kopplungsschema beibehalten zu können, ist im konkreten Ausführungsbeispiel das Kontaktteil 23a so gestaltet, daß es sich über einen Verbindungssteg 110 quer durch das Gehäuse 3 auf die gegenüberliegende Seite der Inhalatorkomponente 2 erstreckt. Wie Fig. 29 zeigt, verläuft der Verbindungssteg 110 unterhalb des schlitzförmigen Kanals 26. Anstatt des Verbindungssteges 110 könnte alternativ auch ein Draht die elektrische Verbindung herstellen. Ferner wäre es alternativ ebenso möglich, die beiden Steckkontakte 93 auf derselben Gehäuseseite aus dem Gehäuse herauszuführen, wobei sich hier naheliegenderweise jene Seite anbieten würde, auf welcher auch die Kontaktteile 23a und 23b angeordnet sind. Schließlich soll auch noch erwähnt sein, daß die plattenförmigen Kontakte bzw. Kontaktteile 23a, 23b und 23c auch durch Leiterplatten oder eine einzelne gemeinsame Leiterplatte gebildet werden können. Dickkupfer-Leiterplatten mit Kupfer-Schichtstärken im Bereich von 100-500μm erhalten wegen der besseren Wärmeableitung den Vorzug. Eine gute Wärmeableitung ist vor allem im Bereich des Kapillarspalts 41 sicherzustellen, um ein Sieden des flüssigen Materials 16 im Kapillarspalt 41 auszuschließen.

Einen wesentlichen Bestandteil des Inhalators bildet der Sensor 99, 100 - siehe Fig. 8, Fig. 18 sowie Fig. 21-22. Der Sensor 99, 100 hat die Aufgabe, den Beginn eines Zuges bzw. einer Inhalation zu detektieren, woraufhin der elektrische Schaltkreis 11 die Zufuhr der elektrischen Energie zum Heizelement des Verbundes 22, 39 aktiviert, und die Verdampfung des flüssigen Materials 16 einsetzt. Es können zumindest zwei verschiedene Arten von Sensoren verwendet werden: im Ausführungsbeispiel nach Fig. 8, besteht der Sensor aus einem Drucksensor 99. Der Drucksensor 99 ist in das Trägergehäuse 10 eingeklebt, und seine elektrischen Anschlüsse oder Pins 101 sind direkt auf der Platine 11 verlötet. Der Drucksensor 99 kommuniziert über eine Bohrung 102 mit der Plenumkammer 27 und mißt bzw. überwacht den Unterdruck in der Plenumkammer 27 - siehe Fig. 18. Als Drucksensor 99 eignet sich beispielsweise der Typ CPCL04GC des Herstellers Honeywell Inc., www.honeywell.com mit einem Meßbereich von +/-10mbar. Der genannte Sensor besteht im Wesentlichen aus einer Nullpunkt-kalibrierten und Temperatur-kompensierten Meßbrücke und kann auf der Platine 11 folgendermaßen beschaltet werden: der negative Sensorausgang wird über einen hochohmigen Widerstand mit einem definierten Widerstandswert - z.B. 2,2MOhm - auf Masse gelegt, wodurch das Ausgangs- oder Meßsignal des Drucksensors 99 geringfügig verzerrt wird, oder anders formuliert, der Offset der Meßbrücke auf einen definierten Wert kalibriert wird. Durch die Verzerrung bzw. durch den Offset wird eine Schaltschwelle vorgegeben, welche einem bestimmten Druck-Schwellwert entspricht. Das auf diese Weise aufbereitete Meßsignal wird auf den Eingang eines als Komparator geschalteten Präzisions-Operationsverstärkers 103 - z.B. des Typs LTC1049CS8 des Herstellers Linear Technology Inc., www.linear.com gelegt. Aus dieser Beschaltung resultiert ein Ausgangssignal, welches den Zugbeginn in digitaler Form überaus schnell und exakt abbildet. Der Drucksensor 99 eignet sich vor allem für die Verwendung in Zug-Inhalatoren, sofern stromaufwärts von der Plenumkammer 27 eine Strömungsdrossel 28 angeordnet ist. In diesem Fall tritt in der Plenumkammer 27 im Verlauf eines Zuges bezogen auf die Umgebung ein Unterdruck auf, welcher typischerweise im Bereich 0-50mbar liegt. Der Druckverlauf hat näherungsweise eine Glockenform. Der Zugbeginn kann auf einfache Weise detektiert werden, indem ein Druck-Schwellwert, wie zuvor beschrieben, vorgegeben wird, welcher ständig mit dem tatsächlich gemessenen Druck verglichen wird. Der Zugbeginn kann als das erstmalige Überschreiten des Druck-Schwellwertes definiert werden. Für den Druck-Schwellwert wird zweckmäßigerweise ein Wert im Bereich 0,2-5mbar gewählt. Je kleiner der Druck-Schwellwert gewählt wird, umso schneller spricht die Zugerkennung an. Eine untere Grenze wird durch die Spezifikationen des jeweiligen verwendeten Drucksensors und Operationsverstärkers gezogen.

Ist im Inhalator keine Strömungsdrossel 28 vorgesehen, so herrscht in der Plenumkammer 27 praktisch Umgebungsdruck. Diese Voraussetzungen sind im Ausführungsbeispiel nach Fig. 21-22 gegeben. Der dargestellte klassische Inhalator arbeitet annähernd unter atmosphärischen Druckbedingungen und ermöglicht eine direkte Lungeninhalation in einem einzigen Schritt. In diesem Fall ist es zweckmäßiger, den Inhalationbeginn mittels eines Strömungssensors 100 zu detektiern. Der Strömungssensor 100 ist im Ausführungsbeispiel nach Fig. 21-22 im Querkanal 29 angeordnet, und dessen Anschlüsse oder Pins 101 sind wieder direkt auf der Platine 11 verlötet. Als Strömungssensor 100 eignet sich vorzugsweise ein Thermistor 100, beispielsweise des Typs GR015 des Herstellers Betatherm Corporation, www.betatherm.com. Der Thermistor 100 ist auf der Platine 11 zu einer Meßbrücke (nicht dargestellt) verschaltet. Die Meßbrücke beinhaltet zur Temperaturkompensation einen typgleichen zweiten Thermistor und wird mittels Präzisionswiderständen auf einen definierten Offset-Schwellwert kalibriert. Das Ausgangssignal der Meßbrücke wird sodann wieder auf den Eingang eines als Komparator geschalteten Operationsverstärkers 103 gelegt. Im Gleichgewichtszustand befinden sich die beiden Thermistoren auf gleichem Temperaturniveau - typischerweise im Bereich 80-200°C, abhängig von der dissipierten Leistung. Sobald nun ein Benutzer mit der Inhalation beginnt, strömt Luft durch den Querkanal 29. Die Luft kühlt den Thermistor 100 ab, wodurch dessen Widerstand steigt. Die Widerstandsänderung wird von der Meßbrücke verarbeitet. In dem Moment, wo das Ausgangssignal der Meßbrücke den Nullpunkt durchschreitet, kippt der Komparator 103 und gibt ein den Inhalationbeginn kennzeichnendes digitales Signal aus.

Die Weiterverarbeitung der von den Sensoren 99, 100 und deren Beschaltungen ausgegebenen Signale erfolgt vorzugsweise in einem integrierten Schaltkreis 104 - siehe Fig. 8 und Fig. 21. Der integrierte Schaltkreis 104 kann auch ein Mikroprozessor sein. Der integrierte Schaltkreis 104 verarbeitet einen Großteil aller elektrischen Signale des Inhalators und führt die für den Betrieb des Inhalators wesentlichen Steuer-Operationen aus. Diese Steuer-Operationen sollen im Folgenden näher erläutert werden: eine zentrale Steuer-Operation stellt die Zufuhr der elektrischen Energie zum Heizelement des Verbundes 22, 39 dar. Die elektrische Energie wird vom Energiespeicher 12 geliefert. Basierend auf dem heutigen Stand der Technik bieten sich Lithium-Polymer- und Lithium-Ionen-Zellen aufgrund ihrer hohen Energie- und Leistungsdichte in besonderer Weise als Energiespeicher 12 an. Im Fall von metallischen Heizelementen findet man bereits mit einer einzelnen Lithium-Polymer- oder Lithium-Ionen-Zelle mit einer Leerlauf- oder Nennspannung von etwa 3,7V das Auslangen. Die Regelung der Energie- und Leistungszufuhr zum Heizelement des Verbundes 22, 39 kann in einfacher Weise dadurch erfolgen, daß die Batteriespannung über die Dauer der Energiezufuhr mit variablem Aussteuerungsgrad zerhackt wird, und die daraus resultierende Nutzspannung am Heizelement angelegt wird. Die resultierende Nutzspannung ist ein Rechteck-Signal mit variablem Tastverhältnis (Duty Cycle). Die Amplitude des Rechteck-Signals entspricht, wenn man von geringen Spannungsverlusten einmal absieht, der Batteriespannung. Die eigentliche Zerhackung erfolgt vorzugsweise mittels eines Leistungs-MOSFETs 105, z.B. des Typs IRF6635 des Herstellers International Rectifier, www.irf.com, welcher dazu geeignet ist, sehr hohe Ströme bei minimalem Drain-Source-Durchlasswiderstand zu schalten. Der integrierte Schaltkreis 104 steuert dabei das Gate des Leistungs-MOSFETs 105. Eine sehr einfache Regelungsstrategie, welche sich im Übrigen auch in erfindungsgemäßen Prototypen bewährt hat, besteht darin, die Dauer der Energiezufuhr in zwei Perioden zu unterteilen - in eine Aufheizperiode und eine daran anschließende Verdampfungsperiode. Im intermittierenden, inhalations- oder zugsynchronen Betrieb des Inhalators orientiert sich die Dauer der Energiezufuhr an der Dauer eines Zuges oder einer Inhalation. Im Fall von Zug-Inhalatoren kann beispielsweise von einer durchschnittlichen Zugdauer von etwa 2,1sec (+/-0,4sec) ausgegangen werden. Derselbe Wert gilt in etwa auch für Zigaretten. Berücksichtigt man, daß auch nach Abschalten der Energiezufuhr wegen der im Verbund 22, 39 noch gespeicherten Wärme zu einem gewissen Grad eine Nachverdampfung stattfindet, erscheint es zweckmäßig, die Dauer der Energiezufuhr etwas kürzer zu wählen, z.B. einen Wert im Bereich 1,5-1,8sec. Bei klassischen Inhalatoren kann es im Sinne eines hohen alveolären Arzneimittel-Absorptionsgrades von Vorteil sein, die Dauer der Energiezufuhr noch weiter zu verkürzen. Zug-Inhalatoren haben nämlich gegenüber klassischen Inhalatoren den Vorteil, daß sich das Arzneimittel sozusagen an der vordersten Front der in die Lunge inhalierten Luftsäule befindet, wodurch das Arzneimittel leichter bis zu den Alveolen vordringen kann. Dagegen geht bei klassischen Inhalatoren das Arzneimittel direkt in die inhalierte Luftsäule über. Hierbei ist zu berücksichtigen, daß ein Endabschitt der inhalierten Luftsäule nur dazu dient, den sogenannten "funktionellen Totraum" (ca. 150-200mL) des Atemsystems zu befüllen. Arzneimittelanteile in diesem Totraum erreichen jedenfalls nicht mehr die Alveolen und sind insofern für eine schnelle systemische Wirkung verloren. Berücksichtigt man ferner, daß die Inhalationsdauer individuell stark schwankt, nämlich etwa zwischen 1,5-3sec, erscheint es zweckmäßig, für die Dauer der Energiezufuhr bei klassischen Inhalatoren einen Wert <1,5sec zu wählen. Während der ersten der zuvor genannten zwei Perioden - der Aufheizperiode - wird der Verbund 22, 39 samt dem im Docht gespeicherten flüssigen Material 16 durch das Heizelement aufgeheizt. Die Verdampfung des flüssigen Materials 16 setzt erst ein, wenn die Temperatur des Verbundes 22, 39 etwa den Siedebereich der niedrig siedenden Fraktionen des flüssigen Materials 16 erreicht hat. Die Aufheizperiode sollte daher möglichst kurz sein. Insofern liegt es nahe, die Batteriespannung in dieser Periode unzerhackt bzw. mit einem Aussteuerungsgrad oder Duty Cycle von 100% an das Heizelement weiterzugeben. Die Dauer der Aufheizperiode hängt vor allem von den Spezifikationen des Verbundes 22, 39 und von der Menge und Zusammensetzung des zu verdampfenden flüssigen Materials 16 ab und sollte möglichst <0,5sec sein. In der anschließenden zweiten Periode - der Verdampfungsperiode - wird der Aussteuerungsgrad wesentlich zurückgenommen, und es erfolgt die eigentliche Verdampfung des flüssigen Materials 16. Die zugeführte Energie wird in dieser zweiten Periode primär zur Verdamfung des flüssigen Materials 16 und sekundär zur Deckung von Energieverlusten verwendet. Durch entsprechende Wahl des Aussteuerungsgrades kann die Verdampfungsleistung und damit auch die pro Zug oder Inhalation verdampfte Menge des flüssigen Materials 16 in gewissen Grenzen gesteuert werden. Eine obere Grenze ist durch das Auftreten einer Siedekrise sowie durch ein lokales Austrocknen und Überhitzen des Dochts gesetzt. Durch eine Zurücknahme bzw. Drosselung des Aussteuerungsgrades kann hingegen einer thermischen Zersetzung des flüssigen Materials 16 entgegengewirkt werden.

Die soeben beschriebene Regelungsstrategie kann beliebig erweitert und verfeinert werden: zum Beispiel kann es sinnvoll sein, auch den Zustand der Batterie in der Regelungsstrategie zu berücksichtigen, da die Batteriespannung mit zunehmender Entladung und zunehmendem Alter der Batterie vor allem unter Last deutlich sinkt. Diesem Effekt kann mit einer Erhöhung des Aussteuerungsgrades begegnet werden. Um diese Korrektur auch in der Aufheizperiode vornehmen zu können, ist es zweckmäßig, die Batteriespannung einer neuen, geladenen Batterie nicht wie früher vorgeschlagen zu 100%, sondern z.B. nur zu 80% auszusteuern, sodaß noch genügend Spielraum für eine Anpassung bleibt.

Die Steuerung der Energiezufuhr zum Heizelement des Verbundes 22, 39 erfordert außerdem verschiedene Hilfs-Operationen: beispielsweise muß vorgesehen werden, daß die Energiezufuhr nach dem Ende eines Verdampfungszyklus nicht gleich nochmals aktiviert werden kann. Vielmehr ist eine Wartezeit einzuhalten, welche dem flüssigen Material 16 genügend Zeit läßt, den Docht von neuem vollständig zu infiltrieren. Die mindestens erforderliche Wartezeit hängt von den jeweiligen Spezifikationen des Verbundes sowie von der Zähigkeit des flüssigen Materials ab. In Prototypen konnte gezeigt werden, und Berechnungen bestätigen, daß bei entsprechender Auslegung eine vollständige Infiltration des Dochts in weniger als 10sec erzielt werden kann. Eine verbindliche Wartezeit in dieser Größenordnung sollte von den meisten Benutzern toleriert werden, vor allem wenn man berücksichtigt, daß im Fall der Zigarette das Intervall zwischen zwei Zügen durchschnittlich 25sec beträgt. Eine solche Wartezeit ist gleichfalls nach dem Ankoppeln einer neuen Inhalatorkomponente 2 an das Inhalatorteil 1 einzuhalten. Eine andere Hilfs-Operation besteht darin, daß die Energiezufuhr zum Heizelement sofort abgebrochen wird, wenn der Benutzer den Zug bzw. die Inhalation vorzeitig abbricht. Auf diese Weise wird verhindert, daß in der Kammer 21 unnötig Dampf gebildet wird.

Eine weitere Steuer-Operation des integrierten Schaltkreises 104 betrifft die Benutzer-Schnittstelle, also die Kommunikation mit dem Benutzer. Der Sensor 99, 100 zur Erkennung des Zug- bzw. Inhalationsbeginns stellt eine Eingabe-Schnittstelle dar und ist als solcher unverzichtbar. In einer sehr einfachen Ausgestaltung der Benutzer-Schnittstelle ist darüberhinaus keine weitere Eingabe-Schnittstelle vorgesehen, nicht einmal ein Ein-Aus-Schalter, wodurch sich die Benutzung des Inhalators ausgesprochen unkompliziert gestaltet. Der Verzicht auf einen Ein-Aus-Schalter setzt natürlich einen entsprechend kleinen Eigenstrombedarf des elektrischen Schaltkreises 11 voraus, worauf im Zuge der Schaltplanerstellung zu achten ist. So kann beispielsweise vorgesehen sein, daß der Schaltkreis 11, solange keine Inhalatorkomponente 2 mit dem Inhalatorteil 1 gekoppelt ist, in einen besonders energiesparenden Sleep-Modus schaltet. Als Ausgabe-Schnittstellen können beispielsweise zwei Leuchtdioden 106 Verwendung finden, deren erste den Ladezustand der Batterie 12 anzeigt, und deren zweite das bevorstehende Wechselintervall der Inhalatorkomponente 2 signalisiert. Die Überwachung des Wechselintervalls der Inhalatorkomponente 2 kann durch einen Zähler erfolgen, welcher die Anzahl der Züge bzw. Inhalationen mitzählt. Der Zähler wird im Zuge des Austausches der Inhalatorkomponente 2 auf null zurückgesetzt (Reset), wobei der Umstand ausgenützt werden kann, daß der Heizelement-Widerstand für einen Moment unendlich groß wird. In einer etwas aufwendigeren Ausgestaltung kann anstatt der Leuchtdioden 106 ein Display (nicht dargestellt) in den Schaltkreisdeckel 7 integriert werden. Das Display kann neben dem Batterie-Ladezustand und dem bevorstehenden Wechsel der Inhalatorkomponente 2 noch weitere Betriebszustände und Informationen anzeigen, beispielsweise die über einen bestimmten Zeitraum insgesamt zugeführte Arzneimitteldosis. Im Fall des Nikotins kann auf diese Weise sehr objektiv der Grad der Nikotin-Abhängigkeit des Benutzers, und im Zuge einer schrittweisen Entwöhnung der tatsächlich erzielte Erfolg festgestellt werden. Schließlich kann das Display den Benutzer in Form einer Benutzerführung bei der Bedienung des Inhalators unterstützen. Als Ausgabe-Schnittstelle kann ferner ein akustischer, vibratorischer oder/und optischer Alarm vorgesehen sein, welcher den Benutzer dabei unterstützt, das jeweilige Arzneimittel zeitgerecht und in der erforderlichen Dosierung zuzuführen. Schließlich kann auch noch eine DatenSchnittstelle, z.B. in Form einer USB- oder Bluetooth- Schnittstelle, vorgesehen sein, über welche insbesondere Firmware- und Software-Updates eingespielt, Diagnosefunktionen ausgeführt und Informationen, insbesondere die verabreichte Arzneimitteldosis betreffend, ausgelesen werden können. Mittels der letzteren Funktion kann ein behandelnder Arzt exakt und objektiv die über einen längeren Zeitraum zugeführte Arzneimitteldosis und deren zeitlichen Verlauf aufzeichnen, auswerten und seine medizinische Behandlung darauf abstimmen.

Eine weitere Steuer-Operation, welche optional vorgesehen werden kann, betrifft die Identifizierung der verwendeten Inhalatorkomponente 2, die Identifizierung des Benutzers, und damit zusammenhängend die Feststellung einer mißbräuchlichen Verwendung des Inhalators. Die Identifizierung der Inhalatorkomponente 2 samt dem in ihr enthaltenen Verbund-Typ und flüssigen Material 16 kann auf einfache Weise durch eine Messung des Heizelement-Widerstandes erfolgen. Dieser Methode sind jedoch gewisse Grenzen gesetzt, weil jeder Arzneimittel-Zubereitung ein bestimmter Verbund-Typ mit einem definierten Heizelement-Widerstand zugeordnet werden muß. Eine etwas aufwendigere Methode besteht darin, in der Inhalatorkomponente 2 einen Identifikations-Chip (nicht dargestellt) anzuordnen, welcher die Inhalatorkomponente 2 eindeutig identifiziert. Mit Hilfe eines solchen Chips ist es möglich, jede einzelne erzeugte und verkaufte Inhalatorkomponente 2 eindeutig zu identifizieren. Der Chip ist vorzugsweise auf einem der beiden plattenförmigen Kontakte 23 angeordnet, wobei es besonders günstig ist, wenn der plattenförmige Kontakt 23 durch eine Leiterplatte gebildet wird. Die im Chip gespeicherte Information wird vom integrierten Schaltkreis 104, welcher in diesem Fall vorzugsweise aus einem Mikroprozessor besteht, ausgelesen. Auf Basis der ausgelesenen Information wählt der Mikroprozessor 104 die für die verwendete Inhalatorkomponente 2 geeigneten Betriebsparameter aus. Ferner kann der Mikroprozessor 104 die jeweilige Inhalatorkomponente 2 nach Erreichen des Wechselintervalls sperren oder durch geeignete Mittel unbrauchbar machen, sodaß mit dieser Inhalatorkomponente 2 keine weiteren Züge oder Inhalationen mehr ausgeführt werden können. Diese Maßnahme dient vor allem der Vermeidung einer mißbräuchlichen Verwendung der Inhalatorkomponente 2. Eine solche mißbräuchliche Verwendung läge beispielsweise vor, wenn ein Benutzer versucht, die Inhalatorkomponente 2 über das Wechselintervall hinaus weiter zu nutzen, indem er beispielsweise den Flüssigkeitsbehälter 4 gewaltsam öffnet und selbst flüssiges Material 16 nachfüllt. Im Fall des Nikotins beträgt die letale Dosis (LD50) zirka 0,5-1,0 mg/kg Körpergewicht. Man kann sich in etwa ausmalen, wie gefährlich ein solcher Mißbrauch für den Benutzer und dessen Umwelt sein würde. Die Gefahr eines solchen Mißbrauchs sowie die Gefährdung der Umwelt durch verbrauchte, weggeworfene Inhalatorkomponenten 2 kann weiter reduziert werden, indem die Inhalatorkomponente 2 nach dem Pfandsystem verkauft wird. Die Identifizierung des Benutzers dient dazu, eine Benutzung des Inhalators durch unbefugte Dritte auszuschließen, und beugt auf diese Weise außerdem einem Diebstahl vor. Die Identifizierung des Benutzers kann beispielsweise über ein Touch-Display durch Eingabe eines Codes, oder auf biometrische Weise mittels Fingerabdruck erfolgen.

Eine weitere Steuer-Operation, welche vom integrierten Schaltkreis 104 ausgeführt werden kann, betrifft das Zell- und Lademanagement der Batterie 12.

Da am Markt für diesen Zweck bereits integrierte Schaltkreise verfügbar sind, kann diese Steuer-Operation alternativ auch in einem separaten integrierten Schaltkreis erfolgen. Die Zufuhr des Ladestroms erfolgt über den Ladestecker 107, welcher an der dem Mundstück 5 abgewandten Stirnseite des Inhalatorteils 1 angeordnet ist - siehe Fig. 3 und Fig. 8. Der Ladestecker 107 kann gleichzeitig Diagnosestecker sein, über welchen mittels eines externen Analysegeräts der elektrische Schaltkreis 11 sowie der Heizelement-Widerstand des Verbundes 22, 39 geprüft, und mögliche Fehler festgestellt werden können.

Die Umsetzung der zuvor beschriebenen Steuer-Operationen in einen Schaltplan kann von jedem auf diesem Gebiet versierten Fachmann unter Anwendung bekannter Methoden geleistet werden, und soll daher in diesem Rahmen auch nicht weiter beschrieben werden.

Abschließend sei nochmals die Funktions- und Betriebsweise des Inhalators zusammenfassend erklärt: der Benutzer macht eine neue Inhalatorkomponente 2 einsatzbereit, indem er sie über die Schnappverbindung 8, 9 mit dem wiederverwendbaren Inhalatorteil 1 koppelt. Das Öffnen des Flüssigkeitsbehälters 4 erfolgt im Ausführungsbeispiel nach Fig. 6 synchron zur Kopplung mit dem Inhalatorteil 1 mittels des Stiftes 46 im Zusammenwirken mit dem Kontaktelement 20 (siehe Fig. 19). Dagegen erfolgt das Öffnen des Flüssigkeitsbehälters 4 im Ausführungsbeispiel nach Fig. 24a und Fig. 24b dadurch, daß der Benutzer den Flüssigkeitsbehälter 4 in das Gehäuse 3 verschiebt (siehe Pfeilrichtung). In beiden Fällen wird ein als Fortsatz 44 (Fig. 19) bzw. als erster Dorn 81 (Fig. 25) ausgebildetes Ende des Kapillarspalts 41 mit dem flüssigen Material 16 benetzt. Der Kapillarspalt 41 übt auf das benetzende flüssige Material 16 eine Kapillarkraft aus, welche bewirkt, daß der Kapillarspalt 41 rasch geflutet wird. Das flüssige Material 16 erreicht den Verbund 22, 39 (siehe Fig. 11). Der Verbund 22, 39 besteht aus einem Docht und einem elektrischen Heizelement. Die Kapillarkräfte im Docht bewirken, daß dieser ebenfalls rasch vom flüssigen Material 16 infiltriert wird. Gleichzeitig wird auch der aus Kapillaren 54 bestehende Pufferspeicher 53 vom flüssigen Material 16 geflutet. Der Pufferspeicher 53 ermöglicht einen lageunabhängigen Betrieb des Inhalators. Die Dauer zwischen dem Öffnen des Flüssigkeitsbehälters 4 bis zur vollständigen Infiltration des Dochts entspricht einer verbindlichen Wartezeit für den Benutzer und beträgt bei entsprechender Auslegung jedenfalls weniger als 10sec. Der Inhalator ist nun betriebsbereit. Der Benutzer führt über das Mundstück 5 im Fall eines erfindungsgemäßen Zug-Inhalators (Fig. 9-10) einen Zug ähnlich wie bei einer Zigarette, und im Fall eines erfindungsgemäßen klassischen Inhalators (Fig. 21-22) eine direkte Lungeninhalation aus. Der Sensor 99,100 (Fig. 8 und Fig. 21) detektiert den Beginn des Zuges bzw. der Inhalation und veranlaßt den integrierten Schaltkreis 104 das Heizelement des Verbundes 22, 39 nach einer vorgegebenen Regelungsstrategie mit elektrischer Energie zu versorgen. Dies führt dazu, daß sich der Verbund 22, 39 blitzartig aufheizt und das flüssige Material 16 im Docht verdampft. Der gebildete Dampf verläßt den Verbund 22, 39 über die in weiten Bereichen des Verbundes freiliegende Dochtoberfläche und mischt sich in der Kammer 21 mit der durch die Lufteinlaßöffnung 26 in die Kammer 21 einströmenden Luft. Durch die Mischung mit der Luft kühlt der Dampf ab und bildet ein Kondensationsaerosol (Fig. 9-10 und Fig. 21-22). Überschüssiges Kondensat, welches nicht zur Bildung des Kondensationsaerosols oder Dampf-Luft-Gemisches beiträgt, wird von in der Kammer 21 angeordneten Schwämmen 57 aufgesaugt und gebunden. Im Ausführungsbeispiel nach Fig. 9-10 (Zug-Inhalator) strömt das gebildete Dampf-Luft-Gemisch oder/und Kondensationsaerosol zur Verbesserung seiner organoleptischen Eigenschaften noch durch das Füllmaterial 61, bevor es schließlich über den Mundstückkanal 66 in die Mundhöhle des Benutzers gelangt. Im Ausführungsbeispiel nach Fig. 21-22 (klassischer Inhalator) tritt das gebildete Dampf-Luft-Gemisch oder/und Kondensationsaerosol durch die von den Leitschaufeln 69 ausgebildete Mündungsöffnung 71 aus der Kammer 21 aus und vereinigt sich mit der durch die Bypassöffnungen 68 einströmenden Bypassluft, um schließlich nach Durchströmen eines im Mundstückkanal 66 optional angeordneten Strömungshomogenisators 72 ebenfalls in die Mundhöhle des Benutzers zu gelangen. Nach einer Wartezeit von wenigen Sekunden hat das flüssige Material 16 den Docht des Verbundes 22, 39 von neuem vollständig infiltriert, und der Inhalator ist bereit für eine weitere Inhalation. Enthält der Flüssigkeitsbehälter 4 beispielsweise 2,5mL effektiv nutzbares flüssiges Material 16, und beinhaltet das flüssige Material Nikotin als Arzneimittel in einer Konzentration von typischerweise 1,5 Vol.-%, dann können mit einer solchen Inhalatorkomponente bis zu 380 Züge oder Inhalationen ausgeführt werden, wenn pro Inhalation 100$\mu$g Nikotin verdampft werden. 380 Züge entsprechen etwa 38 Zigaretten. Werden pro Inhalation nur 50$\mu$g Nikotin verdampft, dann steigert sich die Reichweite auf 760 Inhalationen, welcher Wert etwa vier Packungen Zigaretten entspricht.

Anhand des Arzneimittels Nikotin soll schließlich noch eine beispielhafte Zubereitung des flüssigen Materials 16 offenbart werden, welche in erfindungsgemäßen, als Zug-Inhalatoren ausgelegten Prototypen verdampft wurde. Das hierbei gebildete und verabreichte Kondensationsaerosol kam hinsichtlich der pharmakologischen, pharmakokinetischen sowie organoleptischen Wirkungen dem Rauch einer konventionellen Zigarette sehr nahe. Sämtliche aufgeführten Inhaltsstoffe finden sich auch im Zigarettenrauch wieder.

Tabelle 2: Exemplarische Arzneimittel-Zubereitung auf Basis Nikotin

| Stoff | CAS-Nummer | Massen-% |
|---|---|---|
| Ethanol | 64-17-5 | 68,80 |
| Wasser | 7732-18-5 | 16,50 |
| Glycerol | 56-81-5 | 9,10 |
| Nikotin | 54-11-5 | 1,80 |
| Milchsäure | 50-21-5 | 0,23 |
| Bernsteinsäure | 110-15-6 | 0,28 |
| Lävulinsäure | 123-76-2 | 0,46 |
| Benzoesäure | 65-85-0 | 0,08 |
| Phenylessigsäure | 103-82-2 | 0,08 |
| Essigsäure | 64-19-7 | 1,67 |
| Ameisensäure | 64-18-6 | 0,53 |
| Propionsäure | 79-09-4 | 0,27 |
| Solanon | 1937-54-8 | 0,05 |
| Tabakaromaöle | *) | 0,15 |
| Ambroxid | 6790-58-5 | optional |
| Menthol | 2216-51-5 | optional |
| | Summe: | 100,00 |
| *) mittels überkritischer CO2-Extraktion gewonnene Tabakaromaöle; z.B. | | |

Tabakextrakte der Firma Pro-Chem Specialty Limited, Hong Kong, www.pro-chem-specialty.com, z.B. Produkt-Nr. SF8010, SF8011 oder SF208118; oder gemäß Patent-Publikations-Nr. DE19654945A1, DE19630619A1, DE3218760A1, DE3148335A1 (Adam Müller et al.) hergestellte Tabakaromaöle; Voraussetzung für die Verwendung solcher Tabakaromaöle in der Nikotinlösung ist, daß diese möglichst frei von tabakspezifischen Nitrosaminen (TSNA) sind.

Der Vollständigkeit halber sei auch noch angemerkt, daß in den beschriebenen Inhalator zusätzliche Funktionen integriert sein können, welche über die eigentliche Aufgabe des Inhalators hinausgehen und den Inhalator zum Multifunktionsgerät oder Hybridgerät erweitern. Solche Funktionen können zum Beispiel sein: Uhr, mobile Datenspeicherung, Player-Funktionen (inklusive Diktierfunktion), PDA-Funktionen, Navigationshilfe (GPS), Mobiltelefonie und Fotografie.

**Bezugszeichenliste**

1 Inhalatorteil
2 Inhalatorkomponente
3 Gehäuse
4 Flüssigkeitsbehälter
5 Mundstück
6 Batteriedeckel
7 Schaltkreisdeckel
8 Schnapphaken
9 Rastnase
10 Trägergehäuse
11 elektrischer Schaltkreis, Platine
12 Energiespeicher; Batterie
13 Trennwand
14 Flachkontakt
15 Fenster
16 flüssiges Material; Arzneimittel-Zubereitung
17 Füllloch
18 öffenbarer Verschluß
19 Verschlußdeckel
20 Kontaktelement
21 Kammer
22 flächiger Verbund
23 plattenförmiger Kontakt
24 erste Seite des flächigen Verbundes
25 zweite Seite des flächigen Verbundes
26 Lufteinlaßöffnung; schlitzförmiger Kanal
27 Plenumkammer
28 Strömungsdrossel
29 Querkanal
30 Speiseöffnung
31 Folie; Metallfolie
32 Gewebe; Metalldrahtgewebe
33 offenporige Faserstruktur; Vlies
34 offenporige Sinterstruktur; körniger, faseriger oder flockiger Sinterverbund
35 Kanal; Arterie
36 Loch
37 offenporiger Schaum
38 Trägerlage
39 linienförmiger Verbund
40 Preßstempel
41 Kapillarspalt
42 Oberteil
43 Platte
44 Fortsatz
45 Reservoir

| 46 | Stift |
|---|---|
| 47 | erstes Ende |
| 48 | zweites Ende |
| 49 | Materialschwächung |
| 50 | Scharnier |
| 51 | Querschnittserweiterung |
| 52 | Belüftungskanal |
| 53 | Pufferspeicher |
| 54 | Kapillare; Schlitz |
| 55 | Öffnung |
| 56 | Belüftungsspalt |
| 57 | offenporiger, saugfähiger Körper; Schwamm |
| 58 | Strömungskanal |
| 59 | Wandabschnitt |
| 60 | Spalt |
| 61 | Kühler; Füllmaterial; Tabakfüllung |
| 62 | Füllraum |
| 63 | Lochwand |
| 64 | erstes Drahtgewebe |
| 65 | zweites Drahtgewebe |
| 66 | Mundstückkanal |
| 67 | Sammelkammer |
| 68 | Bypassöffnung |
| 69 | Leitschaufel |
| 70 | Leitschaufelspitze |
| 71 | Mündungsöffnung |
| 72 | Strömungshomogenisator |
| 73 | nicht entriegelbare Blockiervorrichtung; Vorsprung |
| 74 | Zapfen |
| 75 | Nut |
| 76 | Entüftungsöffnung |
| 77 | Entlüftungskanal |
| 78 | erste Öffnung |
| 79 | zweite Öffnung |
| 80 | Foliendichtung |
| 81 | erster Dorn |
| 82 | zweiter Dorn |
| 83 | Mikrosteg |
| 84 | Flüssigkeitsspeicher; offenporiger Schaumstoff |
| 85 | Patronengehäuse |
| 86 | Patrone |
| 87 | Ausnehmung |
| 88 | Deckel |
| 89 | Schnappverbindung |
| 90 | Erhebung |
| 91 | Belüftungsloch |
| 92 | Aussparung |
| 93 | Steckkontakt |
| 94 | Federkontakt |
| 95 | Draht |
| 96 | Zentriervorsprung |
| 97 | Zentrierausnehmung |
| 98 | Entlüftungsloch |
| 99 | Drucksensor |
| 100 | Strömungssensor, Thermistor |
| 101 | elektrischer Anschluß; Pin |
| 102 | Bohrung |
| 103 | Operationsverstärker; Komparator |

104 integrierter Schaltkreis; Mikroprozessor
105 Leistungs-MOSFET
106 Leuchtdiode
107 Ladestecker
108 Ausnehmung
109 Arretier-Plättchen
110 Verbindungssteg

**Patentansprüche**

1. Inhalatorkomponente für die intermittierende, und inhalations- oder zugsynchrone Bildung eines Dampf-Luft-Gemisches oder/und Kondensationsaerosols, umfassend:

   ein Gehäuse (3);
   eine im Gehäuse (3) angeordnete Kammer (21);
   eine Lufteinlassöffnung (26) für die Zufuhr von Luft aus der Umgebung in die Kammer (21);
   ein elektrisches Heizelement zur Verdampfung einer Portion eines flüssigen Materials (16), wobei der gebildete Dampf sich in der Kammer (21) mit der durch die Lufteinlassöffnung (26) zugeführten Luft mischt, und sich das Dampf/Luft-Gemisch oder/und Kondensationsaerosol bildet;
   und einen Docht mit einer Kapillarstruktur, welcher Docht mit dem Heizelement einen Verbund (22) bildet und das Heizelement nach einer Verdampfung von neuem selbsttätig mit dem frischen flüssigen Material (16) versorgt, **dadurch gekennzeichnet, dass** der Verbund (22) eine flächige Struktur umfassend ein auf einer Metallfolie angeordnetes elektrisch nicht leitendes Material mit einer offen-porigen Struktur ist.

2. Inhalatorkomponente nach Anspruch 1, wobei das elektrisch nicht leitende Material Quarzglas ist.

3. Inhalatorkomponente nach Anspruch 1 oder Anspruch 2, wobei der Docht in Dickenrichtung gelocht ist.

4. Inhalatorkomponente nach Anspruch 3, wobei die Lochung mittels eines Lasers durchgeführt wird.

5. Inhalatorkomponente nach einem der Ansprüche 1 bis 4, wobei der Verbund (22) mindestens zwei Schichten aufweist.

6. Inhalatorkomponente nach Anspruch 5, wobei die Schichten mindestens eine der folgenden Strukturen enthalten: Platte, Folie (31), Papier, Gewebe (32), offenporige Faserstruktur (33), offenporige Sinterstruktur (34), offenporiger Schaum (37) und offenporige Abscheidungsstruktur.

7. Inhalatorkomponente nach Anspruch 6, **dadurch gekennzeichnet, dass** die Schichten durch eine Wärmebehandlung miteinander verbunden sind.

8. Inhalatorkomponente nach einem der Ansprüche 1 bis 7, wobei der Verbund (22, 39) die Kammer (21) brückenartig durchsetzt und mit zwei Endabschnitten auf zwei elektrisch leitenden, plattenförmigen Kontakten (23) angebracht ist, und das Heizelement mit den Kontakten (23) elektrisch kontaktiert ist.

9. Inhalatorkomponente nach Anspruch 8, wobei die elektrische Kontaktierung des Heizelements aus einer Schweiß- oder Sinterverbindung besteht.

10. Inhalatorkomponente nach Anspruch 8, **dadurch gekennzeichnet, dass** die elektrische Kontaktierung des Heizelements aus einer Klebeverbindung mittels eines elektrisch leitenden Klebstoffs besteht.

11. Inhalator, umfassend eine Inhalatorkomponente (2) nach einem der Ansprüche 1 bis 10.

**Claims**

1. Inhaler component for the intermittent and inhalation- or puff-synchronous formation of a vapour-air mixture or/and condensation aerosol, comprising:

a housing (3);
a chamber (21) arranged in the housing (3);
an air admission opening (26) for the supply of air from the surroundings into the chamber (21);
an electric heating element for evaporating a portion of a liquid material (16), wherein the vapour formed mixes in the chamber (21) with the air supplied through the air admission opening (26), and the vapour/air mixture or/and condensation aerosol is formed;
and a wick with a capillary structure, which wick forms a composite (22) with the heating element and, after an evaporation operation, resupplies the heating element automatically with the fresh liquid material (16),
**characterized in that** the composite (22) comprises a flat structure comprising an electrically non-conductive material which is arranged on a metal foil and has an open-pored structure.

2. Inhaler component according to Claim 1, wherein the electrically non-conductive material is quartz glass.

3. Inhaler component according to Claim 1 or Claim 2, wherein the wick is perforated in the thickness direction.

4. Inhaler component according to Claim 3, wherein the perforation is carried out by means of a laser.

5. Inhaler component according to one of Claims 1 to 4, wherein the composite (22) comprises at least two layers.

6. Inhaler component according to Claim 5, wherein the layers contain at least one of the following structures: a plate, film/foil (31), paper, fabric (32), open-pored fibre structure (33), open-pored sintered structure (34), open-pored foam (37) and open-pored deposition structure.

7. Inhaler component according to Claim 6, **characterized in that** the layers are connected to one another by a heat treatment.

8. Inhaler component according to one of Claims 1 to 7, wherein the composite (22, 39) passes through the chamber (21) in a bridge-like manner and is mounted by two end portions on two electrically conductive, plate-shaped contacts (23), and the heating element is electrically contact-connected to the contacts (23).

9. Inhaler component according to Claim 8, wherein the electrical contact-connection of the heating element consists of a welded or sintered connection.

10. Inhaler component according to Claim 8, **characterized in that** the electrical contact-connection of the heating element consists of an adhesive connection by means of an electrically conductive adhesive.

11. Inhaler comprising an inhaler component (2) according to one of Claims 1 to 10.


**Revendications**

1. Composant d'inhalateur permettant de former, de manière intermittente, synchronisée avec l'inhalation ou l'aspiration, un mélange vapeur-air ou/et un aérosol de condensation, comprenant :

un boîtier (3) ;
un compartiment (21) disposé dans le boîtier (3) ;
un orifice d'entrée d'air (26) destiné à amener de l'air depuis l'environnement jusque dans le compartiment (21) ;
un élément chauffant électrique pour vaporiser une dose d'une matière liquide (16), dans lequel la vapeur formée se mélange dans le compartiment (21) avec l'air amené à travers l'orifice d'entrée d'air (26), et le mélange vapeur-air ou/et l'aérosol de condensation se forment ;
et une mèche ayant une structure capillaire, laquelle mèche forme un ensemble (22) avec l'élément chauffant et alimente automatiquement l'élément chauffant après une vaporisation à nouveau avec la matière liquide (16) fraîche,
**caractérisé en ce que** l'ensemble (22), en comprenant une structure plane, est un matériau électriquement non conducteur d'une structure à pores ouverts, disposé sur un film métallique.

2. Composant d'inhalateur selon la revendication 1, dans lequel le matériau électriquement non conducteur est du verre de quartz.

**3.** Composant d'inhalateur selon la revendication 1 ou la revendication 2, dans lequel la mèche est perforée dans la direction de l'épaisseur.

**4.** Composant d'inhalateur selon la revendication 3, dans lequel la perforation est effectuée au moyen d'un laser.

**5.** Composant d'inhalateur selon l'une quelconque des revendications 1 à 4, dans lequel l'ensemble (22) présente au moins deux couches.

**6.** Composant d'inhalateur selon la revendication 5, dans lequel les couches comportent au moins l'une des structures suivantes : une plaque, un film (31), du papier, un tissu (32), une structure fibreuse à pores ouverts (33), une structure frittée à pores ouverts (34), une mousse à pores ouverts (37) et une structure de séparation à pores ouverts.

**7.** Composant d'inhalateur selon la revendication 6, **caractérisé en ce que** les couches sont reliées ensemble par un traitement thermique.

**8.** Composant d'inhalateur selon l'une quelconque des revendications 1 à 7, dans lequel l'ensemble (22, 39) traverse la chambre (21) à la manière d'un pont et est fixé par deux parties d'extrémité sur deux contacts (23) en forme de plaque, électriquement conducteurs, et l'élément chauffant est mis en contact électrique avec les contacts (23).

**9.** Composant d'inhalateur selon la revendication 8, dans lequel la mise en contact électrique de l'élément chauffant est composée d'une liaison soudée ou frittée.

**10.** Composant d'inhalateur selon la revendication 8, **caractérisé en ce que** la mise en contact électrique de l'élément chauffant est composée d'une liaison adhésive au moyen d'un adhésif électriquement conducteur.

**11.** Inhalateur, comprenant un composant d'inhalateur (2) selon l'une quelconque des revendications 1 à 10.

**Fig. 1**

EP 4 162 823 B1

**Fig. 2**

**Fig. 3**

**Fig. 4**

Fig. 5

**Fig. 6**

Fig. 7

EP 4 162 823 B1

99

20

10

101

103

106

3

8

9

105

104

11

107

**Fig. 8**

**Fig. 9**

Fig. 10

Fig. 11

EP 4 162 823 B1

Fig. 12a

Fig. 12

**Fig. 13a**

**Fig. 13b**

EP 4 162 823 B1

**Fig. 14a**

**Fig. 14b**

# Fig. 15a

22

34

# Fig. 15b

22

34

36

35

22

38

25

24

37

# Fig. 15c

Fig. 16a

Fig. 16

**Fig. 17**

**Fig. 18**

Fig. 19

Fig. 20

**Fig. 21**

Fig. 22

**Fig. 23**

5

4

68,69

3

5

71

70

EP 4 162 823 B1

Fig. 24b

Fig. 24a

61

**Fig. 25**

**Fig. 26**

EP 4 162 823 B1

Fig. 27

Fig. 28

**Fig. 29**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- GB 25575AD1911 A.D., Elwin Kendal Hill
- US 2057353 A.D., Clinton L. Whittemore
- GB 25575AD1911 AD
- FR 960469, M. Eugène Vacheron
- CA 2309376, Matsuyama Futoshi
- US 6155268 A, Manabu Takeuchi
- US 4922901 A
- US 4947874 A
- US 4947875 A, Johnny L. Brooks
- US 5060671 A
- US 5095921 A, Mary E. Counts, D. Bruce Losee
- US 5322075 A, Seetharama C. Deevi
- US 20050268911 A, Steven D. Cross
- US 5505214 A
- US 5865185 A, Alfred L. Collins
- US 4735217 A, Donald L. Gerth
- EP 1736065 A, Hon Lik
- US 4848374 A, Brian C. Chard
- DE 202006013439 U
- US 2007107879 A1
- US 2003108342 A1
- US 5167242 A, James E. Turner
- US 6790496 B, Gustaf Levander
- US 3433632 A, Raymond J. Elbert
- US 6652804 B, Peter Neumann
- JP 2004332069 A, Tsujimoto Tetsushi
- US 3111396 A, Burton B. Ball
- DE 1950439, Peter Batzies
- AU 6393173, Ralph E. Shackleford
- DE 19654945 A1
- DE 19630619 A1
- DE 3218760 A1
- DE 3148335 A1, Adam Müller